# EUROPEAN PATENT APPLICATION

(11) **EP 2 281 558 A1**
(43) Date of publication of application: **09.02.2011**
(21) Application number: 09384003.1
(22) Date of filing: 06.08.2009
(51) Int. Cl.: A61K 31/135, C07C 215/64, A61K 31/192, A61K 31/196, A61K 31/616, C07C 59/64, C07C 69/84, A61P 29/00, A61P 25/04

(54) **Pharmaceutical compounds of O-Desmethyl-Tramadol and COX-inhibitors**

(71) Applicant: Laboratorios del. Dr. Esteve, S.A., 08041 Barcelona (ES)
(72) Inventor: Buschmann, Helmut Heinrich, 52076 Aachen (Walheim) (DE); Tesson, Nicolas, 08028 Barcelona (ES); Farran, Joan, 08028 Barcelona (ES)
(74) Representative: Peters, Hajo

(57) **Abstract**

The present invention relates to pharmaceutical compounds comprising *O*-desmethyl-Tramadol and at least one COX-inhibitor, especially NSAID, processes for preparation of the same and their uses as medicaments or in pharmaceutical formulations, more particularly for the treatment of pain. The pharmaceutical compound may be a salt or a crystalline form of a salt or a co-crystal.

## Description

The present invention relates to pharmaceutical compounds comprising *O*-desmethyl-Tramadol and at least one COX-inhibitor, especially NSAID, processes for preparation of the same and their uses as medicaments or in pharmaceutical formulations, more particularly for the treatment of pain. The pharmaceutical compound may be a salt or a crystalline form of a salt or a co-crystal.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

There are many drugs (analgesics) that are known to be useful in the treatment or management of pain. In principle, these analgesics are classified by their mechanism of action into opioids and non-opioids analgesics.

Opioids, also known as derivatives of morphine, are frequently used as analgesics in pain. In general, derivatives of morphine are indicated for the treatment of moderate to acute pain in human. The analgesic effect is obtained through their action on morphinic (opioid) receptors, preferably µ-receptors. Among these derivatives of morphine, may be mentioned morphine, codeine, pethidine, dextropropoxyphene methadone, and others.

One of the morphinic derivatives that has shown very good results when orally administrated, and which is now marketed, is Tramadol, also available as a physiologically acceptable salt, particularly as a chlorhydrate. Tramadol is a central acting analgesic drug that exerts its effects by activating opioid receptors and enhancing neuronal monoamine synaptic concentration. There is growing evidence that *O*-desmethyl-Tramadol (M1), the active metabolite of Tramadol, induces potent antinociception and is largely responsible for its opioid effects. *O*-desmethyl-Tramadol is one of the main metabolites of Tramadol. It has been widely used clinically and has a more potent analgesic activity than Tramadol.

*O*-desmethyl-Tramadol, whose chemical name is 2-[(Dimethylamino)methyl]-1-(3-hydroxyphenyl)cyclohexanol, has the following formula:

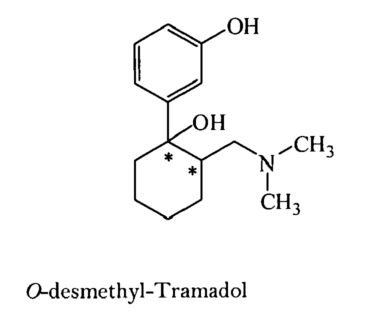

This structure shows two different chiral centers and thus the compound may exist in different diastereoisomers among which the *O*-desmethyl-Tramadol is the *cis*-diastereoisomer: (1*R*, 2*R*), or (1*S*, 2*S*), both also known as (+)-*O*-desmethyl-Tramadol and (-)-*O*-desmethyl-Tramadol and both of which contribute in different ways to the activity of the racemate: (1*R*,2*R*)-(+)-tramadol inhibits 5-HTuptake and is a weak µ opioid agonist; (1*S*,2*S*)-(-)-tramadol inhibits NA uptake; (1*R*,2*R*)-(+)-M1 (O-desmethyltramadol) is µ opioid agonist (6 times more analgesic than (+)-Tramadol ) and (1*S*,2*S*)-(-)-M1 (O-desmethyltramadol) inhibits NA uptake (Liu Hui.-Chen et al. Chirality 2004, 16 112-118). Garrido M.J. et al. (J. Pharmacol. Exp. Ther, 2000, 1, 352-359) further showed the activity of both enantiomers of O-desmethyltramadol and an analgesic enhancement in coadministration.

Tramadol is metabolized via the enzyme-system cytochrome P450 2D6 (CYP2D6). According W. N. Wu (Xenobiotica, 2002, 32(5), 411-425) O-desmethyl tramadol transforms into metabolites of phase I N-desmethyl-O-desmethyl tramadol (via CYP3A4) and hydroxy-O-desmethyl tramadol and also combines in phase II with glucoronic and sulphate by an enzymatic via different than CYPs. According to Wu, there is no evidence that CYP2D6 is involved in the metabolism of O-desmethyl tramadol. On the other hand, interindividual variability in the exposition and the analgesic activity of tramadol could be due to genetic polymorphism of CYP2D6 (Rasmus Steen Pedersen et al. Eur. J. Clin. Pharmacol, (2006), 62, 513-521 and Lars Poudsen et at. Clinical Pharmacology and Therapeutics, 1996, 60(6), 636-644). This seems avoidable with direct administration of O-desmethyltramadol. Therefore, using O-desmethyl-Tramadol instead of Tramadol clearly has advantages.

However, opioids exhibit severe drawbacks, such as tolerance, dependence, risk of addiction and abuse. Therefore, opioids cannot always be given repeatedly or at higher doses as analgesics. For reviewing side effects of opioids one can mention J. Jaffe in "Goodman and Gilman's, The Pharmacological Basis of Therapeutics", 8th edition; Gilman et al.; Pergamon Press, New York, 1990, Chapter 22, pages 522-573. For these reasons, the use of opioids is usually at lower doses and less frequently than the treatment of pain would normally require. Thus, it would be desirable to provide drugable forms of opioids improving the properties of opioids and therefore, allowing to lower the amount of opioids needed to provide an equivalent degree of analgesia.

In this respect it has been proposed to combine opioids with other drugs that are non-opioid analgesic agents. For instance, among these combinations, a pharmaceutical composition comprising Tramadol with non-steroidal anti-inflammatory drugs (NSAIDs) has been reported to be of particular interest (EP-0 546 676).

In addition, US 5,516,803 patent (family of EP-0 546 676) discloses pharmaceutical compositions of Tramadol with non-steroidal antiinflammatory drugs (NSAIDs), specifically Ibuprofen and discloses that the combination of Tramadol·HCl with non-steroidal anti-inflammatories, such as for example Ibuprofen, in one pharmaceutical composition with a ratio of 1:1 1 to 1:200 produces a synergistically enhanced analgesic action and reduces the undesired accompanying symptoms.

Further, US 6,558,701 patent discloses a multilayer tablet for oral administration containing at least one Tramadol layer, at least one Diclofenac layer and at least one separating layer which separates the Tramadol layer(s) and the Diclofenac layer(s) from each other. In addition, "for the treatment of moderate to severe pain" the World Health Organization (WHO) recommends combining opioid analgesics with non-steroidal analgesics in order to produce a more effective pain relief and possibly reduce amounts of analgesic which are necessary to administer".

However, all the above-mentioned suggestions are related to pharmaceutical compositions, wherein opioids and non-opioids analgesics are not associated by chemical interactions in order to form one single entity or one single crystal structure. The art is also completely silent on combination of NSAIDs with M1.

One commonly known group of non-opioid analgesic agents are the well established COX-inhibitors. COX-inhibitors, as their name implies, are effective in relieving pain via inhibiting the enzymes cyclooxygenase (COX) I (constitutive) and/or II (inducible) involved in the production of prostaglandins. COX-inhibitors include non-steroidal anti-inflammatory drugs (NSAIDs) with Acetylsalicylic acid known under its trademark Aspirin - despite being more than 100 years old - being an outstandingly used pharmaceutical. Besides Aspirin other COX-inhibitors whose use generally is also centred on anti-inflammatory action like Ibuprofen, Naproxen or Diclofenac are among the worldwide most frequently applied pharmaceutical compounds. In addition, it has been recently shown that Paracetamol also functions as a COX-II inhibitor (FASEB J., 2008 Feb; 22(2):383-90).

The advantage of COX-inhibitors, such as Paracetamol and the NSAIDs, is that they do not produce tolerance or physical dependence and are also not associated with abuse or addiction. However, for a number of COX-inhibitors a low solubility in water exists. As an example, this is especially true for the very popular and widely used and distributed members of the group of COX-inhibitors, Naproxen, Diclofenac and Ibuprofen, whose poor solubility is a published fact, that has lead to considerably efforts for improvement by using solution enhancers etc. in their formulation. Accordingly the COX-inhibitors like Naproxen, Diclofenac or Ibuprofen hardly seemed to be partners of choice for improving solubility of another also nearly insoluble compound.

As outlined above, *O*-desmethyl-Tramadol has a more potent analgesic activity than Tramadol. However, due to the drawbacks associated with opioids, *O*-desmethyl-Tramadol has to be given at lower doses and less frequent as its use as analgesic to treat pain would normally require. Therefore, in order to lower the amount of *O*-desmethyl-Tramadol needed to provide an equivalent degree of analgesia the association of *O*-desmethyl-Tramadol with non-opioid analgesic would be desirable. However, as shown above many non-opioid analgesics, such as COX-inhibitors, are well-known to be only slightly soluble in water limiting their use in pharmaceutical formulations. In addition, it is also commonly known that there are a number of chemical difficulties to be overcome for obtaining salts of COX-inhibitors or *O*-desmethyl-Tramadol.

Thus, it is an object of the present invention to provide new means improving the properties of *O*-desmethyl-Tramadol, especially in regard to the treatment of pain, by providing new drugable forms of *O*-desmethyl-Tramadol. In addition, it is another object of the present invention to combine *O*-desmethyl-Tramadol with non-opioid analgesic, such as COX-inhibitors, in order to lower the amount of *O*-desmethyl-Tramadol needed to provide an equivalent degree of analgesia.

It is a further object of the present invention to provide a new drugable form of *O*-desmethyl-Tramadol which should combine more than one, preferably most of the following improvements/advantages:
- improvement of physicochemical properties in order to facilitate the formulation, the manufacture, or to enhance the absorption and/or the bioavailability:
   thus
- being more active when compared to *O*-desmethyl-Tramadol base or its hydrochloride salt; or
- providing a form of *O*-desmethyl-Tramadol with a further COX-inhibitors having a beneficial pharmacological effect in itself, thus allowing for a highly efficient dose/weight relation of the final active principle or even
- allowing the use of a lower therapeutic dose of either *O*-desmethyl-Tramadol and the further COX-inhibitor, or of both;
- having a synergistic effect through the combination of *O*-desmethyl-Tramadol and the further COX-inhibitor in the same new drugable form; or
further
- being easily obtainable, easy to manufacture, or
- allowing more flexibility in formulating, or facilitating its formulation,
- being highly soluble, thus allowing better dissolution rates, especially if dissolving in an aqueous physiological surrounding, or
- improve stability of the salt or co-crystal in comparison with the physical mixture of *O-*desmethyl-Tramadol/COX-inhibitor at the same ratio;
- improve hygroscopicity;
- allowing new routes of administration;
also
- allowing - if necessary - to combine *O*-desmethyl-Tramadol with a chemically usually non-compatible active agent in the same formulation or even in immediate contact, without having to isolate *O*-desmethyl-Tramadol
or finally
- minimizing/reducing the side effects, especially the severe side effects, assigned to *O-*desmethyl-Tramadol.

It was found, surprisingly, that *O*-desmethyl-Tramadol and COX-inhibitors as starting elements are suitable to form a single chemical entity by ionic, covalent, hydrogen bonds, van der Waals forces, or π-π interactions. The association of at least two active elements into one chemical entity seems to allow for a better Pharmacokinetic/Pharmacodynamic (PKPD) including also a better penetration of the blood-brain barrier, which helps in the treatment of pain.

Therefore, in a first aspect thereof the object of the present invention is solved by providing a pharmaceutical compound comprising *O*-desmethyl-Tramadol and at least one COX-inhibitor. The group of COX-INHIBITORs includes the NSAIDs (Non steroidal anti-inflammatory drugs).

A "pharmaceutical compound" according to the present invention is a single chemical entity comprising two or more different elements that can be separated by chemical reactions and that have a unique and defined chemical structure. Pharmaceutical compounds consist of a fixed ratio of atoms that are held together in a defined spatial arrangement by ionic, covalent, hydrogen bonds, van der Waals forces, or π-π interactions. According to the present invention the elements of a pharmaceutical compound are *O*-desmethyl-Tramadol, at least one COX-inhibitor, water, ions, solvents like ethanol, or coformers.

In addition, the pharmaceutical compound according to the present invention represents a "drugable form of *O*-desmethyl-Tramadol". A "drugable form" as used herein is defined as any form (salt, amorphous, crystal (of a salt), co-crystal, solution, dispersion, mixture etc.) that *O-*desmethyl-Tramadol might take which still can be formulated into a pharmaceutical formulation usable as a medicament to treat a disease or a symptom, especially pain.

In one embodiment the pharmaceutical compound according to the present invention comprises *O*-desmethyl-Tramadol as racemic *O*-desmethyl-Tramadol, (+)-*O*-desmethyl-Tramadol, or (-)-*O-*desmethyl-Tramadol.

"COX-INHIBITOR" is defined by the basis of their activity being inhibition of cyclooxygenase (COX), one of the two activities of prostaglandine endoperoxide synthase (PGHS). PGHS is a key enzyme in the prostaglandin pathway. Some preferred co-crystal formers in the meaning of this application are those (COX-INHIBITORs/NSAIDs) with a carboxylic acid function, with examples including salicylates, anthranilates, arylacetic acids/arylalkanoic acids, and arylpropionic acids, but also including Coxibs.

The COX-INHIBITORs (which include the NSAIDs) selected according to this preferably are those with a carboxylic acid function. Preferred examples include salicylates, anthranilates, arylacetic acids/arylalkanoic acids, and arylpropionic acids. Therefore, in a preferred embodiment of the invention the pharmaceutical compound comprises *O*-desmethyl-Tramadol and at least one COX-inhibitor selected from NSAIDs, preferably from NSAIDs with a carboxylic acid function. Another preferred embodiment of the invention is the pharmaceutical compound comprising *O-*desmethyl-Tramadol and at least one COX-inhibitor selected from NSAIDs, selected from salicylates, anthranilates, arylacetic acids/arylalkanoic acids, or arylpropionic acids. Other preferred embodiment of the invention are a) a pharmaceutical compound comprising *O-*desmethyl-Tramadol and at least one COX-inhibitor selected from an NSAID being a salicylate; b) a pharmaceutical compound comprising *O*-desmethyl-Tramadol and at least one COX-inhibitor selected from an NSAID being a anthranilate; c) the pharmaceutical compound comprising *O*-desmethyl-Tramadol and at least one COX-inhibitor selected from an NSAID being an arylacetic acid/arylalkanoic acid; or d) a pharmaceutical compound comprising *O-*desmethyl-Tramadol and at least one COX-inhibitor selected from an NSAID being an arylpropionic acid.

Examples of salicylates are: Acetylsalicylic acid, Diflunisal, Ethenzamide, Salicylamide, Triflusal, Fosfosal, and Benorylate. Examples of anthranilates are: Etofenamate, Flufenamic acid, Meclofenamic acid, Mefenamic acid, Niflumic acid, and Tolfenamic acid. Examples of arylacetic acids/arylalkanoic acids are: Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, and Felbinac. Examples of arylpropionic acids are: Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, and Clidanac.

In a further embodiment of the pharmaceutical compound according to the invention the COX-inhibitor or one of the COX-inhibitors is selected from
Acetylsalicylic acid, Diflunisal, Ethenzamide, Salicylamide, Triflusal, Fosfosal, Benorylate, Etofenamate, Flufenamic acid, Meclofenamic acid, Mefenamic acid, Niflumic acid, Tolfenamic acid, Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, Felbinac, Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, and Clidanac; or their stereoisomers, salts or metabolites.

Other examples of NSAIDs are the Coxibs, selective COX-2 inhibitors. Therefore, another preferred embodiment of the invention is a pharmaceutical compound comprising *O*-desmethyl-Tramadol and at least one COX-inhibitor selected from an NSAID being a Coxib. Examples of Coxibs are: Celecoxib, Etoricoxib, Lumiracoxib, Parecoxib, Rofecoxib, Valdecoxib, and Cimicoxib.

Further examples of the COX-inhibitors are para-aminophenol derivatives. Therefore, another preferred embodiment of the invention is a pharmaceutical compound comprising *O*-desmethyl-Tramadol and at least one COX-inhibitor selected from a para-aminophenol derivative. Examples of para-aminophenol derivatives are: Paracetamol, Propacetamol, and Phenidine.

In a further embodiment of the pharmaceutical compound according to the invention the COX-inhibitor or one of the COX-inhibitors is selected from
Acetylsalicylic acid, Diflunisal, Ethenzamide, Salicylamide, Triflusal, Fosfosal, Benorylate, Paracetamol, Propacetamol, Phenidine, Etofenamate, Flufenamic acid, Meclofenamic acid, Mefenamic acid, Niflumic acid, Tolfenamic acid, Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, Felbinac, Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, Clidanac, Metamizol, Propylphenazone, Kebuzone, Mofebutazone, Oxyphenbutazone, Phenylbutazone, Apazone, Isoxicam, Lornoxicam, Piroxicam, Tenoxicam, Ketorolac, Proquazone, Oxaprozine, Ditazole, Etodolac, Meloxicam, Nimesulide, Celecoxib, Etoricoxib, Lumiracoxib, Parecoxib, Rofecoxib, Valdecoxib, Cimicoxib; or their stereoisomers, salts or metabolites;
or
Acetylsalicylic acid, Diflunisal, Ethenzamide, Salicylamide, Triflusal, Fosfosal, Benorylate, Paracetamol, Propacetamol, Phenidine, Etofenamate, Flufenamic acid, Meclofenamic acid, Mefenamic acid, Niflumic acid, Tolfenamic acid, Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, Felbinac, Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, Clidanac, Metamizol, Propylphenazone, Kebuzone, Mofebutazone, Oxyphenbutazone, Phenylbutazone, Apazone, Isoxicam, Lornoxicam, Piroxicam, Tenoxicam, Ketorolac, Proquazone, Oxaprozine, Ditazole, Etodolac, Meloxicam, Nimesulide, Celecoxib, Etoricoxib, Lumiracoxib, Parecoxib, Rofecoxib, Valdecoxib, Cimicoxib, Bermoprofen, Pelubiprofen, Tenosal, Aceneuramic acid, Pirazolac, Xinoprofen, Flobufen, Anirolac, Zoliprofen, Bromfenac, Pemedolac, Dexpemedolac, Bindarit, Romazarit, Fenbufen; or their stereoisomers, salts or metabolites;
preferably is selected from
Acetylsalicylic acid, Triflusal, HTB (2-hydroxy-4-trifluoromethyl benzoic acid), Diflunisal, Meclofenamic acid, Mefenamic acid, Niflumic acid, Flufenamic acid, Diclofenac, Lonazolac, Acemetacin, Indomethacin, Tolmetin, Sulindac, Etodolac, Ketorolac, Flurbiprofen, (*RS*)-Flurbiprofen, (*R*)-Flurbiprofen, Esflurbiprofen, Ibuprofen, (*RS*)-Ibuprofen, (*S*)-(+)-Ibuprofen, Ketoprofen, (*rac*)-Ketoprofen, (*R*)-(-)-Ketoprofen, Bermoprofen, Pelubiprofen, Tenosal, Aceneuramic acid, Pirazolac, Xinoprofen, Flobufen, Anirolac, Zoliprofen, Bromfenac, Pemedolac, Dexpemedolac, Bindarit, Romazarit, Naproxen, (S)-Naproxen, Tiaprofenic acid, Fenbufen, Fenoprofen, Flobufen, Oxaprozin or Paracetamol; their stereoisomers or salts;
more preferably is Diclofenac, Acetylsalicylic acid, Triflusal, Naproxen, (*S*)-Naproxen, (*RS*)-Flurbiprofen (*R*)-Flurbiprofen, Ibuprofen, (*RS*)-Ibuprofen, (*S*)-(+)-Ibuprofen, or Paracetamol; or one of their salts.

In general all of these COX-inhibitors which have at least one stereogenic center are to be understood as being included herein in their racemic form or as diastereoisomers or enantiomers or mixtures thereof.

It was found, surprisingly, that *O*-desmethyl-Tramadol and COX-inhibitors can be combined to form mixed-salts. It was also found, surprisingly, that *O*-desmethyl-Tramadol and COX-inhibitors can be combined to form co-crystals.

Therefore, in one embodiment the pharmaceutical compound according to the present invention is a salt. In another embodiment the pharmaceutical compound according to the present invention is a co-crystal.

The term "salt" of the pharmaceutical compound according to the present invention encompasses also a hydrate or solvate of the pharmaceutical compound.

"Salts" of the pharmaceutical compound according to the present invention are not only surprisingly easily formed and crystallized they also seem to considerable improve the solubility of *O*-desmethyl-Tramadol and of its COX-inhibitor-partner or other properties of it. Also this association of the two active principles (elements) into the same salt exhibits several further advantages. Being linked as ion and counter-ion, they behave as a single chemical entity, thus facilitating the treatments, formulation, dosage etc. In addition to that, with both *O*-desmethyl-Tramadol and COX-inhibitors being active analgesics these mixed salts are highly useful in the treatment of pain, especially also not losing any activity/weight by the addition of pharmacologically useless counterions. In addition, the two active principles are complementing each other in the treatment especially of pain, but possibly also of various other diseases or symptoms. Also the individual dose of the opioid *O*-desmethyl-Tramadol may be lowered, thus allowing a more frequent treatment, still with an equivalent degree of analgesia. Thus, the mixed salts according to the invention do combine a high number of advantages over the state of the art.

Also, the salts of the pharmaceutical compound being a salt according to the invention, should desirably show at least one, preferably more, of the following features
- being active in pain or even more active when compared to 0-desmethyl-Tramadol free base or hydrochloride salt or to the COX-inhibitor; or
- being easily obtainable, or
- being easily crystallized, allowing more flexibility in formulating, or
- being highly soluble allowing good dissolution rates, especially if dissolving in an aqueous physiological surrounding, or
- having as acidic partner of the *O*-desmethyl-Tramadol a molecule having a beneficial pharmacological effect in itself, thus allowing for a highly efficient dose/weight relation of the active principle.

In one embodiment the pharmaceutical compound being a salt according to the present invention being a salt is selected from the group consisting of (*rac*)-*O*-desmethyl-Tramadol - Diclofenac, (+)-*O*-desmethyl-Tramadol - Diclofenac, (*rac*)-*O*-desmethyl-Tramadol - Acetylsalicylic acid, (+)-*O*-desmethyl-Tramadol - Acetylsalicylic acid, (*rac*)-*O*-desmethyl-Tramadol - Triflusal, (+)-*O-*desmethyl-Tramadol - Triflusal, (*rac*)-*O*-desmethyl-Tramadol - (*S*)-Naproxen, (+)-*O*-desmethyl-Tramadol - (*S*)-Naproxen, (-)-*O*-desmethyl-Tramadol - (*S*)-Naproxen, (+)-*O*-desmethyl-Tramadol - (*R*)-Flurbiprofen, (-)-*O*-desmethyl-Tramadol - (*R*)-Flurbiprofen, (*rac*)-*O*-desmethyl-Tramadol - (*rac*)-Flurbiprofen, (+)-*O*-desmethyl-Tramadol - (*S*)-Ibuprofen, or (-)-*O*-desmethyl-Tramadol - (*S*)-Ibuprofen.

In a preferred embodiment of the pharmaceutical compound being a salt according to the present invention the molecular ratio in the salt of *O*-desmethyl-Tramadol to COX-inhibitor is 1:2 to 2:1, preferably is 2:3 to 3:2 or 1:1.

In a further embodiment of the pharmaceutical compound being a salt according to the present invention the salt is a hydrate.

In addition, the possibility to crystallize the salt or hydrate according to the present invention was also demonstrated. Even though also amorphous salts are also an aspect of the current invention, most preferred are crystalline salts. By that way the physico-chemical properties are improved. The formulation of the mixed salt is even easier with a solid to manipulate and an enhanced stability. The solubility, in particular the solubility of *O*-desmethyl-Tramadol - but also in some cases like Naproxen also of the COX-inhibitor - is also greatly augmented.

Therefore, in one embodiment the pharmaceutical compound being a salt according to the present invention is a crystal of the salt or hydrate.

In another embodiment of the pharmaceutical compound being a crystal of the salt according to the present invention the compound is a crystal selected from crystal forms of
- (*rac*)-*O*-desmethyl-Tramadol - Diclofenac (1:1) salt, form A,
- (*rac*)-*O*-desmethyl-Tramadol - Diclofenac (1:1) salt, form B,
- (+)-*O*-desmethyl-Tramadol - Diclofenac-H₂O (1:1:0.7-0.8) salt,
- (*rac*)-*O*-desmethyl-Tramadol - Acetylsalicylic acid (1:1) salt,
- (+)-*O*-desmethyl-Tramadol - Acetylsalicylic acid (1:1) salt, form A,
- (+)-*O*-desmethyl-Tramadol - Acetylsalicylic acid (1:1) salt, form B,
- (*rac*)-*O*-desmethyl-Tramadol - Triflusal (1:1) salt,
- (+)-*O*-desmethyl-Tramadol - Triflusal (1:1) salt,
- (+)-*O*-desmethyl-Tramadol - (*S*)-Naproxen (1:1) salt, form A,
- (+)-*O*-desmethyl-Tramadol - (*S*)-Naproxen (1:1) salt, form B,
- (-)-*O*-desmethyl-Tramadol - (*S*)-Naproxen (1:1) salt, form A,
- (-)-*O*-desmethyl-Tramadol - (*S*)-Naproxen (1:1) salt, form B,
- (*rac*)-*O*-desmethyl-Tramadol - (*S*)-Naproxen (1:1) salt,
- (+)-*O*-desmethyl-Tramadol - (*R*)-Flurbiprofen (1:1) salt,
- (-)-*O*-desmethyl-Tramadol - (*R*)-Flurbiprofen (1:1) salt,
- (+)-*O*-desmethyl-Tramadol - (*S*)-Ibuprofen - H₂O (1:1:0.3) salt,
- (+)-*O*-desmethyl-Tramadol - (*S*)-Ibuprofen (1:1) salt, or
- (-)-*O*-desmethyl-Tramadol - (*S*)-Ibuprofen (1:1) salt.

In one preferred embodiment the pharmaceutical compound according to the present invention is a crystal of a salt of (*rac*)-*O*-desmethyl-Tramadol and Diclofenac with a molecular ratio of 1:1.

In one preferred embodiment, the crystal of a salt of (*rac*)-*O*-desmethyl-Tramadol and Diclofenac with a molecular ratio of 1:1 is present in form A showing a Fourier Transform Infra Red spectrum with absorption bands at 3345.8 (m), 3293.8 (m), 2936.7 (m), 2852.3 (m), 1590.9 (s), 1561.6 (s), 1501.1 (s), 1452.4 (s), 1403.6 (s), 1377.9 (s), 1315.5 (m), 1283.1 (s), 1174.5 (m), 863.6 (m), 773.9 (m), 762.5 (m), 750.0 (m), 737.0 (m), 718.1 (m), 702.5 (m) cm⁻¹. Preferably, the crystal of a salt of (*rac*)-*O*-desmethyl-Tramadol and Diclofenac with a molecular ratio of 1:1 in form A has a triclinic unit cell with the following dimensions:

| | |
|---|---|
| a (Å) | 8.9132(7) |
| b (Å) | 9.0384(8) |
| c (Å) | 17.4577(14) |
| α (°) | 88.769(2) |
| β (°) | 88.979(2) |
| γ (°) | 78.788(2) |

In one preferred embodiment, the crystal of a salt of (*rac*)-*O*-desmethyl-Tramadol and Diclofenac with a molecular ratio of 1:1 1 present in form A shows an X-ray powder diffraction pattern with peaks expressed in d-values in Å at 17.26, 8.83, 8.68, 7.83, 6.91, 6.26, 6.14, 5.80, 5.68, 5.49, 5.40, 4.89, 4.80, 4.42, 4.36, 4.30, 4.15, 3.92, 3.88, 3.79, 3.65, 3.57, 3.49, 3.46, 3.40, 3.26, 3.10, 2.95, 2.80, 2.65, 2.52, and 2.44.

In another preferred embodiment, the crystal of a salt of (*rac*)-*O*-desmethyl-Tramadol and Diclofenac with a molecular ratio of 1:1 1 is present in form B showing a Fourier Transform Infra Red spectrum with absorption bands at 3313.3 (m), 2936.8 (m), 2861.6 (m), 1602.4 (s), 1588.2 (s), 1577.5 (s), 1561.6 (s), 1451.2 (s), 1416.6 (m), 1378.1 (s), 1309.2 (m), 1291.9 (m), 1177.3 (s), 1151.7 (m), 964.7 (m), 781.8 (s), 740.9 (s), 715.7 (m), 656.2 (m), 646.0 (m) cm⁻¹. Preferably, the crystal of a salt of (*rac*)-*O*-desmethyl-Tramadol and Diclofenac with a molecular ratio of 1:1 in form B has a monoclinic unit cell with the following dimensions:

| | |
|---|---|
| a (A) | 15.4.377(11) |
| b (Å) | 8.5862(7) |
| c (A) | 21.9585(16) |
| β (°) | 105.835(2) |

In one preferred embodiment, the crystal of a salt of (*rac*)-*O*-desmethyl-Tramadol and Diclofenac with a molecular ratio of 1:1 present in form B shows an X-ray powder diffraction pattern with peaks expressed in d-values in Å at 10.49, 7.39, 7.02, 6.63, 5.76, 5.60, 5.41, 5.26, 5.15, 4.90, 4.78, 4.54, 4.49, 4.40, 4.31, 4.11, 4.00, 3.83, 3.79, 3.71, 3.66, 3.60, 3.57, 3.49, 3.36, 3.33, 3.25, 3.12, 3.00, 2.82, 2.70, 2.63, 2.52, and 2.43.

In one preferred embodiment the pharmaceutical compound according to the present is a crystal of the hydrate of (+)-*O*-desmethyl-Tramadol, Diclofenac and H₂O with a molecular ratio of 1:1:0.7-0.8. Preferably, the crystal of the hydrate of (+)-*O*-desmethyl-Tramadol, Diclofenac and H₂O with a molecular ratio of 1:1:0.7-0.8 shows a Fourier Transform Infra Red spectrum with absorption bands at 3235.3 (s), 3075.3 (s), 2944.2 (s), 2917.7 (s), 2850.6 (s), 1576.2 (s), 1561.1 (s), 1502.3 (s), 1450.6 (s), 1380.6 (s), 1308.4 (m), 1281.3 (s), 1240.5 (s), 1212.2 (m), 1149.9 (m), 1108.9 (m), 1002.0 (m), 866.4 (s), 748.3 (s), 707.3 (m), 647.8 (m) cm⁻¹. More preferably, the crystal of the hydrate of (+)-*O*-desmethyl-Tramadol, Diclofenac and H₂O with a molecular ratio of 1:1:0.7-0.8 has a triclinic unit cell with the following dimensions:

| | |
|---|---|
| a (Å) | 8.6494(7) |
| b (Å) | 9.2929(8) |
| c (Å) | 10.8666(9) |
| α (°) | 65.489(1) |
| β (°) | 81.350(2) |
| γ (°) | 66.058(1) |

In one preferred embodiment, the crystal of the hydrate of (+)-*O*-desmethyl-Tramadol, Diclofenac and H₂O with a molecular ratio of 1:1:0.7-0.8 shows an X-ray powder diffraction pattern with peaks expressed in d-values in Å at 9.78, 7.89, 7.75, 7.05, 6.97, 5.18, 4.98, 4.87, 4.71, 4.58, 4.24, 4.18, 4.13, 3.95, 3.88, 3.65, 3.45, 3.32, 3.20, 3.04, 2.85, 2.80, and 2.65.

In another preferred embodiment the pharmaceutical compound according to the present invention is a crystal of a salt of (*rac*)-*O*-desmethyl-Tramadol - Acetylsalicylic acid with a molecular ratio of 1:1_{.} Preferably, the crystal of a salt of (*rac*)-*O*-desmethyl-Tramadol - Acetylsalicylic acid with a molecular ratio of 1:1 shows a Fourier Transform Infra Red spectrum with absorption bands at 3374.9 (m, br), 3133.5 (w, br), 2948.9 (w), 2929.2 (w), 1746.8 (s), 1609.7 (s), 1591.6 (s), 1567.7 (s), 1540.9 (w), 1506.4 (w), 1477.0 (m), 1444.7 (s), 1380.8 (s), 1305.8 (s), 1309.1 (w), 1281.4 (m), 1240.1 (s), 1217.6 (s), 1193.0 (s), 1160.4 (w), 1146.9 (w), 1090.7 (w), 1005.4 (s), 917.6 (w), 862.1 (w), 805.7 (m), 758.9 (s) cm⁻¹. More preferably, the crystal of a salt of (*rac*)-*O*-desmethyl-Tramadol - Acetylsalicylic acid with a molecular ratio of 1:1 1 has a monoclinic unit cell with the following dimensions:

| | |
|---|---|
| a (Å) | 17.6939(14) |
| b (Å) | 8.4806(7) |
| c (Å) | 15.5176(12) |
| β (°) | 103.238(1) |

In one preferred embodiment, the crystal of a salt of (*rac*)-*O*-desmethyl-Tramadol - Acetylsalicylic acid with a molecular ratio of 1:1 shows an X-ray powder diffraction pattern with peaks expressed in d-values in Å at 8.62, 7.55, 7.10, 6.54, 5.66, 5.13, 4.76, 4.41, 4.31, 4.25, 4.18, 4.09, 4.01, 3.84, 3.79, 3.70, 3.53, 3.42, 3.25, 3.20, 3.00, 2.91, 2.79, 2.72, 2.59, 2.56, 2.47, 2.42, and 2.36.

In a further preferred embodiment the pharmaceutical compound according to the present invention is a crystal of a salt of (+)-*O*-desmethyl-Tramadol - Acetylsalicylic acid with a molecular ratio of 1:1. In one more preferred embodiment, the crystal of a salt of (+)-*O-*desmethyl-Tramadol - Acetylsalicylic acid with a molecular ratio of 1:1 is present in form A showing a Fourier Transform Infra Red spectrum with absorption bands at 3327.1 (m, br), 2933.4 (m), 1750.2 (s), 1610.0 (s), 1583.0 (s), 1560.2 (s), 1475.0 (m), 1360.5 (s), 1284.8 (s), 1220.1 (s), 1190.5 (s), 1154.4 (m), 1090.7 (m), 1014.2 (w), 959.3 (w), 918.7 (w), 796.0 (m), 760.0 (m) 704.0 (m) cm⁻¹.
In one preferred embodiment, the crystal of a salt of (+)-*O*-desmethyl-Tramadol - Acetylsalicylic acid with a molecular ratio of 1:1 1 present in form A shows an X-ray powder diffraction pattern with peaks expressed in d-values in Å at 13.32, 9.14, 8.34, 7.94, 7.32, 6.61, 6.35, 6.24, 5.87, 5.53, 4.68, 4.56, 4.46, 4.38, 4.16, 3.96, 3.86, 3.68, 3.62, 3.56, 3.51, 3.43, 3.20, 3.17, 3.12, 2.98, 2.85, 2.80, 2.71, 2.57, and 2.48.

In another more preferred embodiment, the crystal of a salt of (+)-*O*-desmethyl-Tramadol - Acetylsalicylic acid with a molecular ratio of 1:1 is present in form B showing a Fourier Transform Infra Red spectrum with absorption bands at 3376.3 (m, br), 2948.4 (m), 2923.9 (m), 1746.0 (s), 1611.2 (m), 1566.9 (m), 1478.6 (m), 1448.1 (m), 1379.7 (m), 1278.7 (m), 1241.0 (m), 1220.1 (m), 1189.6 (s), 1159.8 (m), 1090.2 (m), 1005.3 (m), 915.4 (m), 867.5 (m), 806.2 (m), 788.7 (m), 761.9 (s), 707.0 (m), 6 74.3 (m), 645.3 (m), 539.5 (m) cm⁻¹.
In one preferred embodiment, the crystal of a salt of (+)-*O*-desmethyl-Tramadol - Acetylsalicylic acid with a molecular ratio of 1:1 present in form B shows an X-ray powder diffraction pattern with peaks expressed in d-values in Å at 8.60, 7.60, 7.47, 6.50, 5.89, 5.17, 4.90, 4.68, 4.57, 4.48, 4.30, 4.16, 4.11, 4.05, 3.40, 3.80, 3.73, 3.59, 3.44, 3.30, 3.22, 3.15, 3.07, 2.99, 2.89, 2.77, 2.57, 2.52, 2.42, and 2.37.

In one preferred embodiment the pharmaceutical compound according to the present invention is a crystal of a salt of (*rac*)-*O*-desmethyl-Tramadol - Triflusal with a molecular ratio of 1:1. Preferably, the crystal of a salt of (*rac*)-*O*-desmethyl-Tramadol - Triflusal with a molecular ratio of 1:1 shows a Fourier Transform Infra Red spectrum with absorption bands at 3384.6 (m, br), 3220.5 (m, br), 1770.2 (m), 1617.7 (s), 1594.3 (s), 1440.1 (m), 1410.6 (m), 1371.1 (m), 1330.9 (s), 1277.7 (m), 1234.7 (m), 1214.5 (s), 1195.5 (s), 1168.0 (m), 1155.5 (m), 1131.8 (m), 1105.0 (m), 941.1 (m), 868.1 (m), 706.3 (m) cm⁻¹.
In one preferred embodiment, the crystal of a salt of (*rac*)-*O*-desmethyl-Tramadol - Triflusal with a molecular ratio of 1:1 shows an X-ray powder diffraction pattern with peaks expressed in d-values in Å at 8.66, 7.91, 7.80, 7.27, 6.57, 5.83, 5.30, 4.81, 4.57, 4.47, 4.39, 4.32, 4.21, 4.18, 3.91, 3.87, 3.82, 3.63, 3.49, 3.35, 3.28, 3.21, 3.02, 2.88, 2.81, 2.65, 2.49, and 2.28.

In another preferred embodiment the pharmaceutical compound according to the present invention is a crystal of a salt of (+)-*O*-desmethyl-Tramadol - Triflusal with a molecular ratio of 1:1. Preferably, the crystal of a salt of (+)-*O*-desmethyl-Tramadol - Triflusal with a molecular ratio of 1:1 shows a Fourier Transform Infra Red spectrum with absorption bands at 3375.5 (m, br), 3186.5 (m, br), 2940.0 (m), 1775.5 (s), 1610.6 (s), 1592.3 (s), 1566.6 (s), 1478.6 (m), 1444.6 (s), 1410.6 (m), 1374.4 (s), 1329.2 (s), 1278.7 (m), 1209.1 (s), 1168.2 (s), 1138.9 (s), 1126.0 (s), 1105.7 (s), 1067.5 (m), 1013.1 (m) 1002.9 (m), 943.6 (s), 903.6 (m), 867.0 (m), 859.5 (s), 818.9 (s), 788.8 (m), 758.4 (m), 707.1 (m) cm⁻¹.
In one preferred embodiment, the crystal of a salt of (+)-*O*-desmethyl-Tramadol - Triflusal with a molecular ratio of 1:1 shows an X-Ray powder diffraction pattern with peaks expressed in d-values in Å at 8.90, 8.07, 7.87, 7.00, 6.50, 6.12, 5.57, 5.22, 4.73, 4.46, 4.39, 4.35, 4.18, 4.07, 4.01, 3.97, 3.91, 3.67, 3.56, 3.49, 3.44, 3.39, 3.25, 3.17, 3.13, 3.06, 2.96, 2.78, 2.70, 2.69, 2.63, 2.59, 2.52, 2.48, 2.46, 2.40, and 2.38.

In a further preferred embodiment the pharmaceutical compound according to the present invention is a crystal of a salt of (+)-*O*-desmethyl-Tramadol - (*S*)-Naproxen with a molecular ratio of 1:1. In one more preferred embodiment, the crystal of a salt of (+)-*O*-desmethyl-Tramadol - (*S*)-Naproxen with a molecular ratio of 1:1 1 is present in form A showing a Fourier Transform Infra Red spectrum with absorption bands at 3360.7 (m, br), 2959.4 (m), 2922.8 (m), 2857.0 (m), 1630.6 (m), 1604.8 (s), 1570.2 (s), 1505.2 (m), 1480.7 (s), 1449.8 (s), 1388.6 (s), 1356.8 (s), 1164.4 (m), 1227.1 (s), 1029.2 (m), 926.3 (m), 864.3 (s) 852.2 (m), 786.7 (m), 705.9 (m) cm⁻¹. Preferably, the crystal of a salt of (*rac*)-*O*-desmethyl-Tramadol - (*S*)-Naproxen with a molecular ratio of 1:1 1 in form A has a monoclinic unit cell with the following dimensions:

| | |
|---|---|
| a (Å) | 9.4093(9) |
| b (Å) | 12.4375(11) |
| c (Å) | 11.5172(10) |
| β (°) | 101.423(2) |

In one preferred embodiment, the crystal of a salt of (+)-*O*-desmethyl-Tramadol - (*S*)-Naproxen with a molecular ratio of 1:1 1 present in form A shows an X-ray powder diffraction pattern with peaks expressed in d-values in Å at 11.29, 8.37, 6.71, 6.54, 6.23, 5.80, 5.65, 5.31, 5.16, 4.89, 4.62, 4.44, 4.33, 4.18, 4.05, 3.90, 3.79, 3.68, 3.60, 3.50, 3.36, 3.27, 3.22, 3.16, 3.08, 2.90, 2.81, 2.60, 2.56, 2.51, 2.46, 2.41, and 2.37.

In another more preferred embodiment, the crystal of a salt of (+)-*O*-desmethyl-Tramadol - (*S*)-Naproxen with a molecular ratio of 1:1 1 is present in form B showing a Fourier Transform Infra Red spectrum with absorption bands at 3354.6 (m, br), 3055.8 (m, br), 2938.4 (m), 1603.6 (s), 1479.8 (m), 1390.3 (s), 1359.0 (s), 1263.8 (s), 1113.7 (s), 1164.0 (s), 1118.8 (m), 1030.7 (s), 961.3 (m), 925.3 (m), 888.6 (m), 855.8 (s), 808.5 (m), 780.9 (m), 706.1 (m) cm⁻¹.
In one preferred embodiment, the crystal of a salt of (+)-*O*-desmethyl-Tramadol - (*S*)-Naproxen with a molecular ratio of 1:1 present in form B shows an X-Ray powder diffraction pattern with peaks expressed in d-values in Å at 14.24, 7.88, 7.40, 7.13, 6.68, 6.10, 6.00, 5.61, 4.92, 4.83, 4.74, 4.51, 4.29, 4.22, 4.07, 3.98, 3.83, 3.67, 3.59, 3.39, 3.30, 3.17, 3.09, 3.00, 2.85, 2.75, 2.62, 2.50, and 2.47.

In one preferred embodiment the pharmaceutical compound according to the present invention is a crystal of a salt of (-)-*O*-desmethyl-Tramadol - (*S*)-Naproxen with a molecular ratio of 1:1. In one more preferred embodiment, the crystal of a salt of (-)-*O*-desmethyl-Tramadol - (*S*)-Naproxen with a molecular ratio of 1:1 is present in form A showing a Fourier Transform Infra Red spectrum with absorption bands at 3359.1 (m, br), 3058.7 (m), 3003.7 (m), 2944.3 (m), 2864.2 (w), 1629.4 (m), 1603.9 (s), 1482.3 (m), 1448.1 (s), 1398.7 (s), 1359.8 (m), 1213.5 (s), 1166.5 (s), 1113.4 (s), 1031.9 (s), 1002.3 (m), 924.7 (m), 883.1 (s), 869.4 (s), 809.8 (s), 787.9 (s), 746.2 (m), 704.5, 481.4 (s) cm⁻¹.
In one preferred embodiment, the crystal of a salt of (-)-*O*-desmethyl-Tramadol - (*S*)-Naproxen with a molecular ratio of 1:1 1 present in form A shows an X-ray powder diffraction pattern with peaks expressed in d-values in Å at 11.85, 11.10, 8.67, 8.25, 6.93, 6.18, 5.93, 5.60, 5.34, 5.05, 4.92, 4.73, 4.59, 4.41, 4.33, 4.19, 4.10, 4.00, 3.85, 3.74, 3.68, 3.64, 3.58, 3.47, 3.41, 3.30, 3.22, 3.13, 3.06, 2.96, 2.80, 2.75, 2.45, and 2.39.

In another more preferred embodiment, the crystal of a salt of (-)-*O*-desmethyl-Tramadol - (*S*)-Naproxen with a molecular ratio of 1:1 is present in form B showing a Fourier Transform Infra Red spectrum with absorption bands at 3344.3 (m, br), 2935.3 (m), 2861.6 (w), 1631.1 (m), 1604.9 (s), 1571.8 (s), 1481.1 (m), 1448.3 (s), 1381.6 (s), 1358.0 (s), 1266.4 (s), 1239.8 (s), 1210.8 (s), 1170.8 (m), 1030.1 (s), 926.7 (m), 865.7 (s), 788.7 (m), 705.4 (m), 478.5 (m) cm⁻¹. Preferably, the crystal of a salt of (-)-*O*-desmethyl-Tramadol - (*S*)-Naproxen with a molecular ratio of 1:1 in form B has a monoclinic unit cell with the following dimensions:

| | |
|---|---|
| a (Å) | 9.2616(16) |
| b (Å) | 12.599(2) |
| c (Å) | 11.563(2) |
| β (°) | 102.456(4) |

In one preferred embodiment, the crystal of a salt of (-)-*O*-desmethyl-Tramadol - (*S*)-Naproxen with a molecular ratio of 1:1 present in form B shows an X-ray powder diffraction pattern with peaks expressed in d-values in Å at 11.19, 9.09, 8.41, 7.97, 7.37, 6.73, 6.42, 6.30, 5.72, 5.66, 5.51, 5.35, 5.17, 4.92, 4.53, 4.39, 4.16, 4.15, 4.08, 3.94, 3.80, 3.71, 3.61, 3.52, 3.37, 3.33, 3.24, 3.16, 3.11, 3.03, 2.93, 2.88, 2.83, 2.75, 2.72, 2.62, 2.56, 2.49, 2.45, and 2.43.

In one preferred embodiment the pharmaceutical compound according to the present invention is a crystal of a salt of (*rac*)-*O*-desmethyl-Tramadol - (*S*)-Naproxen with a molecular ratio of 1:1. Preferably, the crystal of a salt of (*rac*)-*O*-desmethyl-Tramadol - (*S*)-Naproxen with a molecular ratio of 1:1 shows a Fourier Transform Infra Red spectrum with absorption bands at 3221.5 (m, br), 2938.9 (m), 2855.7 (m), 1632.0 (m), 1604.0 (s), 1576.3 (s), 1503.6 (m), 1482.0 (m), 1448.8 (s), 1390.0 (s), 1359.9 (s), 1268.1 (s), 1230.0 (s), 1216.2 (s), 1170.8 (m), 1031.3 (m), 1002.0 (m), 926.5 (m), 864.4 (m) 851.6 (m), 791.0 (m), 706.2 (m), 476.4 (m) cm⁻¹.
In one preferred embodiment, the crystal of a salt of (*rac*)-*O*-desmethyl-Tramadol - (*S*)-Naproxen with a molecular ratio of 1:1 shows an X-ray powder diffraction pattern with peaks expressed in d-values in Å at 11.50, 9.67, 8.93, 8.55, 7.39, 6.38, 6.02, 5.76, 5.59, 5.24, 5.19, 5.05, 4.83, 4.63, 4.46, 4.35, 4.24, 4.05, 3.99, 3.84, 3.68, 3.60, 3.39, 3.36, 3.28, 3.19, 3.08, 2.96, 2.92, 2.69, 2.48, and 2.44.

In another preferred embodiment the pharmaceutical compound according to the present invention is a crystal of a salt of (+)-*O*-desmethyl-Tramadol - (*R*)-Flurbiprofen with a molecular ratio of 1:1. Preferably, the crystal of a salt of (+)-*O*-desmethyl-Tramadol - (R)-Flurbiprofen with a molecular ratio of 1:1 shows a Fourier Transform Infra Red spectrum with absorption bands at 3395.6 (br); 2931.0 (m); 2853.7 (w); 1571.0 (s); 1482.5 (s); 1448.1 (s); 1415.3 (m); 1387.7 (s); 1358.1 (s); 1318.9 (m); 1279.0 (s); 1238.4 (m); 1208.2 (m); 1171.9 (m); 1131.8 (m), 1109.0 (w); 1093.0 (w); 999.5 (w); 964.5 (m); 861.7 (m); 703.9 (m) cm⁻¹. More preferably, the crystal of a salt of (+)-*O*-desmethyl-Tramadol - (*R*)-Flurbiprofen with a molecular ratio of 1:1 has an orthorhombic unit cell with the following dimensions:

| | |
|---|---|
| a (Å) | 8.3463(5) |
| b (Å) | 12.5585(8) |
| c (Å) | 26.0291(17) |

In one preferred embodiment, the crystal of a salt of (+)-*O*-desmethyl-Tramadol - (*R*)-Flurbiprofen with a molecular ratio of 1:1 shows an X-ray powder diffraction pattern with peaks expressed in d-values in Å at 13.00, 11.29, 9.03, 7.94, 6.95, 6.71, 6.13, 5.78, 5.66, 5.43, 5.08, 4.93, 4.75, 4.68, 4.41, 4.35, 4.17, 3.98, 3.70, 3.61, 3.52, 3.44, 3.28, 3.16, and 2.99.

In one preferred embodiment the pharmaceutical compound according to the present invention is a crystal of a salt of (-)-*O*-desmethyl-Tramadol - (*R*)-Flurbiprofen with a molecular ratio of 1:1. Preferably, the crystal of a salt of (-)-*O*-desmethyl-Tramadol - (*R*)-Flurbiprofen with a molecular ratio of 1:1 shows a Fourier Transform Infra Red spectrum with absorption bands at 3415.3 (m br); 2972.3 (w), 2932.5 (m), 2853.0 (w), 1570.3 (s); 1482.1 (s); 1451.2 (s); 1415.1 (s), 1389.5 (s), 1358.8 (s), 1274.3 (s), 1242.4 (m), 1207.1 (m), 1772.0 (m), 1131.6 (m), 1109.5 (m), 1000.8 (m), 927.7 (m), 868.2 (m), 790.3 (m), 770.0 (m), 731.8 (m), 705.4 (m) cm⁻¹.
In one preferred embodiment, the crystal of a salt of (-)-*O*-desmethyl-Tramadol *-* (*R*)-Flurbiprofen with a molecular ratio of 1:1 shows an X-ray powder diffraction pattern with peaks expressed in d-values in Å at 11.54, 11.02, 8.49, 7.42, 7.17, 6.91, 6.55, 6.40, 6.13, 6.04, 5.77, 5.67, 5.50, 5.24, 5.16, 5.06, 4.99, 4.72, 4.57, 4.42, 4.37, 4.29, 4.10, 3.92, 3.87, 3.76, 3.66, 3.51, 3.45, 3.33, 3.25, 3.20, 3.02, 2.97, 2.88, 2.63, 2.58, and 2.47.

In one preferred embodiment the pharmaceutical compound according to the present invention is a crystal of a hydrate of (+)-*O*-desmethyl-Tramadol - (*S*)-Ibuprofen - H₂O with a molecular ratio of 1:1:0.3. Preferably, the crystal of a hydrate of (+)-*O*-desmethyl-Tramadol - (*S*)-Ibuprofen - H₂O with a molecular ratio of 1:1:0.3 shows a Fourier Transform Infra Red spectrum with absorption bands at 3403.9 (s, br), 2955.0 (s), 2934.0 (s), 2866.3 (m), 1591.8 (s), 1479.5 (s), 1457.1I (s), 1388.9 (s), 1355.8 (s), 1278.5 (s), 1251.6 (s), 1136.3 (s), 1002.3 (s), 989.4 (s), 880.9 (m), 863.6 (s), 790.2 (s), 706.1 (s) cm⁻¹. More preferably, the crystal of a hydrate of (+)-*O*-desmethyl-Tramadol - (*S*)-Ibuprofen - H₂O with a molecular ratio of 1:1:0.3 has a monoclinic unit cell with the following dimensions:

| | |
|---|---|
| a (Å) | 10.7295(15) |
| b (Å) | 10.0318(15) |
| c (Å) | 12.9665(18) |
| β (°) | 98.776(3) |

In one preferred embodiment, the crystal of a hydrate of (+)-*O*-desmethyl-Tramadol - (*S*)-Ibuprofen - H₂O with a molecular ratio of 1:1:0.3 shows an X-ray powder diffraction pattern with peaks expressed in d-values in Å at 12.85, 10.66, 8.89, 7.93, 7.32, 6.09, 5.90, 5.41, 5.31, 5.15, 5.04, 4. 70, 4.62, 4.39, 4.19, 4.06, 3.97, 3.87, 3.83, 3.65, 3.53, 3.40, 3.33, 3.21, 3.05, 2.93, 2.82, and 2.54.

In another preferred embodiment the pharmaceutical compound according to the present invention is a crystal of a salt of (+)-*O*-desmethyl-Tramadol - (*S*)-Ibuprofen with a molecular ratio of 1:1. Preferably, the crystal of a salt of (+)-*O*-desmethyl-Tramadol - (*S*)-Ibuprofen with a molecular ratio of 1:1 shows a Fourier Transform Infra Red spectrum with absorption bands at 3394.8 (br), 2953.8 (s), 2930.1 (s), 2867.7 (m), 1568.0 (s), 1480.9 (m), 1448.4 (s), 1389.3 (s), 1357.6 (m), 1278.9 (s), 1244.0 (m), 1170.5 (m), 999.6 (m), 864.5 (m), 787.2 (m), 705.1 (m) cm⁻¹.
In one preferred embodiment, the crystal of a salt of (+)-*O*-desmethyl-Tramadol - (*S*)-Ibuprofen with a molecular ratio of 1:1 shows an X-ray powder diffraction pattern with peaks expressed in d-values in Å at 11.35, 8.43, 7.06, 6.37, 6.18, 5.68, 5.25, 5.17, 4.94, 4.81, 4.74, 4.67, 4.41, 4.23, 4.16, 3.95, 3.68, 3.53, 3.44, 3.39, 3.18, 3.03, and 2.68.

In one preferred embodiment the pharmaceutical compound according to the present invention is a crystal of a salt of (-)-*O*-desmethyl-Tramadol - (*S*)-Ibuprofen with a molecular ratio of 1:1. Preferably, the crystal of a salt of (-)-*O*-desmethyl-Tramadol - (*S*)-Ibuprofen with a molecular ratio of 1:1 shows a Fourier Transform Infra Red spectrum with absorption bands at 3359.4 (s, br), 3086.5 (m, br), 3025.7 (m), 2984.5 (m), 2956.3 (s), 2929.7 (s), 2869.4 (m), 1579.4 (br, s), 1510.0 (s), 1482.9 (s), 1447.2 (s), 1411.8 (s), 1389.6 (s), 1357.5 (s), 1277.7 (m), 1240.4 (m), 1209.3 (m), 1170.1 (m), 1108.8 (m), 1000.7 (s), 877.3 (m), 867.1 (s), 860.8 (s), 790.9 (m), 722.5 (m), 705.0 (m), 681.4 (m), 540.7 (m), 453.3 (m) cm⁻¹. More preferably, the crystal of a salt of (-)-*O-*desmethyl-Tramadol - (*S*)-Ibuprofen with a molecular ratio of 1:1 has a monoclinic unit cell with the following dimensions:

| | |
|---|---|
| a (Å) | 8.9646(10) |
| b (Å) | 12.9258(14) |
| c (Å) | 11.3901(13) |
| β (°) | 93.959(2) |

In one preferred embodiment, the crystal of a salt of (-)-*O*-desmethyl-Tramadol - (*S*)-Ibuprofen with a molecular ratio of 1:1 shows an X-ray powder diffraction pattern with peaks expressed in d-values in Å at 11.35, 8.54, 7.36, 6.35, 6.02, 5.68, 5.25, 5.20, 4.96, 4.84, 4.65, 4.48, 4.38, 4.27, 4.23, 4.03, 3.94, 3.89, 3.78, 3.71, 3.64, 3.58, 3.51, 3.44, 3.40, 3.29, 3.27, 3.24, 3.18, 3.13, 3.01, 2.96, 2.92, 2.84, 2.81, 2.78, 2.74, 2.66, 2.60, 2.57, 2.54, 2.53, 2.46, 2.42, 2.35, 2.29, and 2.28.

Beside the ability to form salts, it was further found that *O*-desmethyl-Tramadol was able to form co-crystals with COX-inhibitors. These co-crystals show improved properties if compared to *O*-desmethyl-Tramadol alone, and also good analgesic activity. The co-crystals thus obtained have a specific stoichiometry which depends upon the structure of each COX-inhibitor. Under the proper circumstance this is also another advantage of these new solid drugable forms possibly achieving some modulation of the pharmacological effects. While APIs (Active Pharmaceutical Ingredients) like *O*-desmethyl-Tramadol in general have been recognized to form crystalline polymorphs, solvates, hydrates and amorphous forms for a number of years, there is little knowledge about which APIs will form co-crystals. Co-crystals are a specific type of crystalline form which provide a new avenue to modulate the API form and thus to modulate API properties. Co-crystals contain an API and at least one other component which crystallize together. Selection of the other component helps determine whether a co-crystal will form and what properties the co-crystal will have. Just as a polymorph, solvate, hydrate or amorphous form of an API can modulate stability, solubility, and hygroscopicity, a co-crystal can modulate those same properties.

Thus, in another embodiment the pharmaceutical compound according to the present invention is a co-crystal.

"Co-crystal" as used herein is defined as a crystalline material comprising two or more compounds that are solid at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and π-π interactions. Solvates of *O*-desmethyl-Tramadol that do not further comprise a co-crystal former are not co-crystals according to the present invention. The co-crystals may however, include one or more solvate molecules in the crystal lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, CrystEngComm, 2003, 5(82), 466-467 and Dunitz, CrystEngComm, 2003, 5(91), 506). A recent article by Zaworotko (M. J. Zaworotko, Crystal Growth & Design, Vol. 7, No. 1, 2007, 4-9) gives a definition of co-crystal which is in line with the definition given above and thus also is a definition of "co-crystal" according to this invention. According to this article *"a co-crystal is a multiple component crystal in which all components are solid under ambient conditions when in their pure form. These components consist of a target molecule or ion and a molecular co-crystal former(s); when in a co-crystal, they coexist at the molecular level within a single crystal."*

"Co-crystal former" as used herein is defined as a molecule being an active agent selected from COX-inhibitor, and with which *O*-desmethyl-Tramadol is able to form co-crystals.

In a preferred embodiment of the pharmaceutical compound according to the invention being a co-crystal the COX-inhibitor is/are chosen in such a way that if compared to either *O*-desmethyl-Tramadol alone or to a mixture of *O*-desmethyl-Tramadol and the corresponding active agent/s
- the solubility of the co-crystal is increased; and/or
- the dose response of the co-crystal is increased; and/or
- the efficacy of the co-crystal is increased; and/or
- the dissolution of the co-crystal is increased; and/or
- the bioavailability of the co-crystal is increased; and/or
- the stability of the co-crystal is increased; and/or
- the hygroscopicity of the co-crystal is decreased; and/or
- the form diversity of the co-crystal is decreased; and/or
- the morphology of the co-crystal is modulated.

"Mixture of *O*-desmethyl-Tramadol and the corresponding active agent/s" is defined as a mixture of the active agent or agents in question (COX-inhibitors) with *O*-desmethyl-Tramadol which is only a physical mixture without any coupling forces between the compounds and thus neither includes salts nor co-crystals.

In one preferred embodiment the pharmaceutical compound according to the present invention being a co-crystal comprises *O*-desmethyl-Tramadol as a free base or as its pharmaceutically acceptable salt and at least one COX-inhibitor.

As illustrated in more detail below *O*-desmethyl-Tramadol as its racemate and its enantiomers (+)-*O*-desmethyl-Tramadol and (-)-*O*-desmethyl-Tramadol form co-crystals with COX-inhibitors. Generally the co-crystals obtained have a specific stoichiometry.

In one embodiment of the pharmaceutical compound according to the present invention being a co-crystal the molecular ratio in the co-crystal of *O*-desmethyl-Tramadol to COX-inhibitor is 1:2 to 2:1, preferably is 2:3 to 3:2 or 1:1.

In a further preferred embodiment the pharmaceutical compound according to the present invention being a co-crystal is selected from
- a co-crystal comprising (*rac*)-*O*-desmethyl-Tramadol either as a free base or as its physiologically acceptable salt and at least one COX-inhibitor;
- a co-crystal comprising (-)-*O*-desmethyl-Tramadol either as a free base or as its physiologically acceptable salt and at least one COX-inhibitor;
- a co-crystal comprising (+)-*O*-desmethyl-Tramadol either as a free base or as its physiologically acceptable salt and at least one COX-inhibitor;
- an enantiomeric mixture of co-crystals comprising (-)-*O*-desmethyl-Tramadol either as a free base or as its physiologically acceptable salt and at least one COX-inhibitor and co-crystals comprising (+)-*O*-desmethyl-Tramadol either as a free base or as its physiologically acceptable salt and at least one COX-inhibitor; or
- any of the co-crystals above being solvate co-crystals, preferably being hydrate or alcoholate co-crystals, also being monohydrate co-crystals.

In a more preferred embodiment the pharmaceutical compound according to the present invention is a co-crystal selected from (*rac*)-*O*-desmethyl-Tramadol - (*rac*)-Flurbiprofen, (*rac*)-*O-*desmethyl-Tramadol - (*S*)-Ibuprofen or (*rac*)-*O*-desmethyl-Tramadol·HCl - Paracetamol, preferably is selected from (*rac*)-*O*-desmethyl-Tramadol - (*rac*)-Flurbiprofen (2:3), (*rac*)-*O-*desmethyl-Tramadol - (*S*)-Ibuprofen (2:3) co-crystal or (*rac*)-*O*-desmethyl-Tramadol·HCl - Paracetamol (1:1) co-crystal.

In one preferred embodiment the pharmaceutical compound according to the present invention is a co-crystal of (*rac*)-*O*-desmethyl-Tramadol - (*rac*)-Flurbiprofen with a molecular ratio of 2:3. Preferably, the co-crystal of (*rac*)-*O*-desmethyl-Tramadol - (*rac*)-Flurbiprofen with a molecular ratio of 2:3 shows a Fourier Transform Infra Red spectrum with absorption bands at 3240.4 (m br), 2976.0 (w), 2941.0 (m), 2856.5 (w), 1723.2 (m), 1575.6 (m), 1482.8 (s), 1455.8 (s), 1446.4 (s), 1388.7 (s), 1361.2 (s), 1279.0 (s), 1229.0 (s), 1132.3 (m), 925.2 (m), 765.8 (m), 696.8 (s) cm⁻¹.
In one preferred embodiment, the co-crystal of (*rac*)-*O*-desmethyl-Tramadol - (*rac*)-Flurbiprofen with a molecular ratio of 2:3 shows an X-ray powder diffraction pattern with peaks expressed in d-values in Å at 14.18, 9.57, 9.18, 7.24, 7.11, 6.84, 6.33, 6.20, 5.97, 5.77, 5.40, 5.33, 5.19, 4.99, 4.91, 4.86, 4.78, 4.57, 4.52, 4.42, 4.26, 4.18, 4.04, 3.88, 3.82, 3.70, 3.65, 3.56, 3.45, 3.37, 3.30, 3.15, 3.10, 3.06, 2.97, 2.93, 2.84, 2.69, 2.65, 2.49, 2.46, 2.36, and 2.33.

In one preferred embodiment the pharmaceutical compound according to the invention is a co-crystal of (*rac*)-*O*-desmethyl-Tramadol and (*S*)-Ibuprofen with a molecular ratio of 2:3. Preferably, the co-crystal of (*rac*)-*O*-desmethyl-Tramadol and (*S*)-Ibuprofen with a molecular ratio of 2:3 shows a Fourier Transform Infra Red spectrum with absorption bands at 3251.9 (m, br), 2943.7 (m), 1719.2 (s), 1579.3 (s), 1204.5 (m), 1175.7 (m), 1110.7 (m), 1062.8 (m), 1001.5 (s), 966.5 (m), 879.5 (m), 864.1 (s), 790.4 (s), 706.4 (s), 668.1 (m), 633.8 (m), 596.8 (m), 466.6 (m) cm⁻¹. More preferably, the co-crystal of (*rac*)-*O*-desmethyl-Tramadol and (*S*)-Ibuprofen with a molecular ratio of 2:3 has an orthorhombic unit cell with the following dimensions:

| | |
|---|---|
| a (Å) | 12.6916(11) |
| b (Å) | 18.0869(15) |
| c (Å) | 28.729(3) |

In one preferred embodiment, the co-crystal of a salt of (*rac*)-*O*-desmethyl-Tramadol and Ibuprofen with a molecular ratio of 2:3 shows an X-ray powder diffraction pattern with peaks expressed in d-values in Å at 14.34, 11.61, 9.79, 9.51, 9.07, 8.44, 7.65, 7.04, 6.57, 6.25, 5.85, 5.63, 5.20, 5.10, 5.03, 4.85, 4.74, 4.53, 4.48, 4.35, 4.15, 4.07, 3.96, 3.83, 3.79, 3.74, 3.71, 3.60, 3.52, 3.48, 3.42, 3.39, 3.19, 3.13, 2.74 and 2.53.

In another preferred embodiment the pharmaceutical compound according to the invention is a co-crystal of (*rac*)-*O*-desmethyl-Tramadol·HCl and Paracetamol with a molecular ratio of 1:1. Preferably, the co-crystal of (*rac*)-*O*-desmethyl-Tramadol·HCl and Paracetamol with a molecular ratio of 1:1 shows a Fourier Transform Infra Red spectrum with absorption bands at 3308.5 (s, br), 3216.8 (s, br), 2930.9 (m), 2861.9 (m), 2677.3 (m, br), 1653.6 (s), 1610.2 (m), 1582.4 (s), 1537.5 (s), 1511.9 (s), 1263.6 (s), 1239.3 (m), 1212.8 (m), 1177.7 (m), 1162.3 (m), 1132.5 (m), 986.9 (m), 792.0 (s), 706.3 (m), 523.0 (m), 503.5 (m) cm⁻¹. More preferably, the co-crystal of (*rac*)-*O-*desmethyl-Tramadol·HCl and Paracetamol with a molecular ratio of 1:1 has a monoclinic unit cell with the following dimensions:

| | |
|---|---|
| a (Å) | 23.538(3) |
| b (Å) | 10.9852(14) |
| c (Å) | 9.2583(12) |
| β (°) | 100.862(3) |

In one preferred embodiment, the co-crystal of a salt of (*rac*)-*O*-desmethyl-Tramadol·HCl and Paracetamol with a molecular ratio of 1:1 shows an X-ray powder diffraction pattern with peaks expressed in d-values in Å at 11.47, 9.89, 6.96, 6.43, 5.77, 5.59, 5.48, 4.95, 4.83, 4.70, 4.50, 4.26, 4.14, 3.95, 3.83, 3.62, 3.50, 3.42, 3.32, 3.29, 3.20, 3.02, 2.96, 2.87, 2.83, 2.74, 2.70, 2.58, 2.48, 2.35, and 2.31.

In another aspect the present invention relates to a process for the production of a pharmaceutical compound according to the present invention, wherein the compound is a co-crystal, comprising the steps of:
a) dissolving or suspending a COX-inhibitor and *O*-desmethyl-Tramadol in a solvent or mixture of solvents;
b) mixing or stirring the solution or suspension;
c) optionally before, during or after step (b) cooling or keeping the mixed solution/suspension to or at ambient temperature or below;
d) filtering-off and washing the resulting solid with a solvent; and
e) drying the co-crystals at ambient temperature under vacuum.

"Ambient temperature" is defined here as a temperature between 20 and 25°C, preferably being 20°C. Preferably the cooling in step (c) is done from ambient temperature to approximately 0-5°C.

The solvents usable in this process include water or organic solvents, preferably solvents selected from dichloromethane, methanol, acetone, isobutyl acetate, acetonitrile, ethyl acetate, 2-butanol, dimethylcarbonate, chlorobenzene, butylether, diisopropylether, dimethylformamide, ethanol, hexane, isopropanol, methyl ethyl ketone, methyl isobutyl ketone, methyl t-butyl ether, 3 pentanone, toluene and 1,1,1-trichloroethane, most preferably selected from dichloromethane, toluene, isobutyl acetate and methanol.

In a further aspect the present invention relates to one optional process for the production of a pharmaceutical compound according to the present invention, wherein the compound is a salt or hydrate of a salt, comprising the steps of:
a) dissolving or suspending a COX-inhibitor and *O*-desmethyl-Tramadol in a solvent or mixture of solvents;
b) optionally mixing or stirring the solution or suspenssion;
c) optionally before, during or after step (b) cooling or keeping the mixed solution/suspension to or at ambient temperature or below;
d) optionally filtering-off and/or washing the resulting solid with a solvent; and
e) drying the solid optionally at ambient temperature and/or under vacuum.

"Ambient temperature" is defined here as a temperature between 20 and 25°C, preferably being 20°C. Preferably the cooling in step (c) is done from ambient temperature to approximately 0-5°C.

The solvents usable in this process include water and/or organic solvents, preferably solvents selected from dichloromethane, chloroform, methanol, acetone, isobutyl acetate, butyl acetate, acetonitrile, ethyl acetate, 2-butanol, dimethylcarbonate, chlorobenzene, butylether, diisopropylether, dimethylformamide, ethanol, hexane, methyl ethyl ketone, methyl isobutyl ketone, methyl t-butyl ether, 3 pentanone, toluene, tetrahydrofuran, and 1,1,1-trichloroethane, diethyl ether, and/or isopropyl alcohol.

In one embodiment of the process for the production of a pharmaceutical compound, wherein the compound is a salt or hydrate the ratio of *O*-desmethyl-Tramadol to COX-inhibitor in step a) is 1:3 to 3:1, preferably is 1:1.

The elements of the pharmaceutical compound according to the invention are well-known drugs with analgesic properties sometimes used for a long time worldwide. Due to this it is another aspect the present invention relates to a medicament comprising at least one pharmaceutical compound according to the present invention and optionally one or more pharmaceutically acceptable excipients.

In a further aspect the present invention concerns a pharmaceutical composition comprising a therapeutically effective amount of the pharmaceutical compound according to the present invention, of the crystal of a salt according to the present invention or co-crystal according to the present invention in a physiologically acceptable medium. Physiologically acceptable medium is defined as especially comprising pharmaceutically acceptable auxiliary substances (additives/excipients), especially those suitable for a solid pharmaceutical formulation, of which one or more may be part of the pharmaceutical formulation.

The association of two active elements in the same pharmaceutical compound, such as a salt or co-crystal, exhibits several advantages. Being linked, the elements often behave as a single chemical entity, thus facilitating the treatments, formulation, dosage etc. In addition to that, with both *O*-desmethyl-Tramadol and COX-inhibitor being active analgesics these pharmaceutical compounds are highly useful in the treatment of pain, especially also not losing any activity/weight by the addition of pharmacologically useless counterions as in salts with no API. In addition the two active elements are complementing each other in the treatment especially of pain, but possibly also of various other diseases or symptoms. Thus, the pharmaceutical compounds according to the invention do combine a high number of advantages over the state of the art.

Another advantage is that the association of two active elements into one unique species seems to allow for a better Pharmacokinetic/Pharmacodynamic (PKPD) including also a better penetration of the blood-brain barrier, which helps in the treatment of pain.

In general in most embodiments in which the pharmaceutical compound of *O*-desmethyl-Tramadol and COX-inhibitors are used (e.g. for the treatment of pain) these compounds would be formulated into a convenient pharmaceutical formulation or a medicament. Accordingly a desirable advantage of a pharmaceutical compound according to the present invention would show improved pharmaceutical properties and features, especially when compared to the free base or Tramadol hydrochloride. Thus, the pharmaceutical compound according to the invention should desirably show at least one, preferably more, of the following features:
- to have a very small particle size, e.g. from 300 µm or lower; or
- to be and/or remain essentially free of agglomerates; or
- to be less or not very hygroscopic; or
- to help in formulating controlled release or immediate release formulations; or
- to have a high chemical stability; or
if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parenterally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) and Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the pharmaceutical compound and 40-99 % by weight of one or more auxiliary substances (additives/excipients).

The compositions of the present invention may also be administered topically or via a suppository.

The daily dosage for humans and animals may vary depending on factors that have their basis in the respective species or other factors, such as age, sex, weight or degree of illness and so forth. The daily dosage for humans of the pharmaceutical compound according to the present invention preferably is in the range of 1 to 2000 mg, preferably 5 to 500 milligrams being administered during one or several intakes per day.

In a further aspect the present invention relates to the use of a pharmaceutical compound according to the present invention for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia. Preferably this use is provided in the form of a medicament or a pharmaceutical composition according to the invention as described above.

"Pain" is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

According to the IASP "allodynia" is defined as "a pain due to a stimulus which does not normally provoke pain" (IASP, Classification of chronic pain, 2nd Edition, IASP Press (2002), 210). Even though the symptoms of allodynia are most likely associated as symptoms of neuropathic pain this is not necessarily the case so that there are symptoms of allodynia not connected to neuropathic pain though rendering allodynia in some areas broader than neuropathic pain.

The IASP further draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

According to the IASP "neuropathy" is defined as "a primary lesion or dysfunction in the nervous system" (IASP, Classification of chronic pain, 2nd Edition, IASP Press (2002), 211). Neuropathic pain may have central or peripheral origin.

A related further aspect of the invention is aimed at the use of a salt according to the invention as described above or of a crystalline form of at least one salt according to the invention as described above in the manufacture of a medicament for the treatment of pain, preferably acute pain, chronic pain, neuropathic pain, hyperalgesia, allodynia or cancer pain, including diabetic neuropathy, fibromyalgia or osteoarthritis. Preferably this use is provided for in form of a medicament or a pharmaceutical composition according to the invention as described above.

Another object of the current invention is a method of treatment of pain, preferably acute pain, chronic pain, neuropathic pain, hyperalgesia, allodynia or cancer pain, including diabetic neuropathy or osteoarthritis or fibromyalgia, by providing to a patient in need thereof a sufficient amount of a co-crystal according to the invention as described above. Preferably the co-crystal according to the invention is provided in physiologically suitable form like e.g. in the form of a medicament or a pharmaceutical composition according to the invention as described above.

The present invention is illustrated below with the help of the following figures and examples. These illustrations are given solely by way of example and do not limit the invention.

### Brief description of the figures

Figure 1: DSC analysis of form A (*rac*)-*O*-desmethyl-Tramadol - Diclofenac (1:1) salt
Figure 2: XRPD analysis of form A (*rac*)-*O*-desmethyl-Tramadol - Diclofenac (1:1) salt
Figure 3: Crystal structure of form A (*rac*)-*O*-desmethyl-Tramadol - Diclofenac (1:1) salt

Figure 4: DSC analysis of form B (*rac*)-*O*-desmethyl-Tramadol - Diclofenac (1:1) salt
Figure 5: TG analysis of form B (*rac*)-*O*-desmethyl-Tramadol - Diclofenac (1:1) salt
Figure 6: XRPD analysis of form B (*rac*)-*O*-desmethyl-Tramadol - Diclofenac (1:1) salt
Figure 7: Crystal structure of form B (*rac*)-*O*-desmethyl-Tramadol - Diclofenac (1:1) salt

Figure 8: DSC analysis of (+)-*O*-desmethyl-Tramadol - Diclofenac-H₂O (1:1:0.7-0.8) salt
Figure 9: TG analysis of (+)-*O*-desmethyl-Tramadol - Diclofenac-H₂O (1:1:0.7-0.8) salt
Figure 10: XRPD analysis of (+)-*O*-desmethyl-Tramadol - Diclofenac-H₂O (1:1:0.7-0.8) salt
Figure 11: Crystal structure of (+)-*O*-desmethyl-Tramadol - Diclofenac-H₂O (1:1:0.7-0.8) salt

Figure 12: DSC analysis of (*rac*)-*O*-desmethyl-Tramadol - Acetylsalicylic acid (1:1) salt
Figure 13: TG analysis of (*rac*)-*O*-desmethyl-Tramadol - Acetylsalicylic acid (1:1) salt
Figure 14: XRPD analysis of (*rac*)-*O*-desmethyl-Tramadol - Acetylsalicylic acid (1:1) salt
Figure 15: Crystal structure of (*rac*)-*O*-desmethyl-Tramadol - Acetylsalicylic acid (1:1) salt

Figure 16: DSC analysis of form A (+)-*O*-desmethyl-Tramadol - Acetylsalicylic acid (1:1) salt
Figure 17: TG analysis of form A (+)-*O*-desmethyl-Tramadol - Acetylsalicylic acid (1:1) salt
Figure 18: XRPD analysis of form A (+)-*O*-desmethyl-Tramadol - Acetylsalicylic acid (1:1) salt

Figure 19: DSC analysis of form B (+)-*O*-desmethyl-Tramadol - Acetylsalicylic acid (1:1) salt
Figure 20: TG analysis of form B (+)-*O*-desmethyl-Tramadol - Acetylsalicylic acid (1:1) salt
Figure 21: XRPD analysis of form B (+)-*O*-desmethyl-Tramadol - Acetylsalicylic acid (1:1) salt

Figure 22: DSC analysis of (*rac*)-*O*-desmethyl-Tramadol - Triflusal (1:1) salt
Figure 23: TG analysis of (*rac*)-*O*-desmethyl-Tramadol - Triflusal (1:1) salt
Figure 24: XRPD analysis of (*rac*)-*O*-desmethyl-Tramadol - Triflusal (1:1) salt

Figure 25: DSC analysis of (+)-*O*-desmethyl-Tramadol - Triflusal (1:1) salt
Figure 26: TG analysis of (+)-*O*-desmethyl-Tramadol - Triflusal (1:1) salt
Figure 27: XRPD analysis of (+)-*O*-desmethyl-Tramadol - Triflusal (1:1) salt

Figure 28: DSC analysis of form A (+)-*O*-desmethyl-Tramadol - (*S*)-Naproxen (1:1) salt
Figure 29: TG analysis of form A (+)-*O*-desmethyl-Tramadol - (*S*)-Naproxen (1:1) salt
Figure 30: XRPD analysis of form A (+)-*O*-desmethyl-Tramadol - (*S*)-Naproxen (1:1) salt
Figure 31: Crystal structure of form A (+)-*O*-desmethyl-Tramadol - (*S*)-Naproxen (1:1) salt

Figure 32: DSC analysis of form B (+)-*O*-desmethyl-Tramadol - (*S*)-Naproxen (1:1) salt
Figure 33: TG analysis of form B (+)-*O*-desmethyl-Tramadol - (*S*)-Naproxen (1:1) salt
Figure 34: XRPD analysis of form B (+)-*O*-desmethyl-Tramadol - (*S*)-Naproxen (1:1) salt

Figure 35: DSC analysis of form A (-)-*O*-desmethyl-Tramadol - (*S*)-Naproxen (1:1) salt
Figure 36: TG analysis of form A (-)-*O*-desmethyl-Tramadol - (*S*)-Naproxen (1:1) salt
Figure 37: XRPD analysis of form A (-)-*O*-desmethyl-Tramadol - (*S*)-Naproxen (1:1) salt

Figure 38: DSC analysis of form B (-)-*O*-desmethyl-Tramadol - (*S*)-Naproxen (1:1) salt
Figure 39: TG analysis of form B (-)-*O*-desmethyl-Tramadol - (*S*)-Naproxen (1:1) salt
Figure 40: XRPD analysis of form B (-)-*O*-desmethyl-Tramadol - (*S*)-Naproxen (1:1) salt
Figure 41: Crystal structure of form A (-)-*O*-desmethyl-Tramadol - (*S*)-Naproxen (1:1) salt

Figure 42: DSC analysis of (*rac*)-*O*-desmethyl-Tramadol - (*S*)-Naproxen (1:1) salt
Figure 43: TG analysis of (*rac*)-*O*-desmethyl-Tramadol - (*S*)-Naproxen (1:1) salt
Figure 44: XRPD analysis of (*rac*)-*O*-desmethyl-Tramadol - (*S*)-Naproxen (1:1) salt

Figure 45: DSC analysis of (+)-*O*-desmethyl-Tramadol - (*R*)-Flurbiprofen (1:1) salt
Figure 46: TG analysis of (+)-*O*-desmethyl-Tramadol - (*R*)-Flurbiprofen (1:1) salt
Figure 47: XRPD analysis of (+)-*O*-desmethyl-Tramadol - (*R*)-Flurbiprofen (1:1) salt
Figure 48: Crystal structure of (+)-*O*-desmethyl-Tramadol - (*R*)-Flurbiprofen (1:1) salt

Figure 49: DSC analysis of (-)-*O*-desmethyl-Tramadol - (*R*)-Flurbiprofen (1:1) salt
Figure 50: TG analysis of (-)-*O*-desmethyl-Tramadol - (*R*)-Flurbiprofen (1:1) salt
Figure 51: XRPD analysis of (-)-*O*-desmethyl-Tramadol - (*R*)-Flurbiprofen (1:1) salt

Figure 52: DSC analyses of (*rac*)-*O*-desmethyl-Tramadol - (*rac*)-Flurbiprofen (2:3) co-crystal
Figure 53: TG analysis of (*rac*)-*O*-desmethyl-Tramadol - (*rac*)-Flurbiprofen (2:3) co-crystal
Figure 54: XRPD analysis of (*rac*)-*O*-desmethyl-Tramadol - (*rac*)-Flurbiprofen (2:3) co-crystal

Figure 55: DSC analyses of (+)-*O*-desmethyl-Tramadol - (*S*)-Ibuprofen-H₂O (1:1:0.3) salt
Figure 56: TG analysis of (+)-*O*-desmethyl-Tramadol - (*S*)-Ibuprofen-H₂O (1:1:0.3) salt
Figure 57: XRPD analysis of (+)-*O*-desmethyl-Tramadol - (*S*)-Ibuprofen-H₂O (1:1:0.3) salt
Figure 58: Crystal structure of (+)-*O*-desmethyl-Tramadol - (*S*)-Ibuprofen-H₂O (1:1:0.3) salt

Figure 59: DSC analyses of (+)-*O*-desmethyl-Tramadol - (*S*)-Ibuprofen (1:1) salt
Figure 60: TG analysis of (+)-*O*-desmethyl-Tramadol - (*S*)-Ibuprofen (1:1) salt
Figure 61: XRPD analysis of (+)-*O*-desmethyl-Tramadol - (*S*)-Ibuprofen (1:1) salt

Figure 62: DSC analyses of (-)-*O*-desmethyl-Tramadol - (*S*)-Ibuprofen (1:1) salt
Figure 63: TG analysis of (-)-*O*-desmethyl-Tramadol - (*S*)-Ibuprofen (1:1) salt
Figure 64: XRPD analysis of (-)-*O*-desmethyl-Tramadol - (*S*)-Ibuprofen (1:1) salt
Figure 65: Crystal structure of (-)-*O*-desmethyl-Tramadol - (*S*)-Ibuprofen (1:1) salt

Figure 66: DSC analyses of (*rac*)-*O*-desmethyl-Tramadol - (*S*)-Ibuprofen (2:3) co-crystal
Figure 67: TG analysis of (*rac*)-*O*-desmethyl-Tramadol - (*S*)-Ibuprofen (2:3) co-crystal
Figure 68: XRPD analysis of (*rac*)-*O*-desmethyl-Tramadol - (*S*)-Ibuprofen (2:3) co-crystal
Figure 69: Crystal structure of (*rac*)-*O*-desmethyl-Tramadol - (*S*)-Ibuprofen (2:3) co-crystal

Figure 70: DSC analysis of (*rac*)-*O*-desmethyl-Tramadol·HCl - Paracetamol (1: 1) co-crystal
Figure 71: TG analysis of (*rac*)-O-desmethyl-Tramadol·HCl - Paracetamol (1:1) co-crystal
Figure 72: XRPD analysis of (*rac*)-*O*-desmethyl-Tramadol·HCl - Paracetamol (1:1) co-crystal
Figure 73: Crystal structure of (*rac*)-*O*-desmethyl-Tramadol·HCl - Paracetamol (1:1) co-crystal.

Hydrogen atoms in crystal structure diagrams have been omitted for clarity. The program used: *Mercury* 2.2 - C. F. Macrae, I. J. Bruno, J. A. Chisholm, P. R. Edgington, P. McCabe, E. Pidcock, L. Rodriguez-Monge, R. Taylor, J. van de Streek and P. A. Wood, J. Appl. Cryst. 2008, 41, 466-470.

### EXAMPLES

### General Remarks:

Generally all figures of crystal structures are depicted with hydrogen atoms being omitted for clarity. The program used was Mercury 2.2.
In all XRPD measurements the 2θ values were obtained using copper radiation (Cu_{κα} 1.54060 Å).

### Example 1: Form A (rac)-O-desmethyl-Tramadol-Diclofenac (1:1) salt

To a vial containing Diclofenac (89 mg, 0.30 mmol) diluted with IPA (isopropyl alcohol) (1.5 ml) was added at room temperature (*rac*)-*O*-desmethyl-Tramadol (75 mg, 0.30 mmol, 1 eq.) diluted with 1 ml IPA (isopropyl alcohol). The solution was seeded with a solvate *O*-desmethyl-Tramadol·Diclofenac·CH₂Cl₂ (2:2:1) (obtained previously by precipitation from a solution (*rac*)-*O-*desmethyl-Tramadol and Diclofenac (1:1) in CH₂Cl₂ at 0°C).
The solution was evaporated slowly without stirring at room temperature under atmospheric pressure. After complete evaporation, salt form A (*rac*)-*O*-desmethyl-Tramadol-Diclofenac 1:1 was obtained as colourless prisms (164 mg, quantitative yield).

### ¹H NMR

Proton nuclear magnetic resonance analysis was recorded in deuterated dimethylsulfoxide (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum, in d6-DMSO at 400 MHz shows peaks at 7.83 (s br, 1H); 7.49 (d, *J* = 8.2 Hz, 2H); 7.18-7.11 (m, 2H); 7.06 (t, *J* = 7.8 Hz, 1H); 7.01 (dt, *J* = 1.6 Hz, *J* = 7.8 Hz, 1H); 6.91-6.86 (m, 1H); 6.85-6.78 (m, 2H); 6.54 (ddd, *J* = 0.8 Hz, *J* = 5.5 Hz, *J* = 7.8 Hz, 1H); 6.26 (d, *J* = 8.2 Hz, 1H); 3.62 (s, 2H); 2.29-2.21 (m, 1H); 2.02 (s, 6H); 1.88-1.19 (m, 10H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3345.8 (m), 3293.8 (m), 2936.7 (m), 2852.3 (m), 1590.9 (s), 1561.6 (s), 1501.1 (s), 1452.4 (s), 1403.6 (s), 1377.9 (s), 1315.5 (m), 1283.1 (s), 1174.5 (m), 863.6 (m), 773.9 (m), 762.5 (m), 750.0 (m), 737.0 (m), 718.1 (m), 702.5 (m) cm⁻¹.

### DSC

DSC analysis was recorded with a Mettler DSC822^{e}. A sample of 3.8270 mg was weighed into 40 µl aluminium crucible with a pinhole lid and was heated, under nitrogen (50 ml/min), at 10 °C/min from 30 to 200 °C.

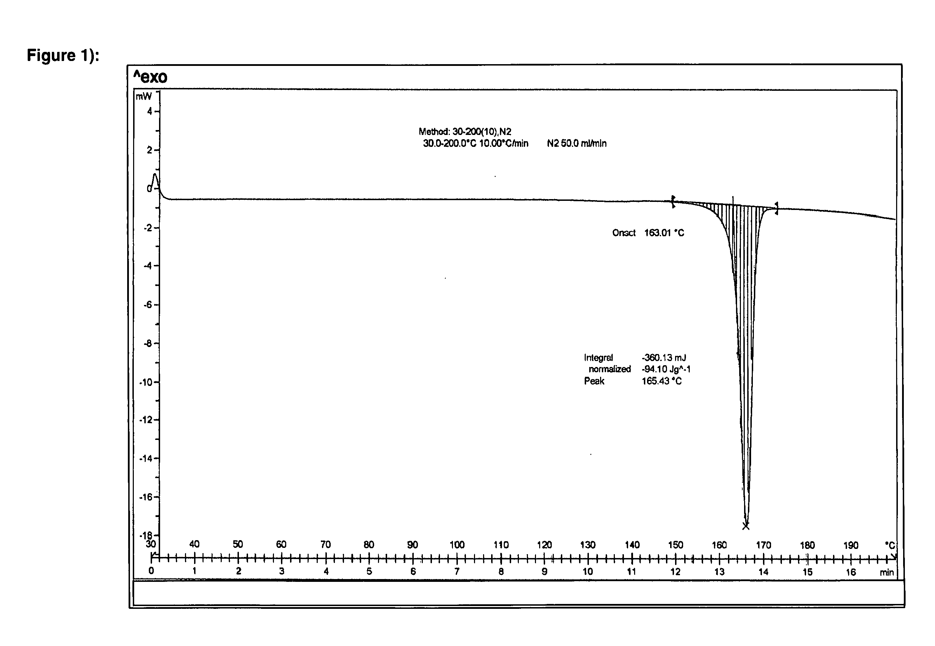

The novel type of crystal of the present invention is **characterized in that** the endothermic sharp peak corresponding to the melting point has an onset at 163.01 °C (fusion enthalpy -94.10 J/g), measured by DSC analysis (10 °C/min) (see figure 1).

### XRPD

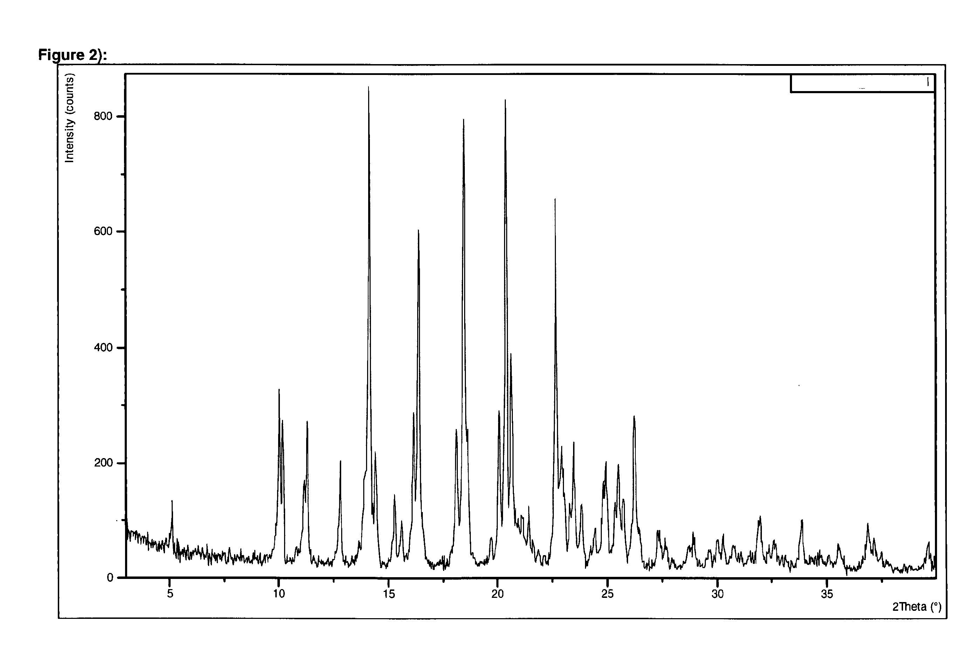

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 2).

### List of selected peaks:

| **2**θ **(°)¹** | **d (Å)** | **I (%)** |
|---|---|---|
| 5.12 | 17.16 | 8 |
| 10.01 | 8.83 | 32 |
| 10.19 | 8.68 | 29 |
| 11.31 | 7.83 | 31 |
| 12.81 | 6.91 | 29 |
| 14.15 | 6.26 | 100 |
| 14.43 | 6.14 | 23 |
| 15.28 | 5.80 | 14 |
| 15.61 | 5.68 | 9 |
| 16.15 | 5.49 | 31 |
| 16.40 | 5.40 | 70 |
| 18.12 | 4.89 | 27 |
| 18.47 | 4.80 | 93 |
| 20.07 | 4.42 | 32 |
| 20.39 | 4.36 | 96 |
| 20.64 | 4.30 | 43 |
| 21.42 | 4.15 | 11 |
| 22.66 | 3.92 | 76 |
| 22.93 | 3.88 | 25 |
| 23.49 | 3.79 | 24 |
| 24.41 | 3.65 | 7 |
| 24.96 | 3.57 | 18 |
| 25.53 | 3.49 | 19 |
| 25.75 | 3.46 | 14 |
| 26.24 | 3.40 | 32 |
| 27.34 | 3.26 | 7 |
| 28.82 | 3.10 | 4 |
| 30.28 | 2.95 | 7 |
| 31.93 | 2.80 | 9 |
| 33.85 | 2.65 | 10 |
| 35.56 | 2.52 | 5 |
| 36.88 | 2.44 | 9 |

| | | |
|---|---|---|
| ¹The 2θ values were obtained using copper radiation (Cu_{κα}). | | |

### Single crystal X-ray diffraction

Crystal structure of salt form A (*rac*)-*O*-desmethyl-Tramadol-Diclofenac 1:1 has been determined from single crystal X-ray diffraction data. The colourless prism used (0.29 × 0.25 × 0.09 mm) was obtained from the crystallization of a solution in IPA (isopropyl alcohol) and water ofequimolar amounts of (*rac*)-*O*-desmethyl-Tramadol·HCl and sodium Diclofenac.
Analysis was performed at room temperature using a Bruker Smart Apex diffractometer with graphite monochromated Mo K_{α} radiation equipped with a CCD detector.
Data were collected using phi and omega scans (program used: SMART 5.6). No significant decay of standard intensities was observed. Data reduction (Lorentz and polarization corrections) and absorption correction were applied (program used: SAINT 5.0).
The structure was solved with direct methods and least-squares refinement of Fₒ² against all measured intensities was carried out (program used: SHELXTL-NT 6.1). All non-hydrogen atoms were refined with anisotropic displacement parameters.

### Relevant structural data:

| Crystal system | Triclinic |
|---|---|
| Space group | *P*-1 |
| a (Å) | 8.9132(7) |
| b (Å) | 9.0384(8) |
| c (Å) | 17.4577(14) |
| α (°) | 88.769(2) |
| β (°) | 88.979(2) |
| γ (°) | 78.788(2) |
| Volume (Å³) | 1379.1(2) |
| Z | 2 |
| D calc. (Mg/m³) | 1.314 |
| N. of refl. | 6468 |
| Refl. with I > 2σ(I) | 4300 |
| R (I > 2σ(I)) | 0.0529 |

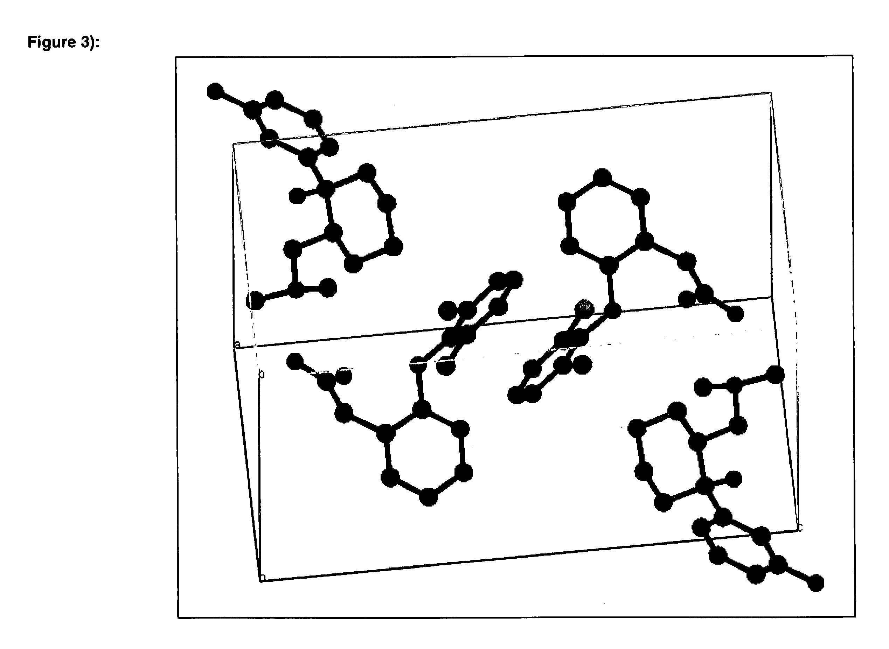

The crystal structure of salt form A (*rac*)-*O*-desmethyl-Tramadol-Diclofenac 1:1 1 is depicted in figure 3.
Simulation of XRPD diffractogram from single crystal data gives an almost identical diagram to the experimental one presented above.

### Example 2a: Form B (rac)-O-desmethyl-Tramadol-Diclofenac (1:1) salt

To an assay tube equipped with magnetic stirrer containing Diclofenac (89 mg, 0.30 mmol) diluted with IPA (isopropyl alcohol) (1 ml) was added at room temperature (*rac*)-*O*-desmethyl-Tramadol (38 mg, 0.15 mmol, 0.5 eq.) diluted with 1 ml IPA (isopropyl alcohol). The solution was seeded with a mechanical mixture of form B and Diclofenac (obtained previously by wet grinding (*rac*)-*O*-desmethyl-Tramadol/Diclofenac (1:2) with AcOEt).
After 10 min, a white solid precipitated. The mixture was stirred 1 h 30 min at room temperature. Then, the solid was filtered with a sinter funnel n°3 and washed 0.15 ml IPA (isopropyl alcohol) (twice). After drying at room temperature under vacuum line, salt form B (*rac*)-*O*-desmethyl-Tramadol-Diclofenac 1:1 was obtained as a white solid (55 mg, 67 % yield).

### Example 2b: Form B (rac)-O-desmethyl-Tramadol-Diclofenac (1:1) salt

To a vial containing Diclofenac (89 mg, 0.30 mmol) diluted with ethanol (1.5 ml) was added at room temperature (*rac*)-*O*-desmethyl-Tramadol (75 mg, 0.30 mmol, 1 eq.) diluted with 1.3 ml ethanol. The solution was seeded with form B.
The solution was evaporated slowly without stirring at room temperature under atmospheric pressure. After complete evaporation, salt form B (*rac*)-*O*-desmethyl-Tramadol-Diclofenac 1:1 was obtained as colorless blocks (164 mg, quantitative yield).

### ¹H NMR

Proton nuclear magnetic resonance analysis was recorded in deuterated dimethylsulfoxide (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in d6-DMSO at 400 MHz shows peaks identical to those of form A.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3323.3 (m), 2936.8 (m), 2861.6 (m), 1602.4 (s), 1588.2 (s), 1577.5 (s), 1561.6 (s), 1451.2 (s), 1416.6 (m), 1378.1 (s), 1309.2 (m), 1291.9 (m), 1277.3 (s), 1151.7 (m), 964.7 (m), 781.8 (s), 740.9 (s), 715.7 (m), 656.2 (m), 646.0 (m) cm⁻¹.

### DSC

DSC analysis was recorded with a Mettler DSC822^{e}. A sample of 2.6700 mg was weighed into 40 µl aluminium crucible with a pinhole lid and was heated, under nitrogen (50 ml/min), at 10 °C/min from 30 to 220 °C.

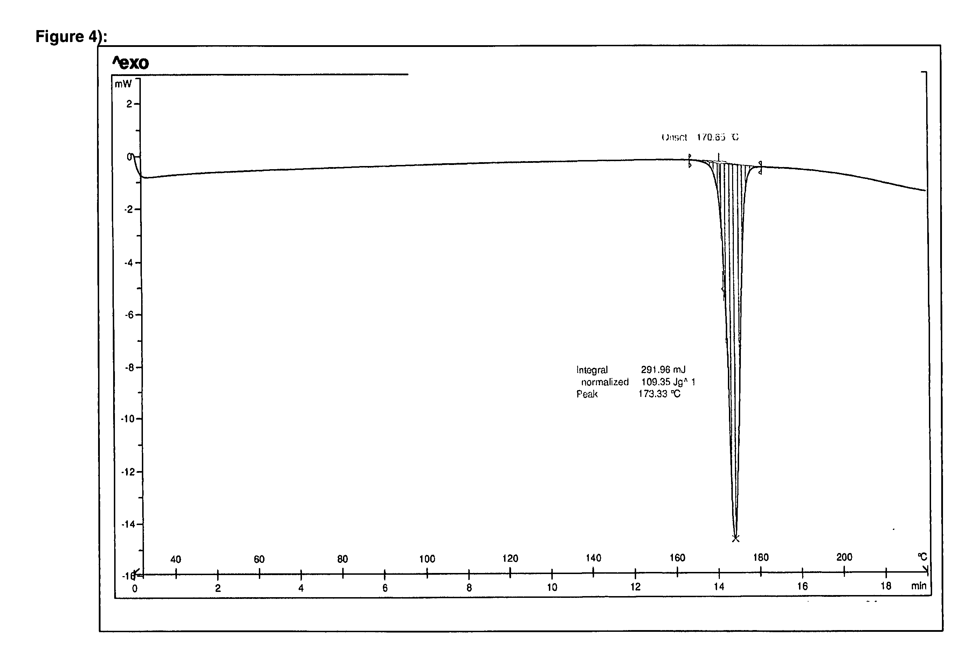

The novel type of crystal of the present invention is **characterized in that** the endothermic sharp peak corresponding to the melting point has an onset at 170.65 °C (fusion enthalpy -109.35 J/g), measured by DSC analysis (10 °C/min) (see figure 4).

### TG

Thermogravimetric analysis was recorded in a thermogravimetric analyzer Mettler TGA/SDTA851^{e}. A sample of 3.9316 mg was weighed into a 70 µl alumina crucible with a pinhole lid and was heated at 10 °C/min from 30 to 200 °C, under nitrogen (50 ml/min).

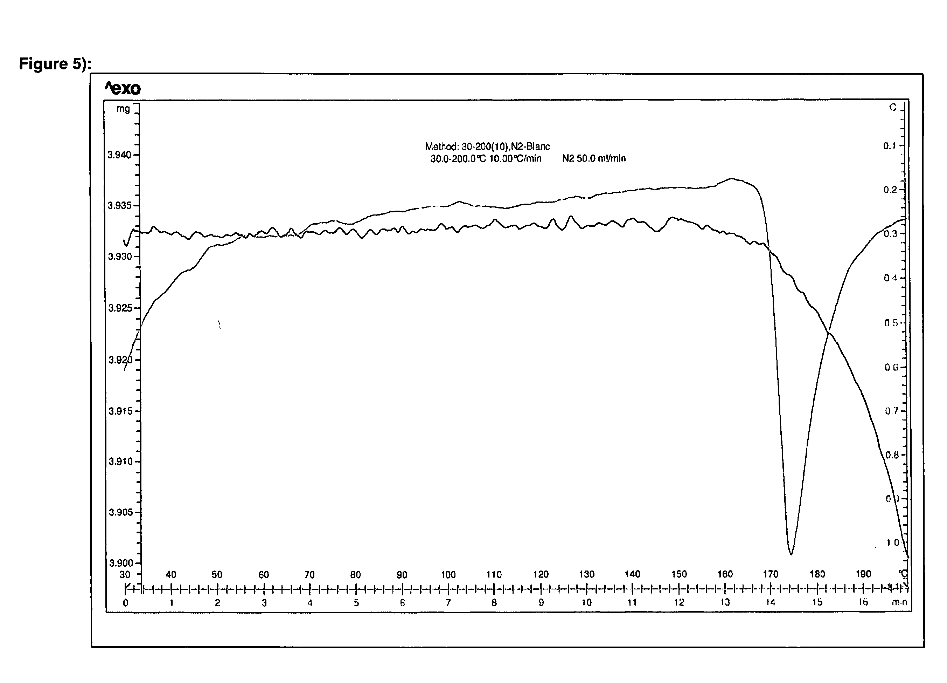

The TG analysis of the crystalline form according to the invention shows no weight loss at temperatures lower than the melting point (see figure 5).

### XRPD

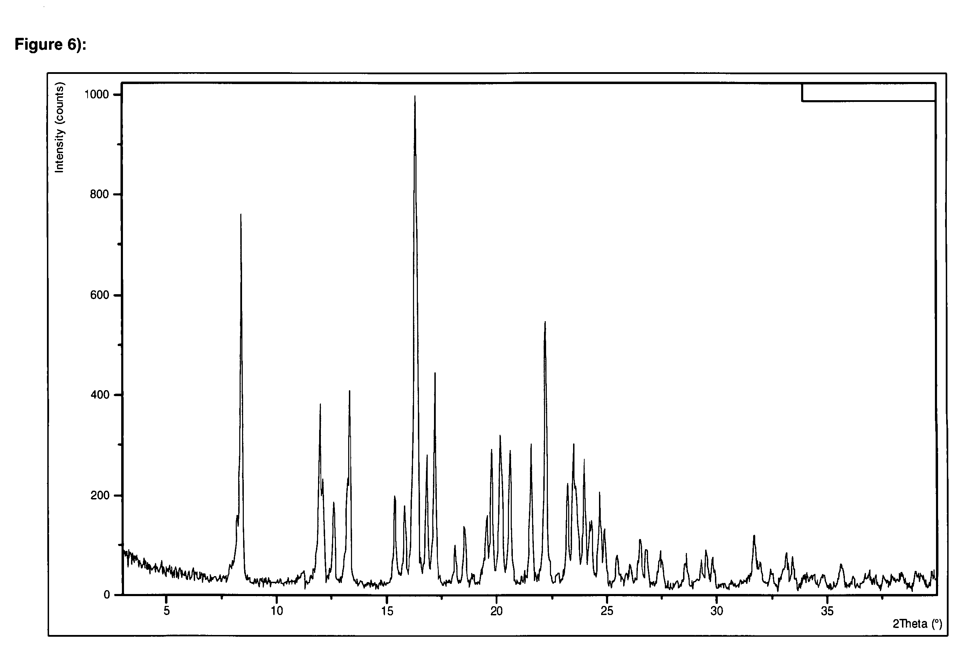

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 6).

### List of selected peaks:

| **2**θ **(°)¹** | **d (Å)** | **I (%)** |
|---|---|---|
| 8.43 | 10.49 | 98 |
| 11.97 | 7.39 | 43 |
| 12.61 | 7.02 | 20 |
| 13.34 | 6.63 | 48 |
| 15.39 | 5.76 | 23 |
| 15.83 | 5.60 | 20 |
| 16.38 | 5.41 | 100 |
| 16.84 | 5.26 | 33 |
| 17.21 | 5.15 | 53 |
| 18.11 | 4.90 | 9 |
| 18.55 | 4.78 | 15 |
| 19.55 | 4.54 | 17 |
| 19.79 | 4.49 | 34 |
| 20.20 | 4.40 | 38 |
| 20.62 | 4.31 | 34 |
| 21.60 | 4.11 | 33 |
| 22.24 | 4.00 | 66 |
| 23.23 | 3.83 | 25 |
| 23.48 | 3.79 | 31 |
| 24.00 | 3.71 | 30 |
| 24.31 | 3.66 | 16 |
| 24.71 | 3.60 | 23 |
| 24.92 | 3.57 | 14 |
| 25.49 | 3.49 | 7 |
| 26.52 | 3.36 | 12 |
| 26.81 | 3.33 | 9 |
| 27.42 | 3.25 | 9 |
| 28.59 | 3.12 | 5 |
| 29.81 | 3.00 | 7 |
| 31.70 | 2.82 | 12 |
| 33.12 | 2.70 | 7 |
| 34.14 | 2.63 | 2 |
| 35.63 | 2.52 | 5 |
| 36.93 | 2.43 | 1 |

| | | |
|---|---|---|
| ¹The 2θ values were obtained using copper radiation (Cu_{Kα}). | | |

### Single crystal X-ray diffraction

The crystal structure of form B has been determined from single crystal X-ray diffraction data. The colourless prism used (0.35 × 0.8 × 0.11 mm) was obtained from the evaporation of an ethanol solution of equimolar amounts of (*rac*)-*O*-desmethyl-Tramadol and Diclofenac.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters.

### Relevant structural data:

| Crystal system | Monoclinic |
|---|---|
| Space group | *P*2₁/*n* |
| a (Å) | 15.4377(11) |
| b (Å) | 8.5862(7) |
| c (Å) | 21.9585(16) |
| β (°) | 105.835(2) |
| Volume (Å³) | 2800.2(4) |
| Z | 4 |
| D calc. (Mg/m³) | 1.294 |
| N. of refl. | 6883 |
| Refl. with I > 2σ(I) | 3559 |
| R (I > 2σ(I)) | 0.0585 |

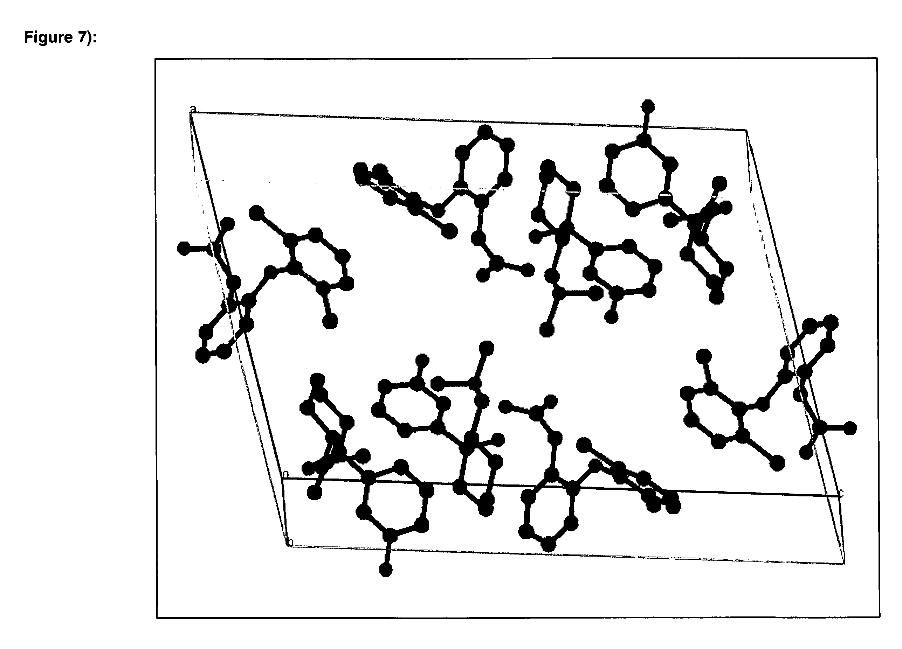

The crystal structure of salt form B (*rac*)-*O*-desmethyl-Tramadol-Diclofenac 1:1 is depicted in figure 7.
Simulation of the XRPD diffractogram from single crystal data gives an almost identical diagram to the experimental one presented above.

### Example 3a: (+)-O-desmethyl-Tramadol-Diclofenac-H₂O (1:1:0.7-0.8) salt

To an assay tube equipped with magnetic stirrer containing Diclofenac (72 mg, 0.24 mmol) and (+)-*O*-desmethyl-Tramadol (60 mg, 0.24 mmol, 1 eq.) was added 2 ml toluene before heating to reflux. A turbid solution was obtained. The mixture was cooled slowly to room temperature: oil appeared over the wall of the assay tube.
The mixture was heated slowly until obtain a white solid (temperature bath 53 °C) and then cooled to room temperature. The white solid was filtered with a sinter funnel (porosity 3) and washed toluene (0.3 ml). After drying at 80 °C under vacuum, salt form (+)-*O*-desmethyl-Tramadol-Diclofenac-H₂O 1:1:0.7 was obtained as a white solid (100 mg, 74 % yield).

### Example 3b: (+)-O-desmethyl-Tramadol-Diclofenac-H₂O (1:1:0.7-0.8) salt

To a vial equipped with magnetic stirrer containing Diclofenac (72 mg, 0.24 mmol) and (+)-*O-*desmethyl-Tramadol (60 mg, 0.24 mmol, 1 eq.) was added ACN (acetonitrile) (1.5 ml) before heating until reach complete dissolution. The solution was cooled slowly to room temperature.
After 10 min, a white solid precipitated. The mixture was stirred 1 h 30 min at room temperature. Then, the solid was filtered with a sinter funnel (porosity 3) and washed 0.2 ml ACN (acetonitrile). After drying at 80 °C under vacuum, salt (+)-*O*-desmethyl-Tramadol-Diclofenac-H₂O 1:1:0.7 was obtained as a white solid (103 mg, 76 % yield).

### Example 3c: (+)-O-desmethyl-Tramadol-Diclofenac-H₂O (1:1:0.7-0.8) salt

To a vial containing Diclofenac (72 mg, 0.24 mmol) and (+)-*O*-desmethyl-Tramadol (60 mg, 0.24 mmol, 1 eq.) was added ACN (acetonitrile) (4 ml) and H₂O (0.1 ml) (complete dissolution).
The solution was evaporated slowly without stirring at room temperature under atmospheric pressure. After complete evaporation, salt (+)-*O*-desmethyl-Tramadol-Diclofenac-H₂O 1:1:0.8 was obtained as colorless plates (135 mg, quantitative yield).

### ¹H NMR

Proton nuclear magnetic resonance analysis was recorded in deuterated dimethylsulfoxide (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum, in d6-DMSO at 400 MHz shows peaks at 7.77 (s br, 1H); 7.50 (d, *J* = 8.2 Hz, 2H); 7.19-7.12 (m, 2H); 7.06 (t, *J* = 7.8 Hz, 1H); 7.01 (dt, *J* = 1.6 Hz, *J* = 7.8 Hz, 1H); 6.91-6.86 (m, 1H); 6.85-6.79 (m, 2H); 6.54 (dd, *J =* 5.5 Hz, *J* = 7.8 Hz, 1H); 6.26 (d, *J* = 7.8 Hz, 1H); 3.63 (s, 2H); 2.28-2.17 (m, 1H); 2.01 (s, 6H); 1.88-1.22 (m, 10H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3235.3 (s), 3075.3 (s), 2944.2 (s), 1917.7 (s), 2850.6 (s), 1576.2 (s), 1561.1 (s), 1502.3 (s), 1450.6 (s), 1380.6 (s), 1308.4 (m), 1281.3 (s), 1240.5 (s), 1212.2 (m), 1149.9 (m), 1108.9 (m), 1002.0 (m), 866.4 (s), 748.3 (s), 707.3 (m), 647.8 (m) cm⁻¹.

### DSC

DSC analysis was recorded with a Mettler DSC822^{e}. A sample of 3.2580 mg was weighed into 40 µl aluminium crucible with a pinhole lid and was heated, under nitrogen (50 ml/min), at 10 °C/min from 30 to 190 °C.

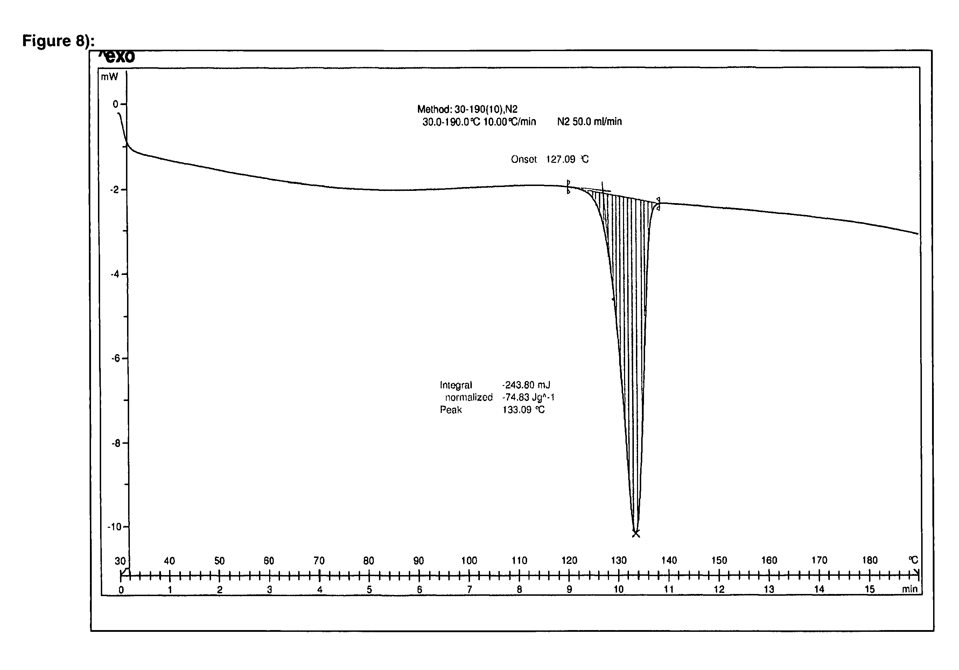

The novel type of crystal of the present invention is **characterized in that** the endothermic sharp peak corresponding to the melting point has an onset at 127.09 °C (fusion enthalpy -74.83 J/g), measured by DSC analysis (10 °C/min) (see figure 8).

### TG

Thermogravimetric analysis was recorded in a thermogravimetric analyzer Mettler TGA/SDTA851^{e}. A sample of 2.1058 mg was weighed into a 70 µl alumina crucible with a pinhole lid and was heated at 10 °C/min from 30 to 200 °C, under nitrogen (50 ml/min).

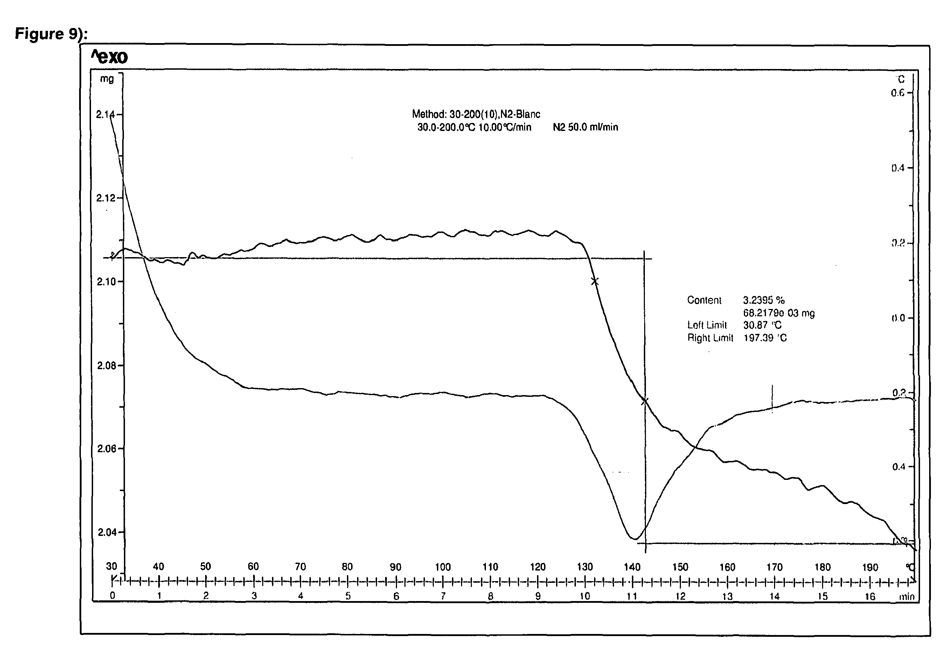

The TG analysis of this crystalline form according to the invention, obtained from the example 2, shows an approximated weight loss of 2.4 % during the melting (between 120 °C and 165 °C): weight of 0.7-0.8 molecule H₂O (see figure 9).

### Karl Fisher

Karl Fisher analysis was recorded with a Metrohm 787 KF Trinito. Analysis of 194 mg sample was carried out using the following reactants: Hydranal-Composite 1 (Riedel de Haën Ref. 34827), Hydranal Methanol Rapid (Riedel de Haën Ref 37817) and Hydranal Water Standard 1.0 (Riedel de Haën Ref. 34828 used to calculate the factor).

The KF analysis of the crystalline form according to the invention shows 2.2-2.4 % water.

### XRPD

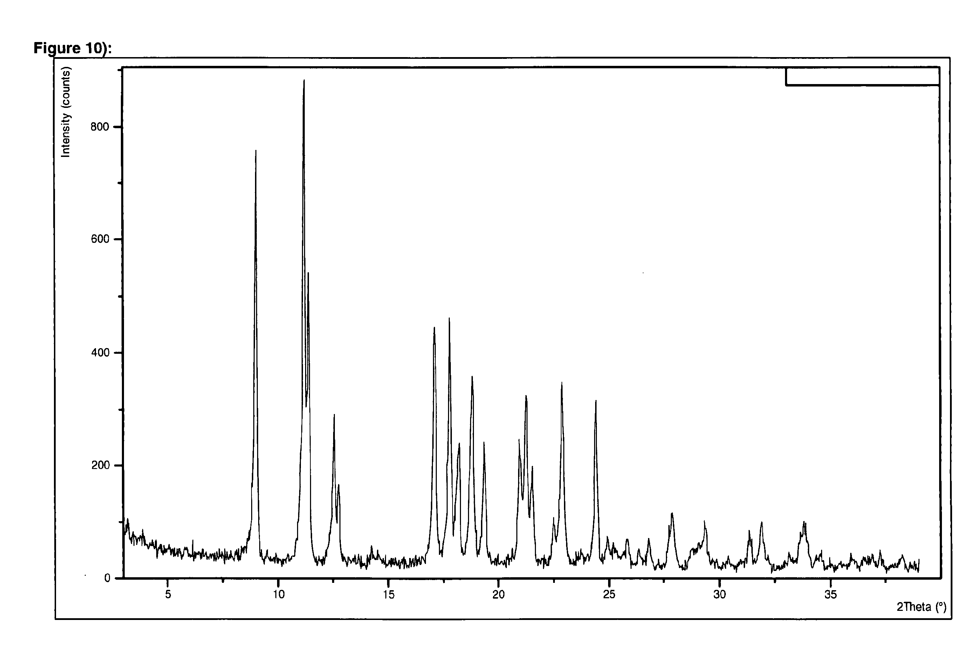

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 10).

### List of selected peaks:

| **2**θ **(°)¹** | **d (Å)** | **I (%)** |
|---|---|---|
| 9.05 | 9.78 | 85 |
| 11.21 | 7.89 | 100 |
| 11.42 | 7.75 | 60 |
| 12.56 | 7.05 | 31 |
| 12.70 | 6.97 | 20 |
| 17.12 | 5.18 | 49 |
| 17.80 | 4.98 | 52 |
| 18.21 | 4.87 | 26 |
| 18.84 | 4.71 | 39 |
| 19.37 | 4.58 | 25 |
| 20.96 | 4.24 | 26 |
| 21.27 | 4.18 | 36 |
| 21.53 | 4.13 | 20 |
| 22.51 | 3.95 | 11 |
| 22.91 | 3.88 | 38 |
| 24.42 | 3.65 | 33 |
| 25.85 | 3.45 | 5 |
| 26.83 | 3.32 | 5 |
| 27.86 | 3.20 | 11 |
| 29.35 | 3.04 | 9 |
| 31.38 | 2.85 | 6 |
| 31.91 | 2.80 | 10 |
| 33.88 | 2.65 | 8 |

| | | |
|---|---|---|
| ¹The 2θ values were obtained using copper radiation (Cu_{Kα}). | | |

### Single crystal X-ray diffraction

This crystal structure has been determined from single crystal X-ray diffraction data. The colourless prism used (0.50 × 0.28 × 0.11 mm) was obtained from the evaporation of an acetonitrile solution of equimolar amounts of (+)-*O*-desmethyl-Tramadol and Diclofenac. Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters, except oxygen of the disordered water molecule.

### Relevant structural data:

| Crystal system | Triclinic |
|---|---|
| Space group | *P*1 |
| a (Å) | 8.6494(7) |
| b (Å) | 9.2929(8) |
| c (Å) | 10.8666(9) |
| α (°) | 65.489(1) |
| β (°) | 81.350(2) |
| γ (°) | 66.058(1) |
| Volume (Å³) | 726.17(10) |
| Z | 1 |
| D calc. (Mg/m³) | 1.289 |
| N. of refl. | 4006 |
| Refl. with I > 2σ(I) | 3555 |
| R (I > 2σ(I)) | 0.0387 |

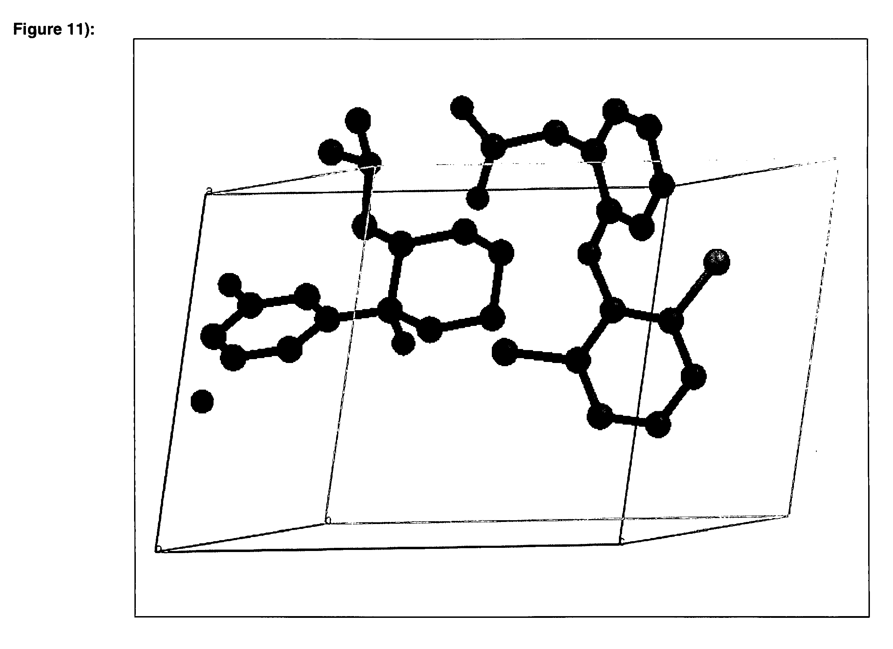

The crystal structure of salt (+)-*O*-desmethyl-Tramadol-Diclofenac-H₂O 1:1:0.8 is depicted in figure 11 (only one position of oxygen of disordered water is shown).
Simulation of the XRPD diffractogram from single crystal data gives an almost identical diagram to the experimental one presented above.

### Example 4a: (rac)-O-desmethyl-Tramadol-Acetylsalicylic acid (1:1) salt

To an assay tube equipped with a magnetic stirrer containing (*rac*)-*O*-desmethyl-Tramadol (58 mg, 0.23 mmol) and Acetylsalicylic acid (42 mg, 0.23 mmol, 1 eq.), toluene (1.5 ml) was added at room temperature. The resultant slurry was stirred for 18 h. Then, the white solid was filtered with a sintered funnel (porosity 4) and washed with cold toluene (2 x 0.2 ml). After vacuum drying at room temperature, salt (*rac*)-*O*-desmethyl-Tramadol-Acetylsalicylic acid (1:1) was obtained (83 mg, 83 % yield).

### Example 4b: (rac)-O-desmethyl-Tramadol-Acetylsalicylic acid (1:1) salt

To an assay tube equipped with a magnetic stirrer containing (*rac*)-*O*-desmethyl-Tramadol (54 mg, 0.22 mmol) and Acetylsalicylic acid (41 mg, 0.23 mmol, 1.05 eq.), was added at room temperature chloroform (0.8 ml). The resultant mixture was stirred as a homogenous colourless solution. After 20 min of stirring, the precipitation of a white solid started. The reaction mixture was stirred for 4 h and filtered with a sintered funnel (porosity 4) before washing with cold chloroform (2 × 0.4 ml). After drying under vacuum at room temperature, salt (*rac*)-*O-*desmethyl-Tramadol-Acetylsalicylic acid (1:1) was obtained as a white solid (84 mg, 88 % yield).

### ¹H NMR

Proton nuclear magnetic resonance analysis was recorded in deuterated water (D₂O) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in D₂O at 400 MHz shows peaks at 7.54 (dd, *J* = 7.8 Hz, *J =* 1.8 Hz, 1H); 7.36 (td, *J* = 7.8 Hz, *J*₂ = 1.7 Hz, 1H); 7.22 (t, *J* = 7.6 Hz, 2H); 7.00 (dd, *J* = 8.2 Hz, *J* = 1.0 Hz, 1H); 6.96 (d, *J* = 8.0 Hz, 1H); 6.90 (t, *J*= 2.0 Hz, 1H); 6.72 (dd, *J* = 8.2 Hz, *J* = 2.2 Hz, 1H); 2.79 (dd, *J* = 13.2 Hz, *J* = 10.0 Hz, 1H); 2.55 (t, *J*= 2.4 Hz, 1H); 2.53 (s, 6H); 2.19 (s, 3H); 2.14 (t, *J* = 7.4 Hz, 2H); 1.86-1.78 (m, 1H), 1.74-1.64 (m, 2H), 1.62-1.57 (m, 1H); 1.53-1.32 (m, 3H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3374.9 (m, br), 3133.5 (w, br), 2948.9 (w), 2929.2 (w), 1746.8 (s), 1609.7 (s), 1591.6 (s), 1567.7 (s), 1540.9 (w), 1506.4 (w), 1477.0 (m), 1444.7 (s), 1380.8 (s), 1305.8 (s), 1309.1 (w), 1281.4 (m), 1240.1 (s), 1217.6 (s), 1193.0 (s), 1160.4 (w), 1146.9 (w), 1090.7 (w), 1005.4 (s), 917.6 (w), 862.1 (w), 805.7 (m), 758.9 (s) cm⁻¹.

### DSC

DSC analysis was recorded with a Mettler DSC822^{e}. A sample of 1.6990 mg was weighed into 40 µl aluminium crucible with a pinhole lid and was heated, under nitrogen (50 ml/min), at 10 °C/min from 30 to 310 °C.

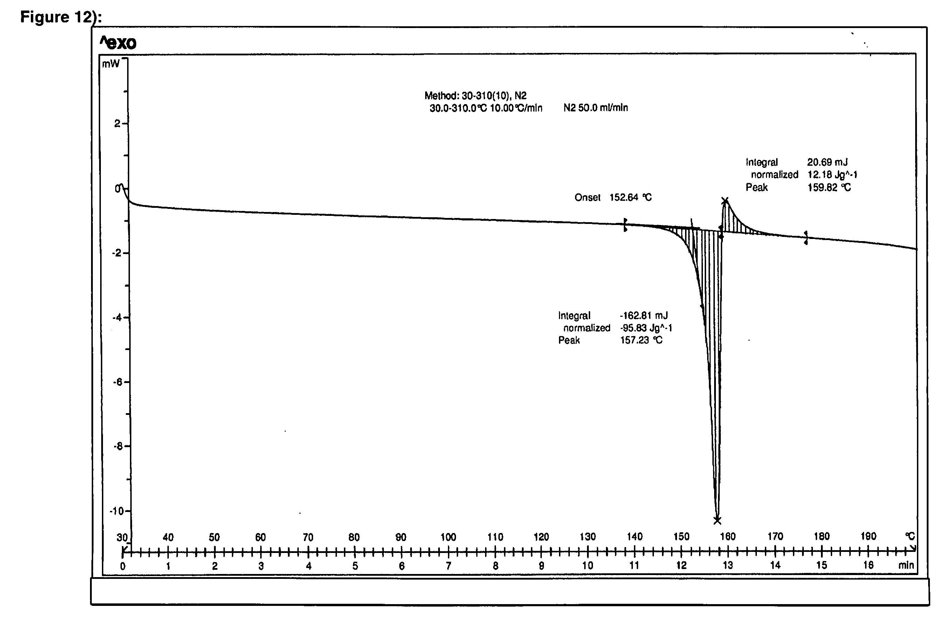

The novel type of crystal of the present invention is **characterized in that** the endothermic sharp peak corresponding to the melting point has an onset at 151.64 °C (fusion enthalpy -95.83 J/g), measured by DSC analysis (10 °C/min) (see figure 12).

### TG

Thermogravimetric analysis was recorded in a thermogravimetric analyzer Mettler TGA/SDTA851^{e}. A sample of 3.4395 mg was weighed into a 70 µl alumina crucible with a pinhole lid and was heated at 10 °C/min from 30 to 180 °C, under nitrogen (50 ml/min).

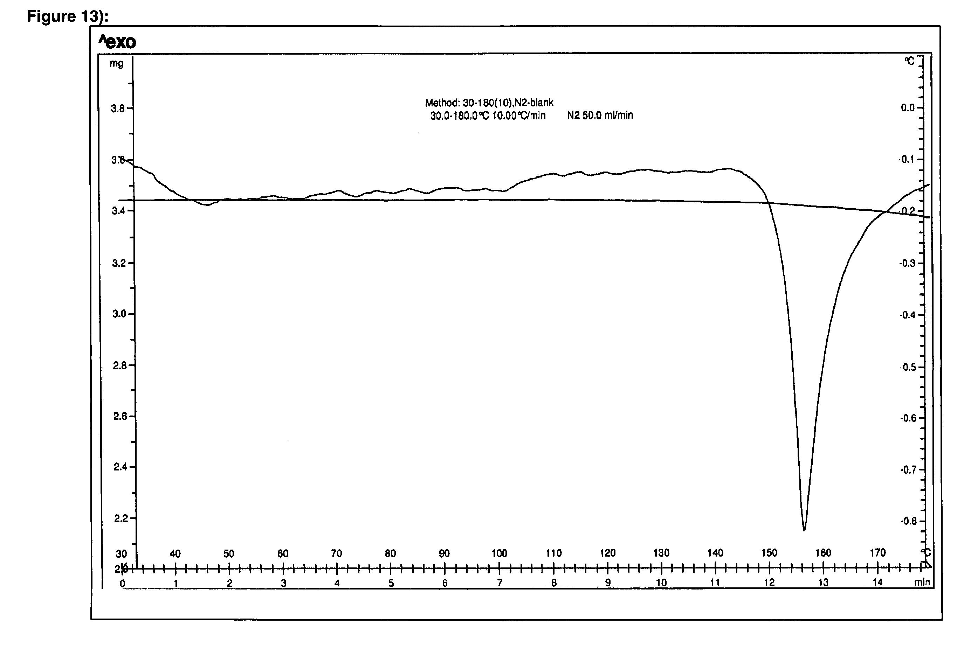

The TG analysis of the crystalline form according to the invention shows no weight loss at temperatures lower than the melting point (see figure 13).

### XRPD

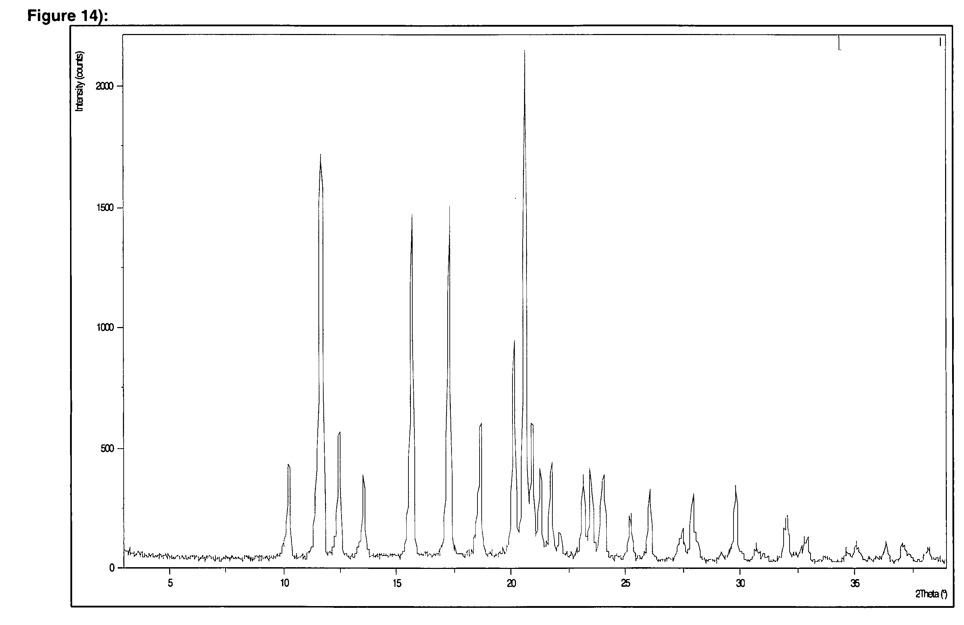

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 14).

### List of selected peaks:

| **2θ (°)** | **d (Å)** | **I (%)** |
|---|---|---|
| 10.27 | 8.62 | 18 |
| 11.72 | 7.55 | 78 |
| 12.47 | 7.10 | 25 |
| 13.53 | 6.54 | 17 |
| 15.66 | 5.66 | 69 |
| 17.29 | 5.13 | 70 |
| 18.65 | 4.76 | 27 |
| 20.12 | 4.41 | 43 |
| 20.60 | 4.31 | 100 |
| 20.92 | 4.25 | 27 |
| 21.27 | 4.18 | 18 |
| 21.74 | 4.09 | 19 |
| 22.12 | 4.02 | 5 |
| 23.14 | 3.84 | 17 |
| 23.49 | 3.79 | 15 |
| 24.05 | 3.70 | 16 |
| 25.22 | 3.53 | 7 |
| 26.05 | 3.42 | 14 |
| 27.44 | 3.25 | 6 |
| 27.91 | 3.20 | 13 |
| 29.78 | 3.00 | 15 |
| 30.71 | 2.91 | 2 |
| 32.03 | 2.79 | 9 |
| 32.94 | 2.72 | 4 |
| 34.61 | 2.59 | 3 |
| 35.08 | 2.56 | 4 |
| 36.34 | 2.47 | 3 |
| 37.08 | 2.42 | 3 |
| 38.17 | 2.36 | 3 |

| | | |
|---|---|---|
| ¹The 2θ values were obtained using copper radiation (Cu_{Kα}). | | |

### Single crystal X-ray

This crystal structure has been determined from single crystal X-ray diffraction data. The colourless prism used (0.35 × 0.30 × 0.29 mm) was obtained from the evaporation of a THF solution of equimolar amounts of (*rac*)-*O*-desmethyl-Tramadol and Acetylsalicylic acid.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters.

### Relevant structural data:

| Crystal system | Monoclinic |
|---|---|
| Space group | Cc |
| a (Å) | 17.6939(14) |
| b (Å) | 8.4806(7) |
| c (Å) | 15.5176(12) |
| β (°) | 103.232(1) |
| Volume (Å³) | 2266.7(3) |
| Z | 4 |
| D calc. (Mg/m³) | 1.259 |
| N. of refl. | 4599 |
| Refl. with I > 2σ(I) | 3741 |
| R (I > 2σ(I)) | 0.0454 |

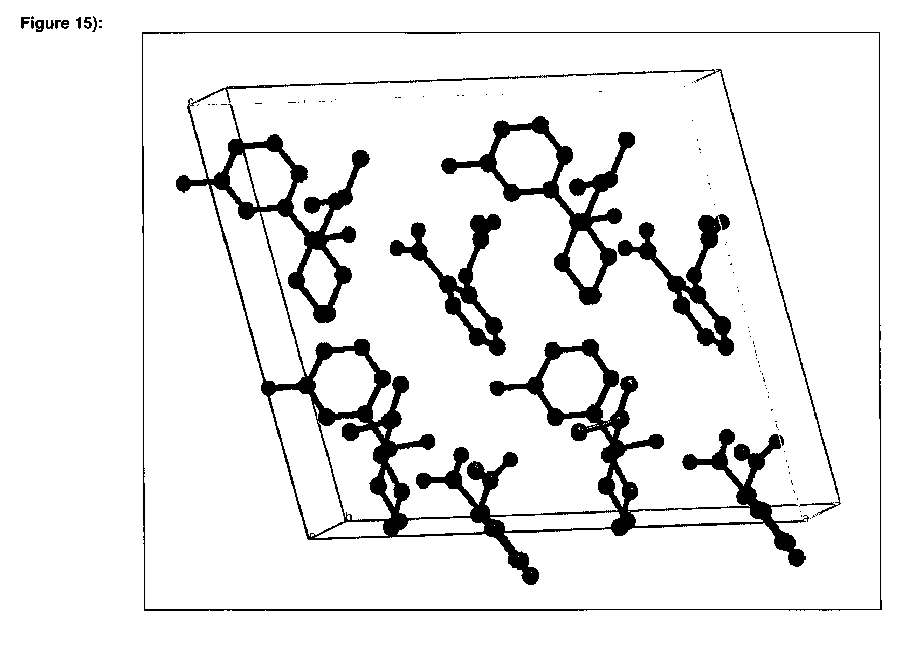

The crystal structure of salt of (*rac*)-*O*-desmethyl-Tramadol-Acetylsalicylic acid (1:1) is depicted in figure 15.
Simulation of the XRPD diffractogram from single crystal data gives an almost identical diagram to the experimental one presented above.

### Example 5a: Form A (+)-O-desmethyl-Tramadol-Acetylsalicylic acid (1:1) salt

To an assay tube equipped with magnetic stirrer containing (+)-*O*-desmethyl-Tramadol (56 mg, 0.22 mmol) and Acetylsalicylic acid (43 mg, 0.24 mmol, 1.1 eq.), was added MIK (methyl isobutyl ketone) (0.7 ml) at room temperature. After the total dissolution of the reactants, precipitation was observed. The crude was stirred for 6 h and was filtered with a sintered funnel (porosity 4), the resultant white solid was washed with MIK (methyl isobutyl ketone) (1 ml). After vacuum drying at room temperature, salt form A (+)-*O*-desmethyl-Tramadol-Acetylsalicylic acid (1:1) was obtained (78 mg, 82 % yield).

### Example 5b: Form A (+)-O-desmethyl-Tramadol-Acetylsalicylic acid (1:1) salt

To an assay tube equipped with magnetic stirrer containing (+)-*O*-desmethyl-Tramadol (55 mg, 0.22 mmol) and Acetylsalycilic acid (40 mg, 0.22 mmol, 1 eq.), was added acetonitrile (0.7 ml) at room temperature. The resultant white suspension was stirred for 4 h. Then, the mixture was diluted with 1 ml of acetonitrile before filtering with a sintered funnel (porosity 4) and washing with cold acetonitrile (0.3 ml). After vacuum drying at room temperature, salt form A (+)-*O-*desmethyl-Tramadol-Acetylsalicylic acid (1:1) was obtained (66 mg, 69 % yield).

### ¹H NMR

Proton nuclear magnetic resonance analysis was recorded in deuterated DMSO (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in d6-DMSO at 400 MHz shows peaks at 7.85 (dd, *J* = 7.8 Hz, *J* = 1.8 Hz, 1H); 7.52 (t, *J*= 7.4 MHz, 1H); 7.28 (t, *J* = 7.6 MHz, 1H); 7.09 (d, *J* = 8.4 MHz, 1H); 7.03 (t, *J* = 7.8 Hz, 1H);6.86(s, 1H); 6.79 (d, *J*= 8.0 Hz, 1H); 6.52 (dd, *J* = 7.6 Hz, *J* = 2.4 Hz, 1H); 2.23 (t, *J*= 9 Hz, 1H); 2.19 (s, 3H); 2.00 (s, 6H); 1.83-1.56 (m, 6H), 1.50-1.40 (m, 3H); 1.33-1.25 (m, 1H) ppm.

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3327.1 (m, br), 1933.4 (m), 1750.2 (s), 1610.0 (s), 1583.0 (s), 1560.2 (s), 1475.0 (m), 1360.5 (s), 1248.8 (s), 1220.1 (s), 1190.5 (s), 1154.4 (m), 1090.7 (m), 1014.2 (w), 959.3 (w), 918.7 (w), 796.0 (m), 760.0 (m) 704.0 (m) cm⁻¹.

### DSC

DSC analysis was recorded with a Mettler DSC822^{e}. A sample of 1.2760 mg was weighed into a 40 µl aluminium crucible with a pinhole lid, and was heated, under nitrogen (50 ml/min), at 10 °C/min from 30 to 200 °C.

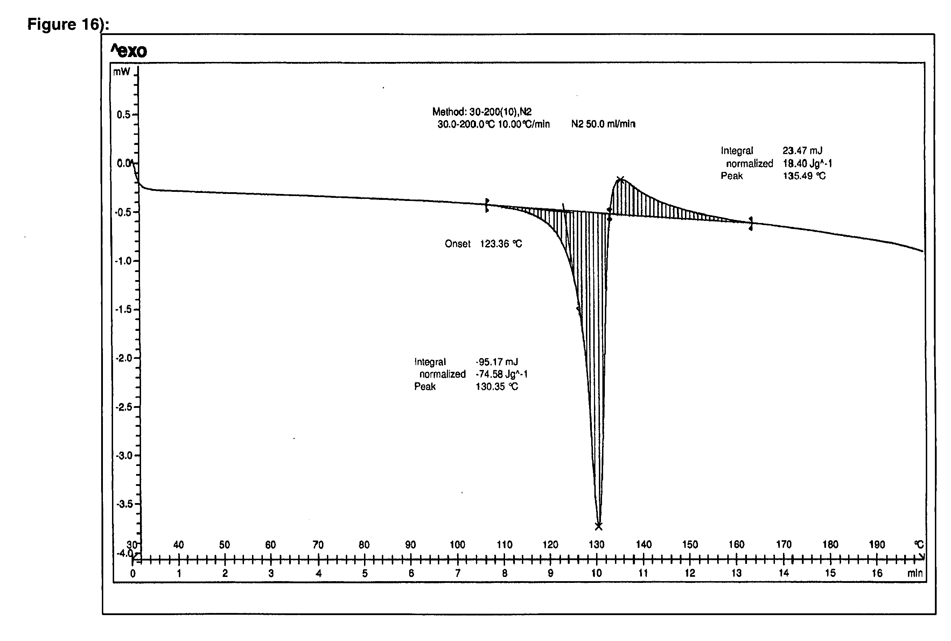

The novel type of crystal of the present invention is **characterized in that** the endothermic sharp peak corresponding to the melting point has an onset at 123.36 °C (fusion enthalpy -74.58 J/g), measured by DSC analysis (10 °C/min) (see figure 16).

### TG

Thermogravimetric analysis was recorded in a thermogravimetric analyzer Mettler TGA/SDTA851^{e}. A sample of 6.5445 mg was weighed into a 70 µl alumina crucible with a pinhole lid, and was heated at 10 °C/min from 30 to 200 °C, under nitrogen (50 ml/min).

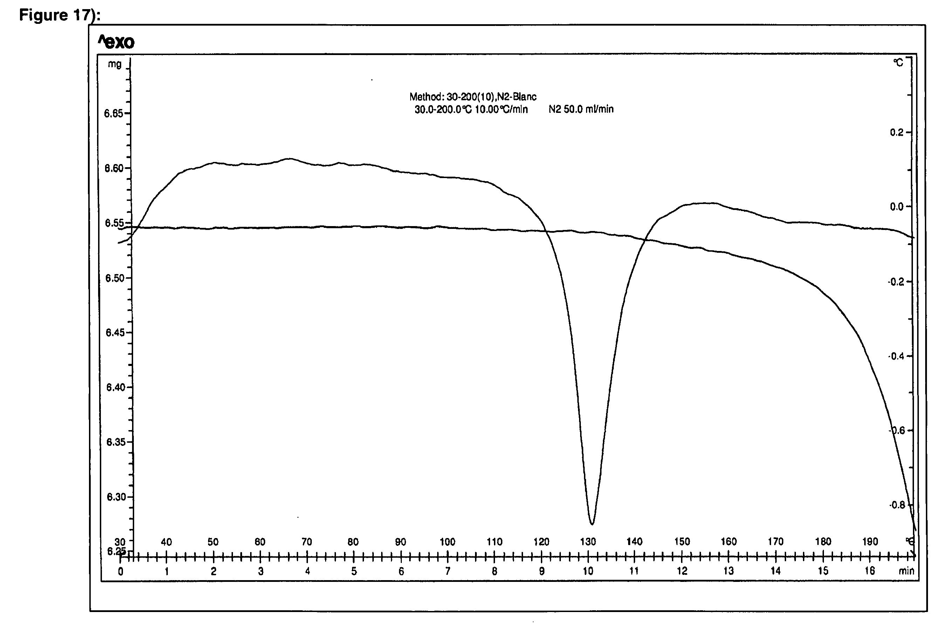

The TG analysis of the crystalline form according to the invention shows no weight loss at temperatures lower than the melting point (see figure 17).

### XRPD

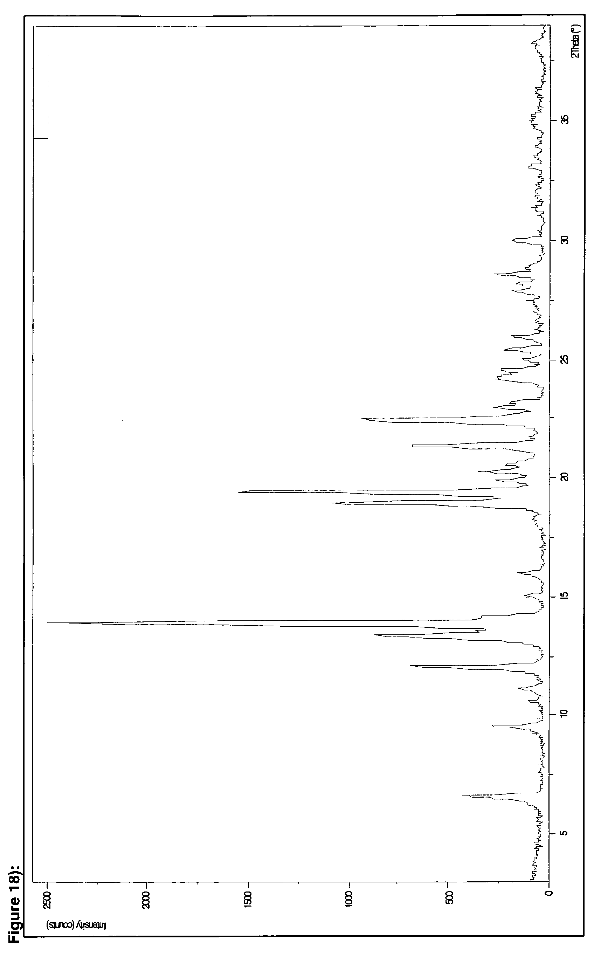

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 18).

### List of selected peaks:

| **2**θ **(°)¹** | **d (Å)** | **I (%)** |
|---|---|---|
| 6.64 | 13.32 | 16 |
| 9.57 | 9.24 | 10 |
| 10.61 | 8.34 | 2 |
| 11.15 | 7.94 | 5 |
| 12.09 | 7.32 | 27 |
| 13.40 | 6.61 | 34 |
| 13.94 | 6.35 | 100 |
| 14.10 | 6.24 | 12 |
| 15.10 | 5.8 | 3 |
| 16.02 | 5.53 | 5 |
| 18.96 | 4.68 | 43 |
| 19.45 | 4.56 | 60 |
| 19.91 | 4.46 | 10 |
| 20.27 | 4.38 | 13 |
| 21.38 | 4.16 | 26 |
| 22.46 | 3.96 | 35 |
| 23.00 | 3.86 | 10 |
| 14.18 | 3.68 | 9 |
| 24.58 | 3.62 | 9 |
| 25.01 | 3.56 | 4 |
| 25.41 | 3.51 | 7 |
| 25.95 | 3.43 | 6 |
| 27.89 | 3.20 | 7 |
| 28.20 | 3.17 | 6 |
| 28.58 | 3.12 | 10 |
| 30.02 | 2.98 | 6 |
| 31.36 | 2.85 | 2 |
| 31.95 | 2.80 | 2 |
| 33.08 | 2.71 | 3 |
| 34.96 | 2.57 | 2 |
| 36.23 | 1.48 | 1 |

| | | |
|---|---|---|
| ¹The 2θ values were obtained using copper radiation (Cu_{Kα}). | | |

### Example 6a: Form B (+)-O-desmethyl-Tramadol-Acetylsalicylic acid (1:1) salt

To an assay tube equipped with magnetic stirrer containing (+)-*O*-desmethyl-Tramadol (56 mg, 0.22 mmol) and Acetylsalicylic acid (41 mg, 0.23 mmol, 1.05 eq.), was added toluene (0.7 ml) at room temperature. The resultant slurry was stirred for 7 h. The white solid was filtered with a sintered funnel (porosity 4) and washed with toluene (0.5 ml). After vacuum drying at room temperature, salt form B of the (+)-*O*-desmethyl-Tramadol-Acetylsalicylic acid (1:1) was obtained (95 mg, quantitative yield).

### Example 6b: Form B (+)-O-desmethyl-Tramadol Acetylsalicylic acid (1:1) salt

To an assay tube equipped with magnetical stirrer containing (+)-*O*-desmethyl-Tramadol (55 mg, 0.22 mmol) and Acetylsalycilic acid (41 mg, 0.23 mmol, 1.05 eq.), was added IPA (isopropyl alcohol) (0.5 ml) at room temperature. The resultant solution was stirred and in a few minutes precipitation of a white solid was observed. After 1 h of stirring, the white suspension was diluted with additional IPA (isopropyl alcohol) (0.5 ml) and filtered with a sintered funnel (porosity 4), before washing with IPA (isopropyl alcohol) (0.3 ml). After vacuum drying at room temperature, salt form B (+)-*O*-desmethyl-Tramadol-Acetylsalicylic acid (1:1) was obtained (71 mg, 75 % yield).

### ¹H NMR

Proton nuclear magnetic resonance analysis was recorded in deuterated DMSO (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in d6-DMSO at 400 MHz shows peaks identical to those of form A (+)-*O-*desmethyl-Tramadol-Acetylsalicylic acid (1:1).

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3376.3 (m, br), 2948.4 (m), 2923.9 (m), 1746.0 (s), 1611.2 (m), 1566.9 (m), 1478.6 (m), 1448.1 (m), 1379.7 (m), 1278.7 (m), 1241.0 (m), 1220.1 (m), 1189.6 (s), 1159.8 (m), 1090.2 (m), 1005.3 (m), 915.4 (m), 867.5 (m), 806.2 (m), 788.7 (m), 761.9 (s), 707.0 (m), 674.3 (m), 645.3 (m), 539.5 (m) cm⁻¹.

### DSC

DSC analysis was recorded with a Mettler DSC822^{e}. A sample of 1.4380 mg was weighed into a 40 µl aluminium crucible with a pinhole lid, and was heated, under nitrogen (50 ml/min), at 10 °C/min from 30 to 200 °C.

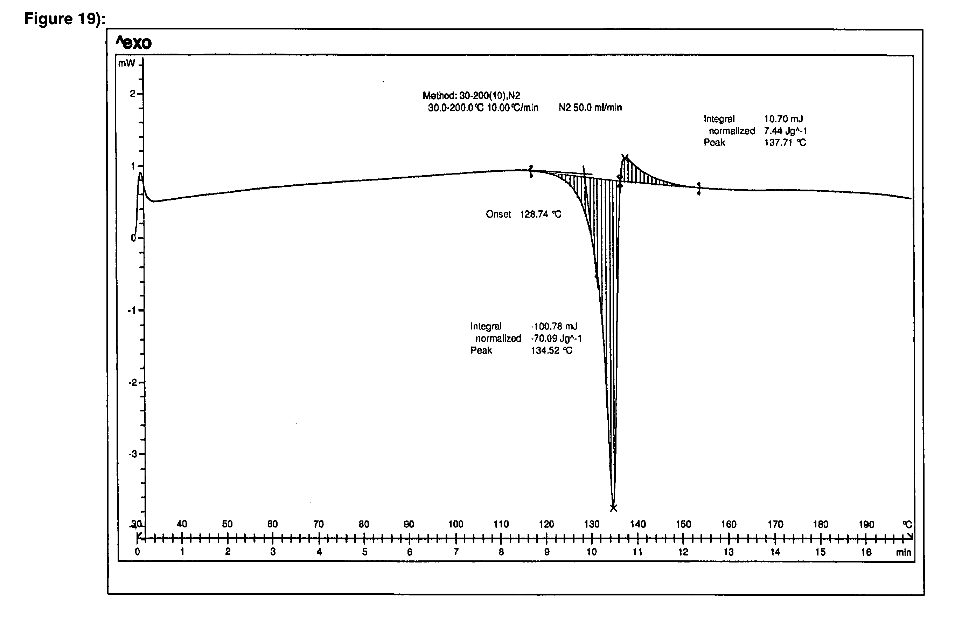

The novel type of crystal of the present invention is **characterized in that** the endothermic sharp peak corresponding to the melting point has an onset at 128.74 °C (fusion enthalpy -70.09 J/g), measured by DSC analysis (10 °C/min) (see figure 19).

### TG

Thermogravimetric analysis was recorded in a thermogravimetric analyzer Mettler TGA/SDTA851^{e}. A sample of 7.6657 mg was weighed into a 70 µl alumina crucible with a pinhole lid, and was heated at 10 °C/min from 30 to 200 °C, under nitrogen (50 ml/min).

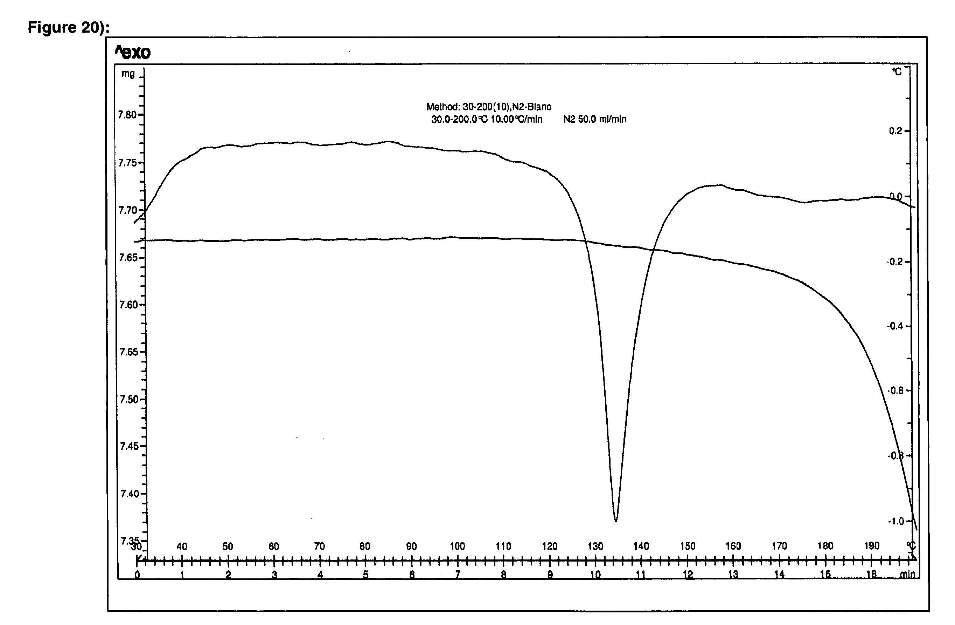

The TG analysis of the crystalline form according to the invention shows no weight loss at temperatures lower than the melting point (see figure 20).

### XRPD

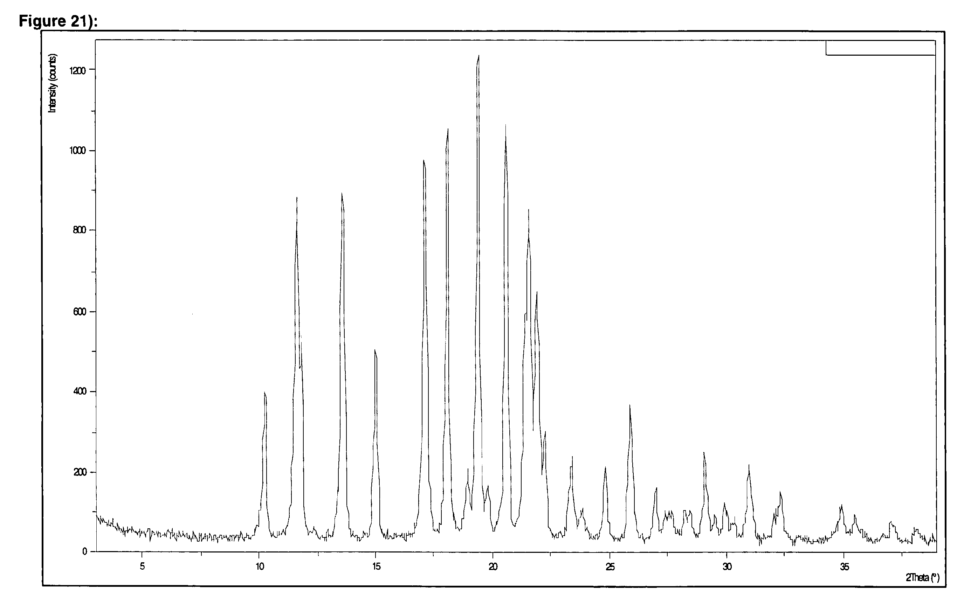

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 21).

### List of selected peaks:

| **2**θ **(°)¹** | **d (Å)** | **I (%)** |
|---|---|---|
| 10.28 | 8.60 | 30 |
| 11.65 | 7.60 | 70 |
| 11.85 | 7.47 | 43 |
| 13.63 | 6.50 | 69 |
| 15.04 | 5.89 | 38 |
| 17.15 | 5.17 | 78 |
| 18.11 | 4.90 | 84 |
| 18.95 | 4.68 | 14 |
| 19.42 | 4.57 | 100 |
| 19.82 | 4.48 | 11 |
| 20.62 | 4.30 | 84 |
| 21.38 | 4.16 | 42 |
| 21.60 | 4.11 | 67 |
| 21.94 | 4.05 | 47 |
| 22.25 | 3.40 | 22 |
| 23.41 | 3.80 | 17 |
| 23.83 | 3.73 | 6 |
| 24.83 | 3.59 | 15 |
| 25.88 | 3.44 | 22 |
| 27.01 | 3.30 | 10 |
| 27.65 | 3.22 | 5 |
| 18.34 | 3.15 | 5 |
| 29.09 | 3.07 | 18 |
| 29.90 | 2.99 | 8 |
| 30.96 | 2.89 | 15 |
| 32.32 | 2.77 | 9 |
| 34.91 | 2.57 | 7 |
| 35.60 | 2.52 | 3 |
| 37.10 | 2.42 | 4 |
| 38.05 | 2.37 | 3 |

| | | |
|---|---|---|
| ¹The 2θ values were obtained using copper radiation (Cu_{Kα}). | | |

### Example 7a: (rac)-O-desmethyl-Tramadol-Triflusal (1:1) salt

To an assay tube equipped with a magnetic stirrer containing (*rac*)-*O*-desmethyl-Tramadol (40 mg, 0.16 mmol) and Triflusal (40 mg, 0.16 mmol, 1 eq.), was added isobutyl acetate (0.5 ml). The resultant white suspension was stirred for 1.5 h at room temperature, 2 h at 0-4 °C and maintained for 15 h at 2 °C (without stirring). The white solid was filtered with a sintered funnel (porosity 4). After vacuum drying at room temperature, salt form (*rac*)-*O*-desmethyl-Tramadol-Triflusal (1:1) was obtained (26 mg, 33 % yield).

### Example 7b: (rac)-O-desmethyl-Tramadol-Triflusal (1:1) salt

To an assay tube containing (*rac*)-*O*-desmethyl-Tramadol (49 mg, 0.20 mmol) and Triflusal (49 mg, 0.20 mmol, 1 eq.), was added ethyl acetate (0.4 ml) at room temperature. The resultant suspension was cooled to 0-4 °C with an ice-water bath and stirred 40 min. Then, the suspension was seeded with form obtained in example 7a, stirred 2.5 h at 0 °C and maintained 18 h at -20 °C (without stirring). The white solid was filtered with a sintered funnel (porosity 4). After vacuum drying at room temperature, salt form (*rac*)-*O*-desmethyl-Tramadol-Triflusal (1:1) was obtained (75 mg, 77 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in CDCl₃ at 400 MHz shows peaks at 8.01 (d, *J* = 8.4 Hz, 1H); 7.47 (d, *J* = 8.0 Hz, 1H); 7.30 (s, 1H), 7.19 (t, *J* = 8.0 Hz, 1H); 7.13 (s, 1H); 6.88 (d, *J* = 7.2 Hz, 1H); 6.76 (dd, *J* = 8.0 Hz, *J* = 2.4 Hz, 1H); 2.86-2.79 (m, 1H); 2.66-2.62 (m, 1H); 2.50 (s (br), 6H); 2.30 (s, 3H); 2.24-2.15 (m, 2H); 1.87-1.75 (m, 3H); 1.74-1.57 (m, 4H); 1.45-1.33 (m, 1H) ppm.

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3384.6 (m, br), 3220.5 (m, br), 1770.2 (m), 1617. (s), 1594.3 (s), 1440.1 (m), 1410.6 (m), 1371.1 (m), 1330.9 (s), 1277.7 (m), 1234.7 (m), 1214.5 (s), 1195.5 (s), 1168.0 (m), 1155.5 (m), 1131.8 (m), 1105.0 (m), 941.1 (m), 868.1 (m), 706.3 (m) cm⁻¹.

### DSC

DSC analysis was recorded with a Mettler DSC822^{e}. A sample of 1.6550 mg was weighed into a 40 µl aluminium crucible with a pinhole lid, and was heated, under nitrogen (50 ml/min), at 10 °C/min from 30 to 200 °C.

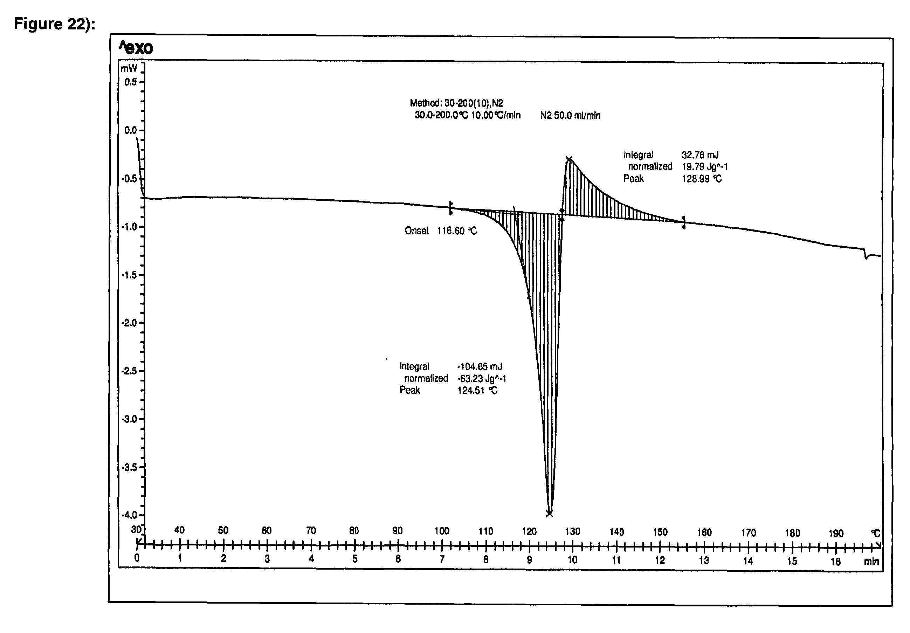

The novel type of crystal of the present invention is **characterized in that** the endothermic sharp peak corresponding to the melting point has an onset at 116.60 °C (fusion enthalpy -63.23 J/g), measured by DSC analysis (10 °C/min) (see figure 22).

### TG

Thermogravimetric analysis was recorded in a thermogravimetric analyzer Mettler TGA/SDTA851^{e}. A sample of 2.724-8 mg was weighed into a 70 µl alumina crucible with a pinhole lid, and was heated at 10 °C/min from 30 to 200 °C, under nitrogen (50 ml/min).

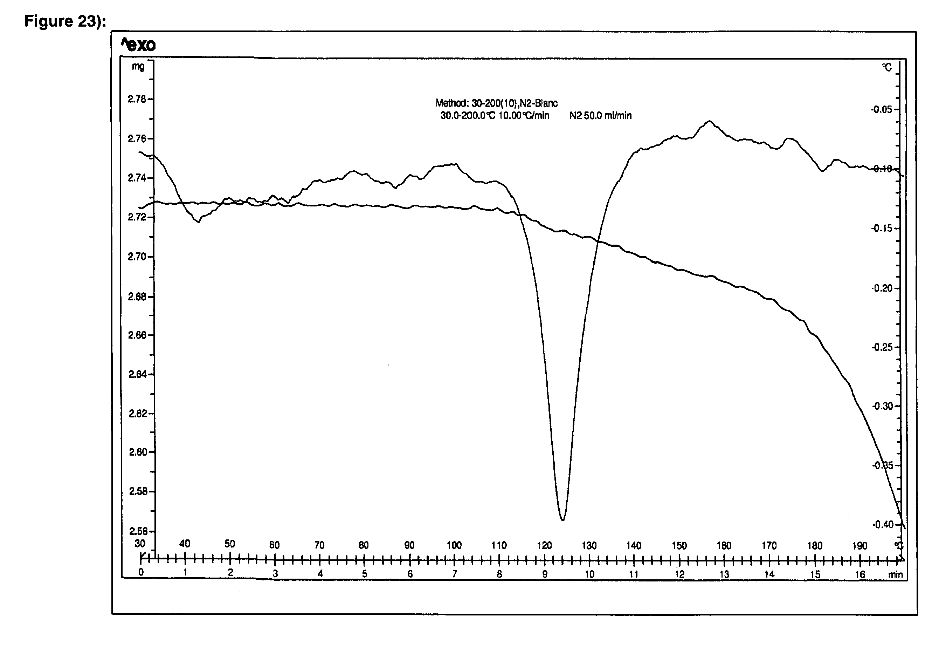

The TG analysis of the crystalline form according to the invention shows no weight loss at temperatures lower than the melting point (see figure 23).

### XRPD

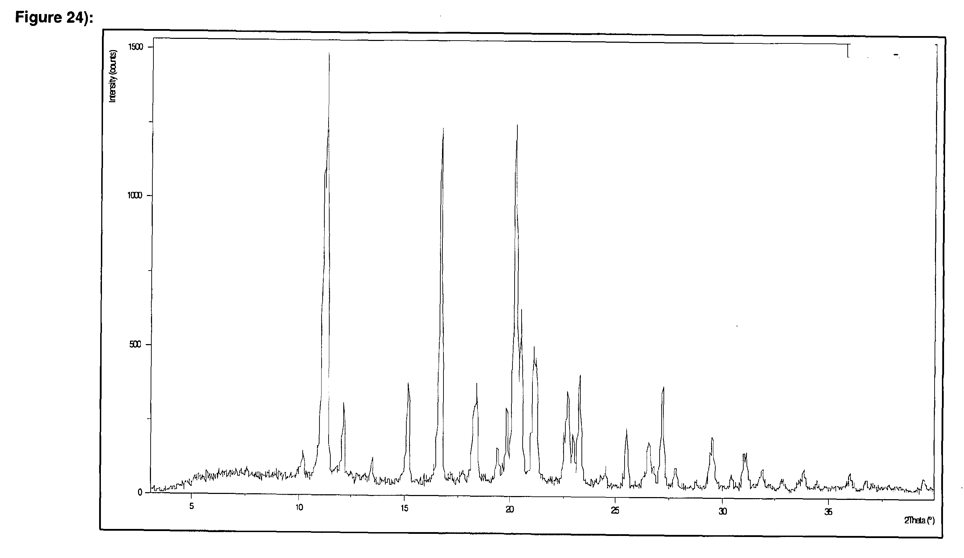

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 34.7° per minute (see figure 24).

### List of selected peaks:

| **2**θ **(°)¹** | **d (Å)** | **I (%)** |
|---|---|---|
| 10.22 | 8.66 | 6 |
| 11.19 | 7.91 | 70 |
| 11.35 | 7.80 | 100 |
| 12.17 | 7.27 | 19 |
| 13.48 | 6.57 | 5 |
| 15.19 | 5.83 | 25 |
| 16.73 | 5.30 | 85 |
| 18.44 | 4.81 | 14 |
| 19.44 | 4.57 | 9 |
| 19.84 | 4.47 | 18 |
| 20.21 | 4.39 | 86 |
| 10.54 | 4.32 | 42 |
| 21.11 | 4.21 | 32 |
| 21.26 | 4.18 | 27 |
| 22.76 | 3.91 | 22 |
| 22.99 | 3.87 | 12 |
| 23.29 | 3.82 | 27 |
| 24.51 | 3.63 | 4 |
| 25.49 | 3.49 | 15 |
| 26.59 | 3.35 | 10 |
| 27.19 | 3.28 | 25 |
| 27.84 | 3.21 | 5 |
| 29.57 | 3.02 | 12 |
| 31.09 | 2.88 | 8 |
| 31.85 | 2.81 | 4 |
| 33.87 | 2.65 | 4 |
| 36.04 | 1.49 | 3 |
| 39.50 | 2.28 | 3 |

| | | |
|---|---|---|
| ¹The 2θ values were obtained using copper radiation (Cu_{Kα}). | | |

### Example 8a: (+)-O-desmethyl-Tramadol-Triflusal (1:1) salt

To an assay tube equipped with magnetic stirrer containing (+)-*O*-desmethyl-Tramadol (53 mg, 0.21 mmol) and Triflusal (53 mg, 0.21 mmol, 1 eq.), was added isobutyl acetate (0.5 ml). The resultant mixture was stirred 1 h at room temperature and 4 h at (-5)-(-10) °C. The white solid was filtered with a sintered funnel (porosity 4) and washed with cold isobutyl acetate (0.5 ml). After vacuum drying at room temperature, salt form (+)-*O*-desmethyl-Tramadol-Triflusal (1:1) was obtained as a white solid (59 mg, 56 % yield).

### Example 8b: (+)-O-desmethyl-Tramadol-Triflusal (1:1) salt

To an assay tube equipped with magnetic stirrer containing (+)-*O*-desmethyl-Tramadol (43 mg, 0.17 mmol) and Triflusal (43 mg, 0.17 mmol, 1 eq.), was added dichloromethane (0.5 ml). The resultant solution was stirred for 0.5 h at room temperature and 0.5 h at (-5)-(-10) °C before seeding with form obtained in example 8a at (-5)-(-10) °C. Then, the mixture was allowed to stir at this temperature for 1.5 h. The solid was filtered with a sintered funnel (porosity 4) and washed with cold dichloromethane (0.5 ml). After vacuum drying at room temperature, salt form (+)-*O*-desmethyl-Tramadol-Triflusal (1:1) was obtained as a white solid (70 mg, 81 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in CDCl₃ at 400 MHz shows peaks identical to those of salt form (*rac*)-*O* desmethyl-Tramadol-Triflusal (1:1) (see example 7).

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3375.5 (m, br), 3186.5 (m, br), 2940.0 (m), 1775.5 (s), 1610.6 (s), 1592.3 (s), 1566.6 (s), 1478.6 (m), 1444.6 (s), 1410.6 (m), 1374.4 (s), 1329.2 (s), 1278.7 (m), 1209.1 (s), 1168.2 (s), 1138.9 (s), 1126.0 (s), 1105.7 (s), 1067.5 (m), 1013.1 (m) 1002.9 (m), 943.6 (s), 903.6 (m), 867.0 (m), 859.5 (s), 818.9 (s), 788.8 (m), 758.4 (m), 707.1 (m) cm⁻¹.

### DSC

DSC analysis was recorded with a Mettler DSC822^{e}. A sample of 1.6020 mg was weighed into a 40 µl aluminium crucible with a pinhole lid, and was heated, under nitrogen (50 ml/min), at 10 °C/min from 30 to 200 °C.

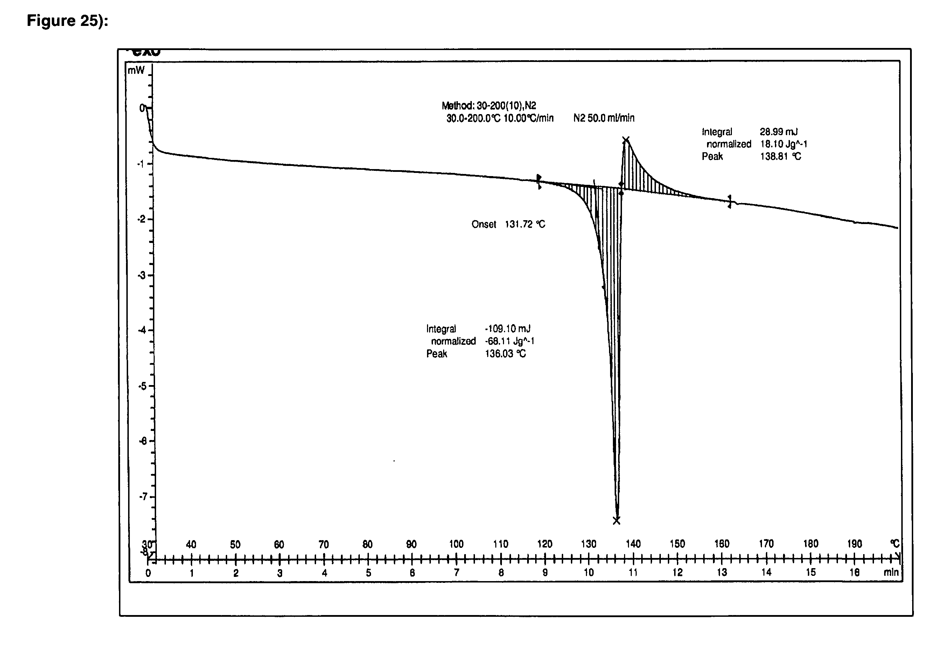

The novel type of crystal of the present invention is **characterized in that** the endothermic sharp peak corresponding to the melting point has an onset at 131.72 °C (fusion enthalpy -68.11 J/g), measured by DSC analysis (10 °C/min) (see figure 25).

### TG

Thermogravimetric analysis was recorded in a thermogravimetric analyzer Mettler TGA/SDTA851^{e}. A sample of 3.8055 mg was weighed into a 70 µl alumina crucible with a pinhole lid, and was heated at 10 °C/min from 30 to 200 °C, under nitrogen (50 ml/min).

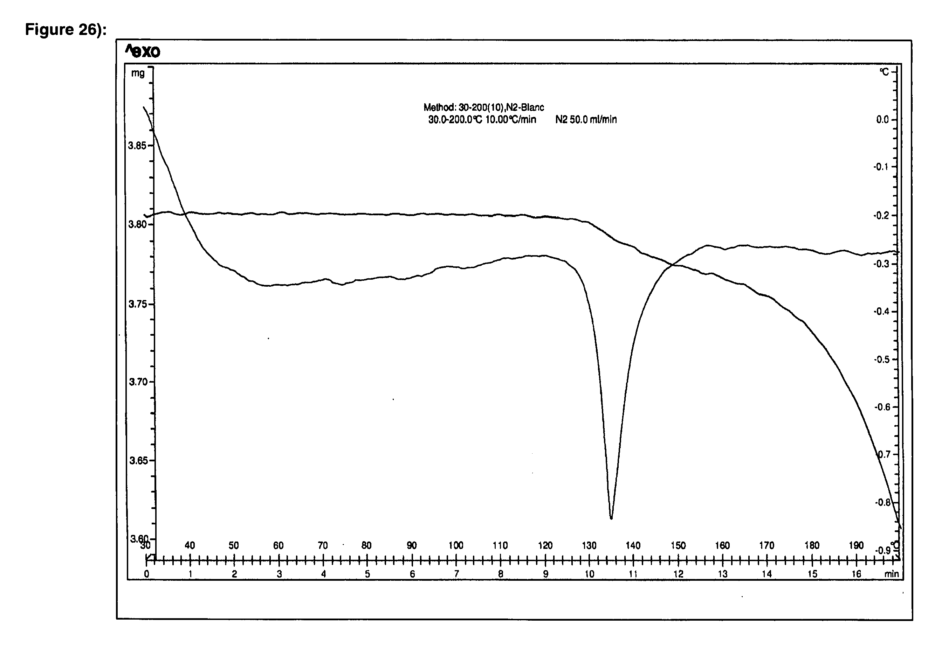

The TG analysis of the crystalline form according to the invention shows no weight loss at temperatures lower than the melting point (see figure 26).

### XRPD

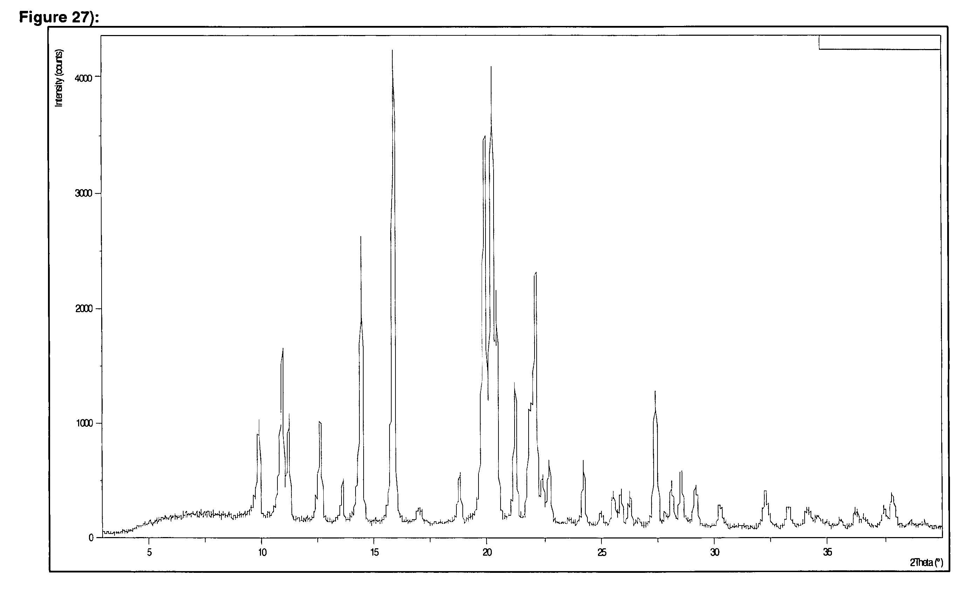

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 27).

### List of selected peaks:

| **2**θ **(°)¹** | **d (Å)** | **I (%)** |
|---|---|---|
| 9.93 | 8.90 | 20 |
| 10.96 | 8.07 | 37 |
| 11.25 | 7.87 | 22 |
| 12.64 | 7.00 | 20 |
| 13.62 | 6.50 | 9 |
| 14.47 | 6.12 | 62 |
| 15.90 | 5.57 | 100 |
| 16.99 | 5.22 | 3 |
| 18.78 | 4.73 | 11 |
| 19.91 | 4.46 | 85 |
| 20.22 | 4.39 | 99 |
| 20.43 | 4.35 | 50 |
| 21.25 | 4.18 | 29 |
| 21.85 | 4.07 | 24 |
| 22.14 | 4.01 | 54 |
| 22.42 | 3.97 | 11 |
| 22.72 | 3.91 | 14 |
| 24.26 | 3.67 | 13 |
| 24.99 | 3.56 | 3 |
| 25.55 | 3.49 | 7 |
| 25.87 | 3.44 | 7 |
| 26.27 | 3.39 | 7 |
| 27.42 | 3.25 | 29 |
| 18.11 | 3.17 | 10 |
| 28.53 | 3.13 | 12 |
| 29.18 | 3.06 | 9 |
| 30.23 | 2.96 | 5 |
| 32.23 | 2.78 | 8 |
| 33.20 | 2.70 | 4 |
| 33.31 | 2.69 | 4 |
| 34.06 | 2.63 | 4 |
| 34.57 | 2.59 | 2 |
| 35.56 | 2.52 | 2 |
| 36.20 | 2.48 | 4 |
| 36.58 | 2.46 | 2 |
| 37.45 | 2.40 | 4 |
| 37.77 | 2.38 | 6 |

| | | |
|---|---|---|
| ¹The 2θ values were obtained using copper radiation (Cu_{Kα}). | | |

### Example 9a: Form A (+)-O-desmethyl-Tramadol-(S)-Naproxen (1:1) salt

To an assay tube equipped with magnetic stirrer containing (+)-*O*-desmethyl-Tramadol (52 mg, 0.21 mmol) and (*S*)-Naproxen (48 mg, 0.11 mmol, 1 eq.) was added CH₃CN (acetonitrile) (0.2 ml). The resultant mixture was heated until reach complete dissolution. Then, the solution was stirred and slowly evaporated at room temperature. After 5 days a gel was formed and was dissolved in CH₃CN (acetonitrile) (0.2 ml). Immediately, a white precipitate appeared. The solid was filtered with a sintered funnel (porosity 4), washed with CH₃CN (acetonitrile) (0.3 ml) and dried under vacuum at room temperature to give salt form A (+)-*O*-desmethyl-Tramadol-(*S*)-Naproxen (1:1) (86 mg, 86 % yield).

### Example 9b: Form A (+)-O-desmethyl-Tramadol-(S)-Naproxen (1:1) salt

To an assay tube equipped with magnetic stirrer containing (+)-*O*-desmethyl-Tramadol (52 mg, 0.11 mmol) and (*S*)-Naproxen (48 mg, 0.21 mmol, 1 eq.), was added methyl isobutyl ketone (0.3 ml). The resultant mixture was heated until reach complete dissolution. After cooling to room temperature, the solution was stirred overnight. A white precipitate appeared during the night and was filtered with a sintered funnel (porosity 4), washed with methyl isobutyl ketone (0.3 ml) and dried under vacuum at room temperature to give salt form A (+)-*O*-desmethyl-Tramadol-(*S*)-Naproxen (1:1), as a white solid (90 mg, 90 % yield).

### Example 9c: Form A (+)-O-desmethyl-Tramadol-(S)-Naproxen (1:1) salt

To an assay tube equipped with magnetic stirrer containing (+)-*O*-desmethyl-Tramadol (52 mg, 0.21 mmol) and (*S*)-Naproxen (48 mg, 0.21 mmol, 1 eq.) was added toluene (0.8 ml). The resultant mixture was heated until reach complete dissolution. The solution was cooled to room temperature and stirred overnight. A white precipitate was formed. The solid was filtered with a sintered funnel (porosity 4), washed with toluene (0.3 ml) and dried under vacuum at room temperature to give salt form A (+)-*O*-desmethyl-Tramadol-(*S*)-Naproxen (1:1) (90 mg, 90 % yield).

### Example 9d: Form A (+)-O-desmethyl-Tramadol-(S)-Naproxen (1:1) salt

To an assay tube equipped with magnetic stirrer containing (+)-*O*-desmethyl-Tramadol (50 mg, 0.20 mmol) was added (*S*)-Naproxen (50 mg, 0.22 mmol, 1.1 eq.) dissolved in Et₂O (Diethyl ether) (2 ml). The resultant suspension was stirred at room temperature for 2 h. The white solid was filtered with a sintered funnel (porosity 4), washed with Et₂O (Diethyl ether) (0.4 ml) and dried under vacuum at room temperature to give salt form A (+)-*O*-desmethyl-Tramadol-(*S*)-Naproxen (1:1) (84 mg, 88 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving *5*-10 mg of sample in 0.6 ml of deuterated solvent.

¹H NMR spectrum in CDCl₃ at 400 MHz shows peaks at 7.70 (s, 1H); 7.66-7.64 (m, 2H); 7.46 (dd, *J* = 8.4 Hz and 2.0 Hz, 1H); 7.19-7.09 (m, 4H); 6.89 (d, *J* = 7.6 Hz, 1H); 6.72 (dd, *J* = 8.0 Hz and 2.4 Hz, 1H); 3.90 (s, 3H); 3.84 (q, *J* = 7.1 Hz, 1H); 2.52 (dd, *J* = 13.6 Hz and 6.0 Hz, 1H); 2.24 (dd, *J* = 13.8 Hz and 1.8 Hz, 1H); 2.18 (s, 6H); 1.99-1.91 (m, 1H); 1.88-1.49 (m, 10H); 1.31-1.18 (m, 1H) ppm.

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3360.7 (m, br), 2959.4 (m), 2922.8 (m), 2857.0 (m), 1630.6 (m), 1604.8 (s), 1570.2 (s), 1505.2 (m), 1480.7 (s), 1449.8 (s), 1388.6 (s), 1356.8 (s), 1264.4 (m), 1227.1 (s), 1029.2 (m), 926.3 (m), 864.3 (s), 852.2 (m), 786.7 (m), 705.9 (m) cm⁻¹.

### DSC

DSC analysis was recorded with a Mettler DSC822^{e}. A sample of 1.2900 mg was weighed into 40 µl aluminium crucible with a pinhole lid and was heated, under nitrogen (50 ml/min), at 10 °C/min from 30 to 200 °C.

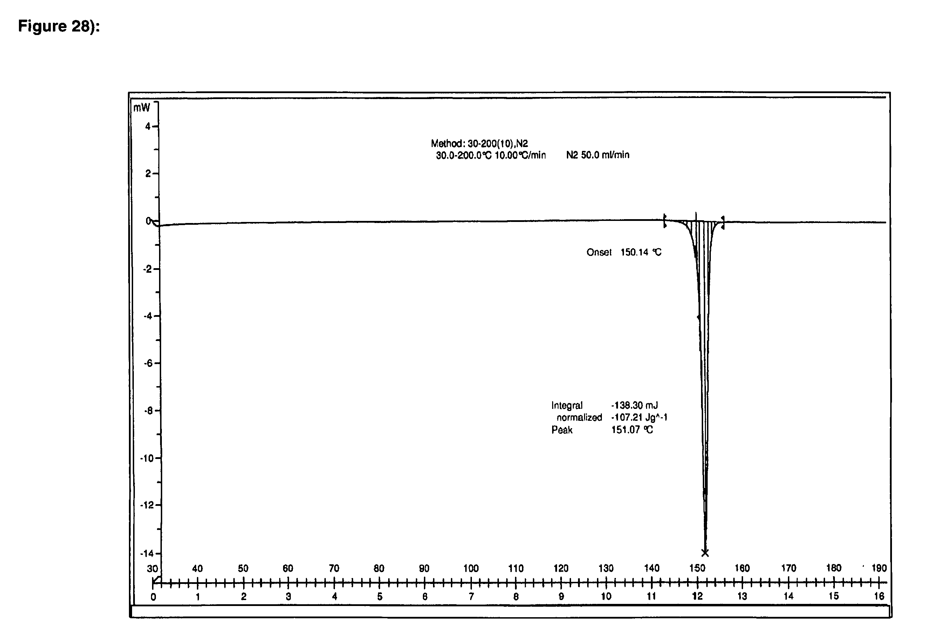

The novel type of crystal of the present invention is **characterized in that** the endothermic sharp peak corresponding to the melting point has an onset at 150.14 °C (fusion enthalpy -107.21 J/g), measured by DSC analysis (10 °C/min) (see figure 28).

### TG

Thermogravimetric analysis was recorded in a thermogravimetric analyzer Mettler TGA/SDTA851^{e}. A sample of 1.4196 mg was weighed into a 70 µl aluminium crucible with a pinhole lid and was heated at 10 °C/min from 30 to 200 °C, under nitrogen (50 ml/min).

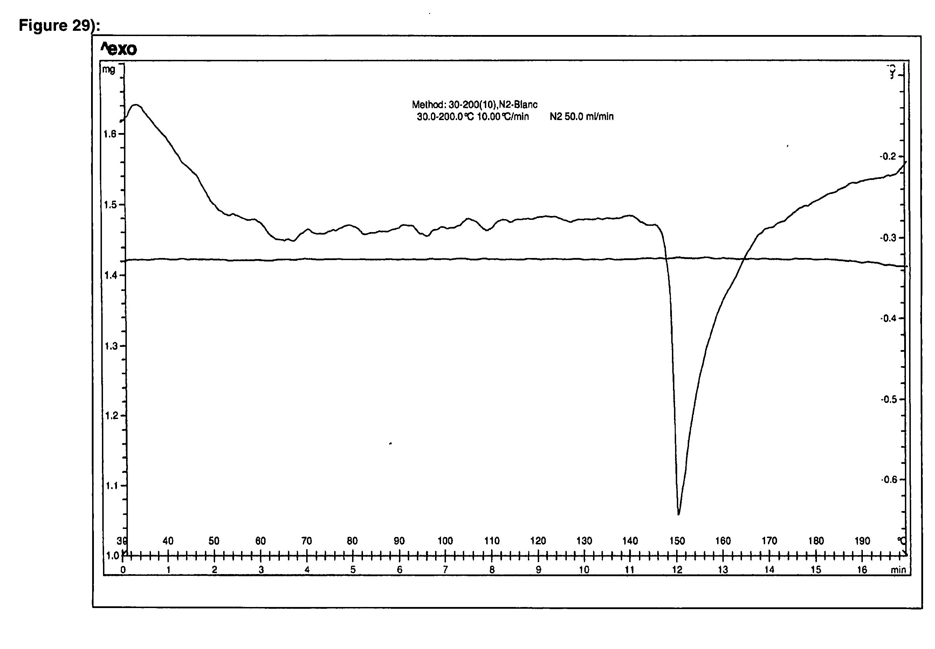

The TG analysis of crystalline form A (+)-*O*-desmethyl-Tramadol-(*S*)-Naproxen (1:1) salt according to the invention shows no weight loss between 30 and 200 °C (see figure 29).

### XRPD

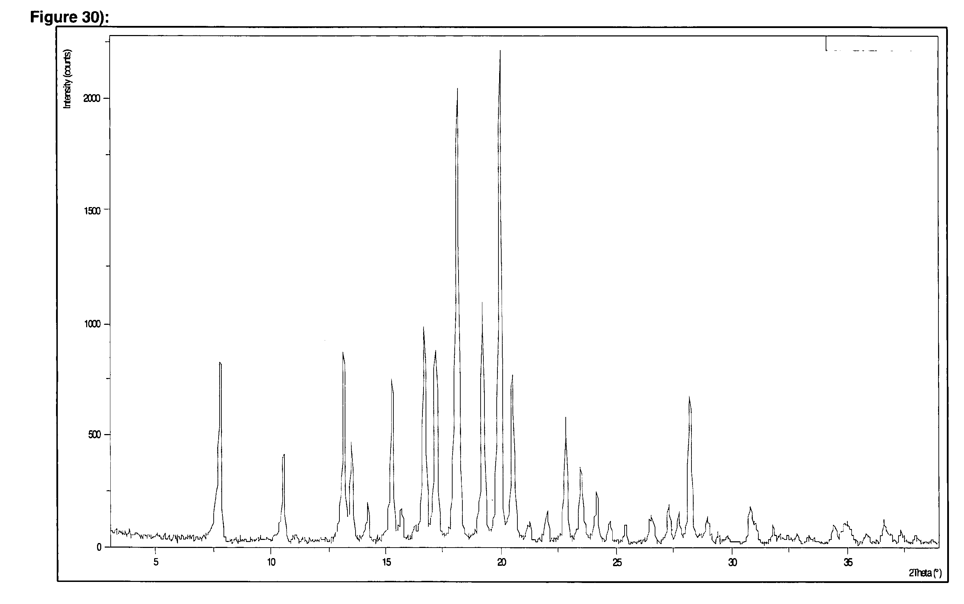

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 30).

### List of selected peaks:

| **2**θ **(°)¹** | **d (Å)** | **I (%)** |
|---|---|---|
| 7.83 | 11.29 | 36 |
| 10.57 | 8.37 | 18 |
| 13.19 | 6.71 | 39 |
| 13.53 | 6.54 | 20 |
| 14.22 | 6.23 | 8 |
| 15.29 | 5.80 | 33 |
| 15.68 | 5.65 | 7 |
| 16.69 | 5.31 | 44 |
| 17.19 | 5.16 | 39 |
| 18.14 | 4.89 | 88 |
| 19.22 | 4.62 | 48 |
| 19.98 | 4.44 | 100 |
| 20.51 | 4.33 | 34 |
| 21.24 | 4.18 | 4 |
| 21.97 | 4.05 | 6 |
| 22.81 | 3.90 | 25 |
| 23.47 | 3.79 | 15 |
| 24.18 | 3.68 | 9 |
| 24.73 | 3.60 | 3 |
| 25.42 | 3.50 | 3 |
| 26.56 | 3.36 | 4 |
| 27.26 | 3.27 | 7 |
| 27.69 | 3.22 | 6 |
| 28.20 | 3.16 | 29 |
| 28.99 | 3.08 | 4 |
| 30.82 | 2.90 | 7 |
| 31.87 | 2.81 | 2 |
| 34.44 | 2.60 | 3 |
| 35.02 | 2.56 | 4 |
| 35.85 | 2.51 | 1 |
| 36.59 | 2.46 | 5 |
| 37.34 | 2.41 | 2 |
| 37.97 | 2.37 | 1 |

| | | |
|---|---|---|
| ¹The 2θ values were obtained using copper radiation (Cu_{Kα}). | | |

### Single crystal X-ray diffraction

The crystal structure of form A (+)-*O*-desmethyl-Tramadol-(*S*)-Naproxen (1:1) salt has been determined from single crystal X-ray diffraction data. The colourless prism used (0.55 x 0.39 x 0.07 mm) was obtained from the evaporation of a MIK (methyl isobutyl ketone) solution of equimolar amounts of (+)-*O*-desmethyl-Tramadol and (*S*)-Naproxen.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters, except the disordered carbon atom of the methoxy group.

### Relevant structural data:

| Crystal system | Monoclinic |
|---|---|
| Space group | *P*2₁ |
| a (Å) | 9.4093(9) |
| b (Å) | 12.4.375(11) |
| c (Å) | 11.5172(10) |
| β (°) | 101.423(2) |
| Volume (Å³) | 1321.1(2) |
| Z | 2 |
| D calc. (Mg/m³) | 1.206 |
| N. of refl. | 5982 |
| Refl. with I > 2σ(I) | 3858 |
| R (I > 2σ(I)) | 0.0520 |

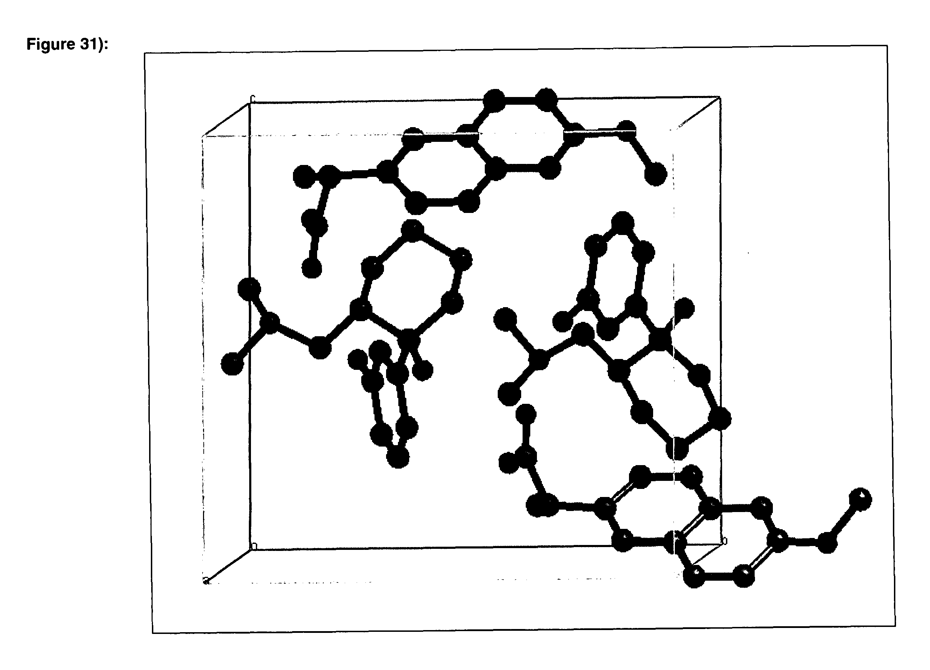

The crystal structure of form A (+)-*O*-desmethyl-Trainadol-(*S*)-Naproxen (1:1) salt is depicted in figure 31.
Simulation of the XRPD diffractogram from single crystal data gives an almost identical diagram to the experimental one presented above.

### Example 10a: Form B: (+)-O-desmethyl-Tramadol-(S)-Naproxen (1:1) salt

To an assay tube equipped with a magnetic stirrer containing (+)-*O*-desmethyl-Tramadol (52 mg, 0.21 mmol) and (*S*)-Naproxen (48 mg, 0.21 mmol, 1 eq.) was added toluene (0.8 ml). The resultant mixture was heated until reach complete dissolution. The solution was cooled to room temperature and stirred overnight. A white precipitate was formed. The solid was filtered with a sintered funnel (porosity 4), washed with toluene (0.2 ml) and dried under vacuum at room temperature to give salt form B (+)-*O*-desmethyl-Tramadol-(*S*)-Naproxen (1:1), as a white solid (82 mg, 82 % yield).

### Example 10b: Form B: (+)-O-desmethyl-Tramadol-(S)-Naproxen (1:1) salt

To an assay tube equipped with a magnetic stirrer containing (+)-*O*-desmethyl-Tramadol (52 mg, 0.21 mmol), was added AcOiBu (isobutyl acetate) (0.2 ml). The resultant suspension was cooled to 0-5 °C and seeded with form obtained in example 10a before adding dropwise (addition time 15 min) a solution of (*S*)-Naproxen (48 mg, 0.21 mmol, 1 eq.) diluted in AcOiBu (isobutyl acetate) (1 ml) (the solvent was previously heated to obtain complete dissolution of (*S*)-Naproxen). After 2 hours the white solid was filtered with a sintered funnel (porosity 4), washed with AcOiBu (isobutyl acetate) (0.3 ml) and dried under vacuum at room temperature to give salt form B (+)-*O*-desmethyl-Tramadol-(*S*)-Naproxen (1:1) (86 mg, 86 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in CDCl₃ at 400 MHz shows peaks identical to those of form A (see example 9)

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3354.6 (m, br), 3055.8 (m, br), 2938.4 (m), 1603.6 (s), 1479.8 (m), 1390.3 (s), 1359.0 (s), 1263.8 (s), 1213.7 (s), 1164.0 (s), 1118.8 (m), 1030.7 (s), 961.3 (m), 925.3 (m), 888.6 (m), 855.8 (s), 808.5 (m), 780.9 (m), 706.1 (m) cm⁻¹.

### DSC

A sample of 1.4560 mg was weighed into a 40 µl aluminium crucible with a pinhole lid, and was heated, under nitrogen (50 ml/min), at °C/min from 30 to 300 °C.

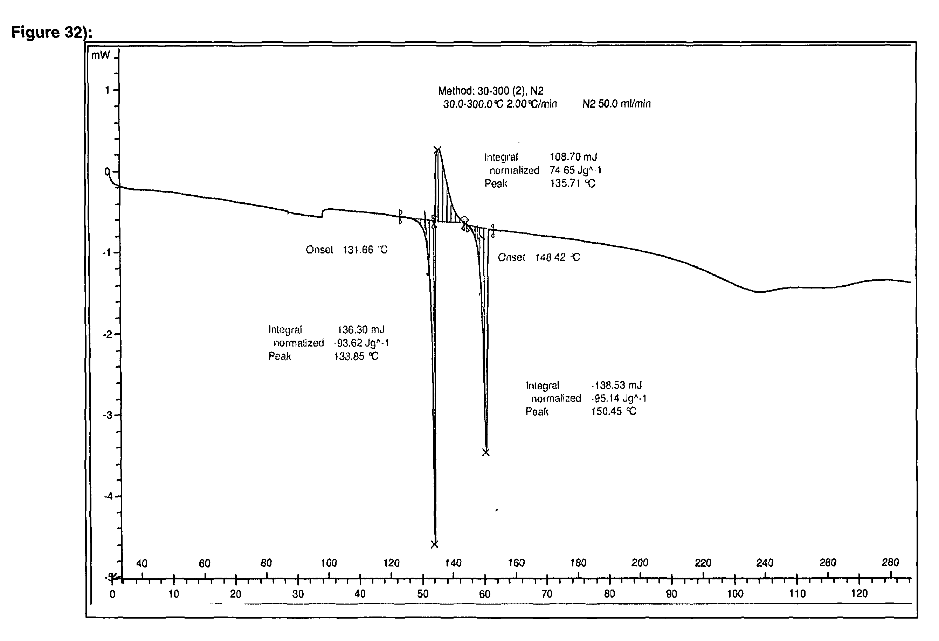

The novel type of crystal of the present invention (form B) is **characterized in that** the endothermic sharp peak corresponding to the melting point has an onset at 131.66 °C (fusion enthalpy -93.62 J/g), measured by DSC analysis (2 °C/min) (see figure 32). Another endothermic sharp peak is observed corresponding to the melting point of form A with an onset at 148.42 °C (fusion enthalpy -95.14 J/g) due to the transformation of form B to form A.

### TG

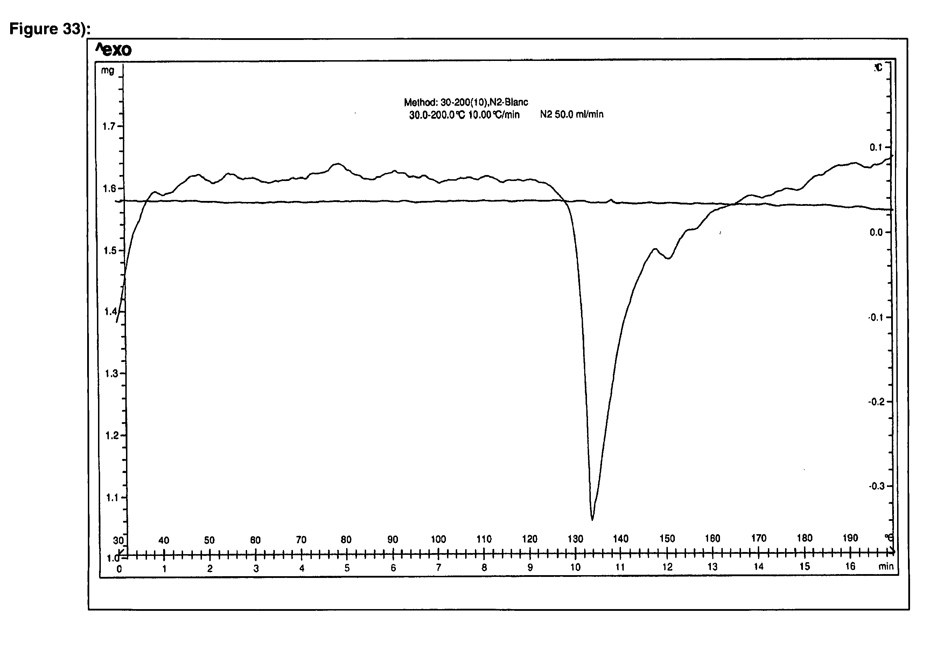

Thermogravimetric analysis was recorded in a thermogravimetric analyzer Mettler TGA/SDTA851^{e}. A sample of 1.5786 mg was weighed into a 70 µl aluminium crucible with a pinhole lid, and was heated at 10 °C/min from 30 to 200 °C, under nitrogen (50 ml/min).
The TG analysis of crystalline form B according to the invention shows no weight loss between 30 and 200 °C (see figure 33).

### XRPD

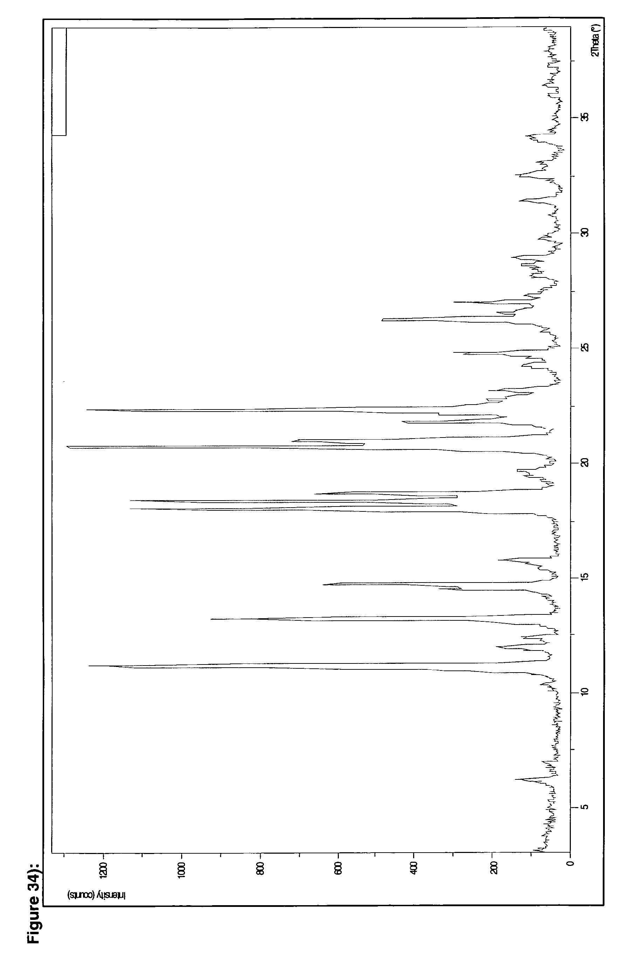

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 34).

### List of selected peaks:

| **2**θ **(°)¹** | **d (Å)** | **I (%)** |
|---|---|---|
| 6.21 | 14.24 | 7 |
| 11.22 | 7.88 | 92 |
| 11.97 | 7.40 | 11 |
| 12.41 | 7.13 | 6 |
| 13.26 | 6.68 | 70 |
| 14.51 | 6.10 | 20 |
| 14.75 | 6.00 | 48 |
| 15.80 | 5.61 | 12 |
| 18.03 | 4.92 | 87 |
| 18.38 | 4.83 | 87 |
| 18.71 | 4.74 | 49 |
| 19.70 | 4.51 | 8 |
| 20.72 | 4.29 | 100 |
| 21.06 | 4.22 | 43 |
| 21.82 | 4.07 | 32 |
| 22.34 | 3.98 | 95 |
| 23.20 | 3.83 | 13 |
| 24.28 | 3.67 | 7 |
| 24.80 | 3.59 | 18 |
| 26.28 | 3.39 | 36 |
| 27.03 | 3.30 | 22 |
| 28.15 | 3.17 | 5 |
| 28.94 | 3.09 | 9 |
| 29.82 | 3.00 | 3 |
| 31.42 | 2.85 | 8 |
| 32.54 | 2.75 | 8 |
| 34.20 | 2.62 | 6 |
| 35.98 | 2.50 | 2 |
| 36.38 | 2.47 | 1 |

| | | |
|---|---|---|
| ¹The 2θ values were obtained using copper radiation (Cu_{Kα}). | | |

### Example 11a: Form A (-)-O-desmethyl-Tramadol-(S)-Naproxen (1:1) salt

To an assay tube equipped with a magnetic stirrer containing (-)-*O*-desmethyl-Tramadol (52 mg, 0.21 mmol) and (*S*)-Naproxen (48 mg, 0.11 mmol, 1 eq.), was added CH₃CN (acetonitrile) (0.3 ml). The resultant mixture was heated until reach complete dissolution. The solution was slowly cooled to room temperature and, then, stirred 5 hours at room temperature (a white solid precipitated after 5 minutes). The precipitate was filtered with a sintered funnel (porosity 4), washed with CH₃CN (acetonitrile) (0.2 ml) and dried under vacuum at room temperature to give salt form A (-)-*O*-desmethyl-Tramadol-(*S*)-Naproxen (1:1), as a white solid (89 mg, 89 % yield).

### Example 11b: Form A (-)-O-desmethyl-Tramadol-(S)-Naproxen (1:1) salt

To an assay tube equipped with a magnetic stirrer containing (-)-*O*-desmethyl-Tramadol (52 mg, 0.21 mmol) and (*S*)-Naproxen (48 mg, 0.21 mmol, 1 eq.), was added methyl isobutyl ketone (0.3 ml) and heated until reach complete dissolution. The solution was slowly cooled to room temperature and, then, stirred at room temperature overnight. A white precipitate appeared during the night and was filtered with a sintered funnel (porosity 4), washed with methyl isobutyl ketone (0.2 ml) and dried under vacuum at room temperature to give salt form A (-)-*O-*desmethyl-Tramadol-(*S*)-Naproxen (1:1), as a white solid (86 mg, 86 % yield).

### Example 11c: Form A (-)-O-desmethyl-Tramadol-(S)-Naproxen (1:1) salt

To an assay tube equipped with a magnetic stirrer containing (-)-*O*-desmethyl-Tramadol (52 mg, 0.21 mmol) and (*S*)-Naproxen (48 mg, 0.21 mmol, 1 eq.), was added isopropanol (0.4 ml). The resultant solution was stirred at room temperature overnight. A white precipitate was formed. The solid was filtered with a sintered funnel (porosity 4), washed with isopropanol (0.2 ml) and dried under vacuum at room temperature to give salt form A (-)-*O*-desmethyl-Tramadol-(*S*)-Naproxen (1:1), as a white solid (74 mg, 74 % yield).

### Example 11d: Form A (-)-O-desmethyl-Tramadol-(S)-Naproxen (1:1) salt

To an assay tube equipped with a magnetic stirrer containing (-)-*O*-desmethyl-Tramadol (52 mg, 0.21 mmol) and (*S*)-Naproxen (48 mg, 0.21 mmol, 1 eq.) was added acetone (0.3 ml). The resultant solution was stirred at room temperature overnight. A white precipitate was formed. The solid was filtered with a sintered funnel (porosity 4), washed with acetone (0.2 ml) and dried under vacuum at room temperature to give salt form A (-)-*O*-desmethyl-Tramadol-(*S*)-Naproxen (1:1), as a white solid (74 mg, 74 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in CDCl₃ at 400 MHz shows peaks at = 7.67 (s, 1H); 7.62-7.58 (m, 2H); 7.46 (dd, *J* = 8.2 Hz, *J* = 1.8 Hz, 1H); 7.16-7.06 (m, 4H); 6.80 (d, *J* = 7.6 Hz, 1H); 6.72 (dd, *J =* 7.6 Hz, *J* = 2.0 Hz, 1H); 3.89 (s, 3H); 3.82 (q, *J* = 7.1 Hz, 1H); 2.60 (dd, *J* = 13.8 Hz, *J* = 7.0 Hz, 1H); 2.24 (dd, *J =* 13.2 Hz, *J =* 2.0 Hz, 1H); 2.21 (s, 6H); 1.96-1.87 (m, 1H); 1.78-1.42 (m, 10H); 1.29-1.15 (m, 1H) ppm.

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3359.1 (m, br), 3058.7 (m), 3003.7 (m), 2944.3 (m), 2864.2 (w), 1629.4 (m), 1603.9 (s), 1482.3 (m), 1448.1 (s), 1398.7 (s), 1359.8 (m), 1213.5 (s), 1166.5 (s), 1113.4 (s), 1031.9 (s), 1002.3 (m), 924.7 (m), 883.1 (s), 869.4 (s), 809.8 (s), 787.9 (s), 746.2 (m), 704.5 (s), 481.4 (s) cm⁻¹.

### DSC

DSC analysis was recorded with a Mettler DSC822^{e}. A sample of 1.5480 mg was weighed into a 40 µl aluminium crucible with a pinhole lid and was heated, under nitrogen (50 ml/min), at 10 °C/min from 30 to 200 °C.

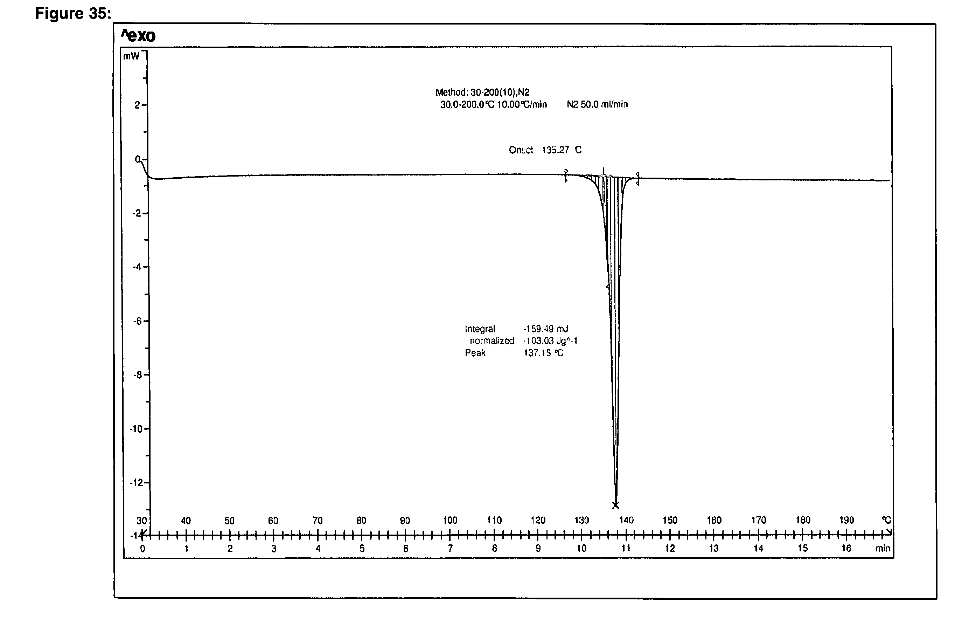

The novel type of crystal of the present invention form A (-)-*O*-desmethyl-Tramadol-(*S*)-Naproxen (1:1) salt is **characterized in that** the endothermic sharp peak corresponding to the melting point has an onset at 135.27 °C (fusion enthalpy -103.03 J/g), measured by DSC analysis (10 °C/min), see figure 35.

### TG

Thermogravimetric analysis was recorded with a thermogravimetric analyzer Mettler TGA/SDTA851^{e}. A sample of 3.2062 mg was weighed into a 70 µl aluminium crucible with a pinhole lid, and was heated at 10 °C/min from 30 to 200 °C, under nitrogen (50 ml/min).

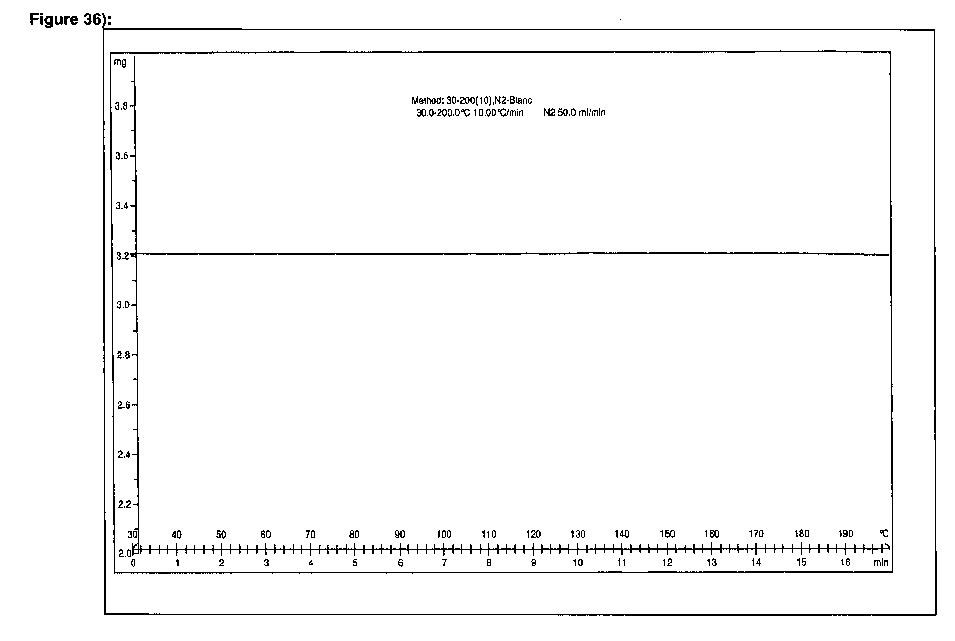

The TG analysis of crystalline form A (-)-*O*-desmethyl-Tramadol-(*S*)-Naproxen (1:1) salt according to the invention shows no weight loss between 30 and 200 °C (see figure 36).

### XRPD

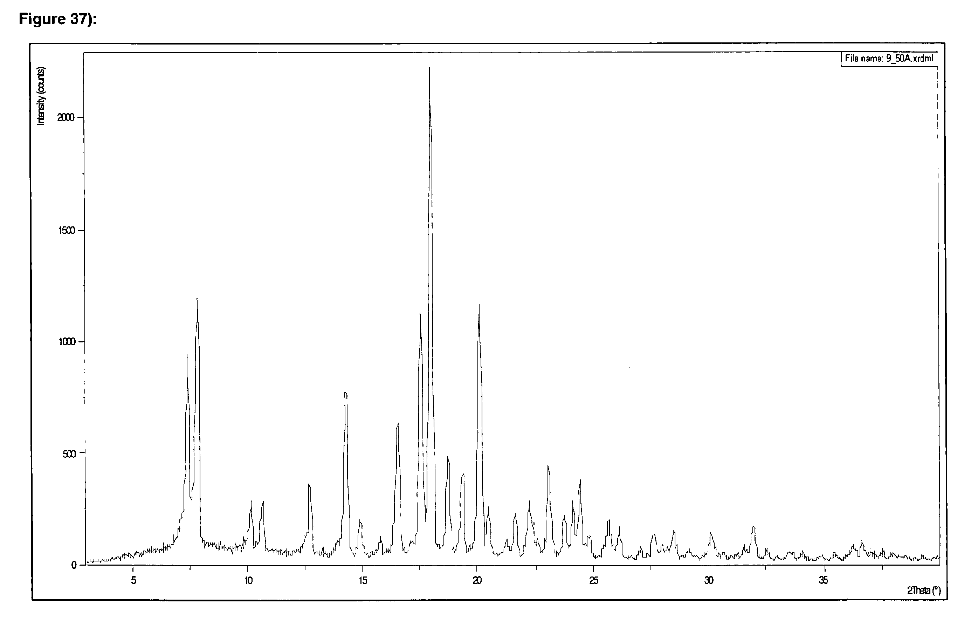

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 34.7° per minute (see figure 37).

### List of selected peaks:

| **2**θ **(°)¹** | **d (Å)** | **I (%)** |
|---|---|---|
| 7.46 | 11.85 | 41 |
| 7.90 | 11.20 | 55 |
| 10.21 | 8.67 | 10 |
| 10.72 | 8.25 | 11 |
| 12.77 | 6.93 | 13 |
| 14.32 | 6.18 | 35 |
| 14.93 | 5.93 | 7 |
| 15.82 | 5.60 | 3 |
| 16.59 | 5.34 | 23 |
| 17.58 | 5.05 | 52 |
| 18.03 | 4.92 | 100 |
| 18.75 | 4.73 | 21 |
| 19.35 | 4.59 | 17 |
| 20.14 | 4.41 | 53 |
| 20.50 | 4.33 | 9 |
| 21.23 | 4.19 | 3 |
| 21.66 | 4.10 | 9 |
| 22.24 | 4.00 | 11 |
| 23.10 | 3.85 | 19 |
| 23.78 | 3.74 | 9 |
| 24.16 | 3.68 | 12 |
| 24.46 | 3.64 | 17 |
| 24.84 | 3.58 | 5 |
| 25.69 | 3.47 | 8 |
| 26.17 | 3.41 | 5 |
| 27.04 | 3.30 | 2 |
| 27.67 | 3.22 | 5 |
| 28.50 | 3.13 | 6 |
| 29.17 | 3.06 | 1 |
| 30.15 | 2.96 | 5 |
| 31.99 | 2.80 | 7 |
| 32.54 | 2.75 | 2 |
| 36.67 | 2.45 | 3 |
| 37.56 | 2.39 | 1 |

| | | |
|---|---|---|
| ¹The 2θ values were obtained using copper radiation (Cu_{Kα}). | | |

### Example 12a: Form B (-)-O-desmethyl-Tramadol-(S)-Naproxen (1:1) salt

To an assay tube equipped with magnetic stirrer containing (-)-*O*-desmethyl-Tramadol (50 mg, 0.20 mmol) was added (*S*)-Naproxen (50 mg, 0.22 mmol, 1.1 eq.) dissolved in Et₂O (Diethyl ether) (2 ml). The resultant suspension was stirred 4.5 hours at room temperature. Then, the white solid was filtered with a sintered funnel (porosity 4), washed with Et₂O (Diethyl ether) (0.4 ml) and dried under vacuum at room temperature to give salt form B (-)-*O*-desmethyl-Tramadol-(*S*)-Naproxen (1:1) (79 mg, 83 % yield).

### Example 12b: Form B (-)-O-desmethyl-Tramadol-(S)-Naproxen (1:1) salt

To an assay tube equipped with magnetic stirrer containing (-)-*O*-desmethyl-Tramadol (52 mg, 0.21 mmol) and (*S*)-Naproxen (48 mg, 0.21 mmol, 1 eq.) was added toluene (0.8 ml). The resultant mixture was heated until reach complete dissolution. The solution was slowly cooled to room temperature and, then, seeded with a mixture of form obtained in example 12a and form B (-)-*O*-desmethyl-Tramadol- (*S*)-Naproxen (1:1) salt (obtained from crystallization (*rac*)-*O-*desmethyl-Tramadol + (*S*)-Naproxen 1:1 in methyl isobutyl ketone). After 5 min, it turned cloudy and a dense precipitate was formed. This mixture was stirred at room temperature during 2 hours and the dense precipitate was transformed into a white solid which was filtered with a sintered funnel (porosity 4), washed with toluene (0.3 ml) and dried under vacuum at room temperature to give form B (-)-*O*-desmethyl-Tramadol-(*S*)-Naproxen (1:1) (74 mg, 74 % yield).

### Example 12c: Form B (-)-O-desmethyl-Tramadol-(S)-Naproxen (1:1) salt

To an assay tube equipped with magnetic stirrer containing (-)-*O*-desmethyl-Tramadol (52 mg, 0.21 mmol) and (*S*)-Naproxen (48 mg, 0.21 mmol, 1 eq.) was added AcOiBu (isobutyl acetate) (1 ml). The resultant solution was seeded with a mixture of form obtained in example 12a and form B (-)-*O*-desmethyl-Tramadol-(*S*)-Naproxen (1:1) salt (obtained from crystallization (*rac*)-*O-*desmethyl-Tramadol + (*S*)-Naproxen 1:1 in methyl isobutyl ketone) and stirred at room temperature during 2 hours (after 5 minutes, a white solid precipitated). Then, the precipitate was filtered with a sintered funnel (porosity 4), washed with AcOiBu (isobutyl acetate) (0.2 ml) and dried under vacuum at room temperature to give form B (-)-*O*-desmethyl-Tramadol- (*S*)-Naproxen (1:1) (74 mg, 74 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in CDCl₃ at 400 MHz shows peaks identical to those of form A (see example 11).

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3344.3 (m, br), 2935.3 (m), 2861.6 (w), 1631.1 (m), 1604.9 (s), 1571.8 (s), 1481.1 (m), 1448.3 (s), 1381.6 (s), 1358.0 (s), 1266.4 (s), 1239.8 (s), 1210.8 (s), 1170.8 (m), 1030.1 (s), 926.7 (m), 865.7 (s), 788.7 (m), 705.4 (m), 478.5 (m) cm⁻¹.

### DSC

DSC analysis was recorded with a Mettler DSC822^{e}. A sample of 1.9560 mg was weighed into a 40 µl aluminium crucible with a pinhole lid and was heated, under nitrogen (50 ml/min), at 10 °C/min from 30 to 200 °C.

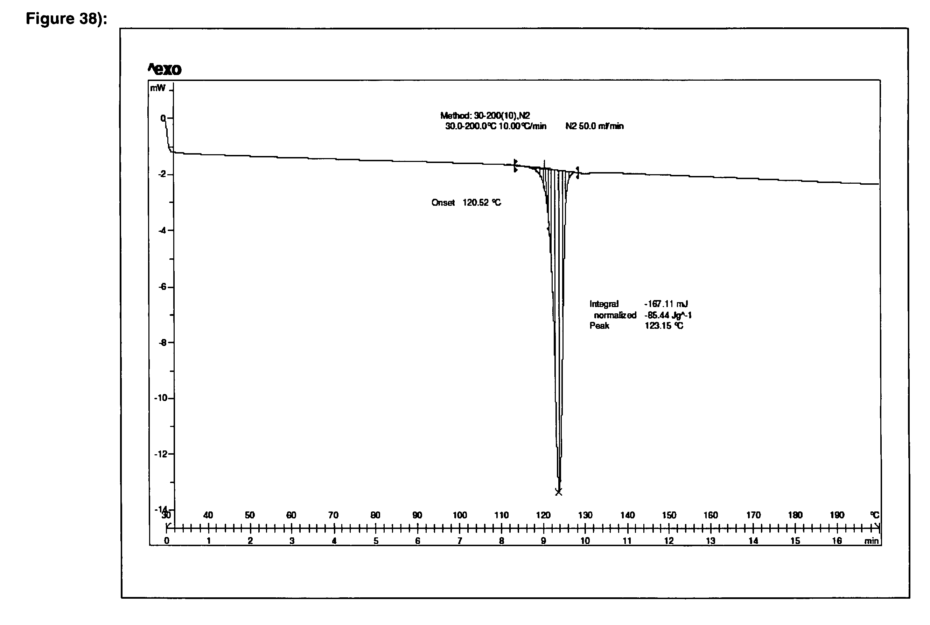

The novel type of crystal of the present invention form B (-)-*O*-desmethyl-Tramadol-(*S*)-Naproxen (1:1) is **characterized in that** the endothermic sharp peak corresponding to the melting point has an onset at 120.52 °C (fusion enthalpy -85.44 J/g), measured by DSC analysis (10 °C/min), see figure 38.

### TG

Thermogravimetric analysis was recorded with a thermogravimetric analyzer Mettler TGA/SDTA851^{e}_{.} A sample of 3.9462 mg was weighed into a 70 µl aluminium crucible with a pinhole lid, and was heated at 10 °C/min from 30 to 200 °C, under nitrogen (50 ml/min).

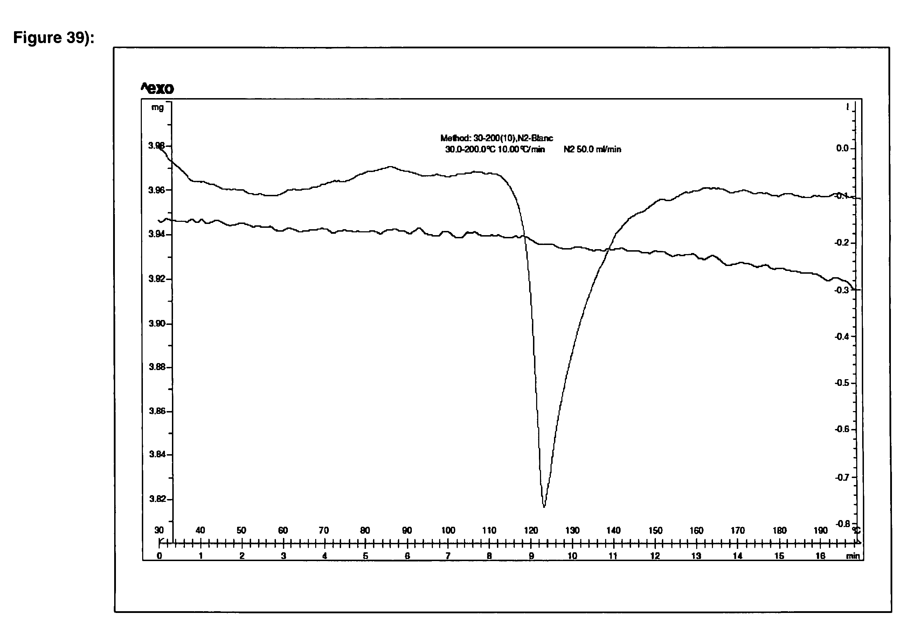

The TG analysis of crystalline form B according to the invention shows no weight loss between 30 and 200 °C (see figure 39).

### XRPD

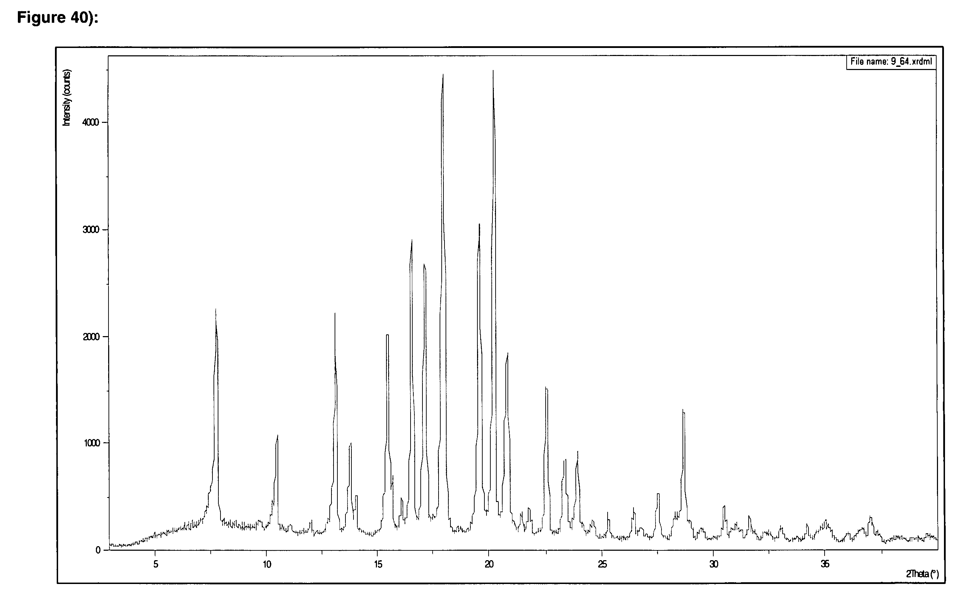

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 40).

### List of selected peaks:

| **2θ (°)¹** | **d (Å)** | **I (%)** |
|---|---|---|
| 7.83 | 11.29 | 43 |
| 9.73 | 9.09 | 2 |
| 10.52 | 8.41 | 18 |
| 11.10 | 7.97 | 1 |
| 12.01 | 7.37 | 2 |
| 13.15 | 6.73 | 45 |
| 13.79 | 6.42 | 18 |
| 14.07 | 6.30 | 8 |
| 15.49 | 5.72 | 41 |
| 15.66 | 5.66 | 12 |
| 16.09 | 5.51 | 7 |
| 16.58 | 5.35 | 60 |
| 17.16 | 5.17 | 57 |
| 18.02 | 4.92 | 83 |
| 19.60 | 4.53 | 65 |
| 20.24 | 4.39 | 100 |
| 20.84 | 4.26 | 39 |
| 21.43 | 4.15 | 5 |
| 21.81 | 4.08 | 6 |
| 22.58 | 3.94 | 32 |
| 23.42 | 3.80 | 15 |
| 23.96 | 3.71 | 17 |
| 24.64 | 3.61 | 3 |
| 25.30 | 3.52 | 5 |
| 26.45 | 3.37 | 6 |
| 26.81 | 3.33 | 2 |
| 27.54 | 3.24 | 10 |
| 28.29 | 3.16 | 5 |
| 28.68 | 3.11 | 28 |
| 29.48 | 3.03 | 2 |
| 30.51 | 2.93 | 7 |
| 31.02 | 2.88 | 3 |
| 31.61 | 2.83 | 4 |
| 32.54 | 2.75 | 1 |
| 32.97 | 2.72 | 3 |
| 34.21 | 2.62 | 3 |
| 35.07 | 2.56 | 4 |
| 36.03 | 2.49 | 1 |
| 36.68 | 2.45 | 2 |
| 37.02 | 2.43 | 5 |

| | | |
|---|---|---|
| ¹The 2θ values were obtained using copper radiation (Cu_{Kα}). | | |

### Single crystal X-ray diffraction

The crystal structure of form B (-)-*O*-desmethyl-Tramadol-(*S*)-Naproxen (1:1) salt has been determined from single crystal X-ray diffraction data. The colorless prism used (0.15 × 0.12 × 0.09 mm) was obtained from the cold evaporation of an isobutyl acetate solution of equimolar amounts of (-)-*O*-desmethyl-Tramadol and (*S*)-Naproxen.
Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters, except the disordered carbon atom of the methoxy group.

### Relevant structural data:

| Crystal system | Monoclinic |
|---|---|
| Space group | *P*2₁ |
| a (Å) | 9.2616(16) |
| b (Å) | 12.599(2) |
| c (Å) | 11.563(2) |
| β (°) | 102.456(4) |
| Volume (Å³) | 1317.5(4) |
| Z | 2 |
| D calc. (Mg/m³) | 1.209 |
| N. of refl. | 5399 |
| Refl. with I > 2σ(1) | 1964 |
| R (I > 2□(I)) | 0.0835 |

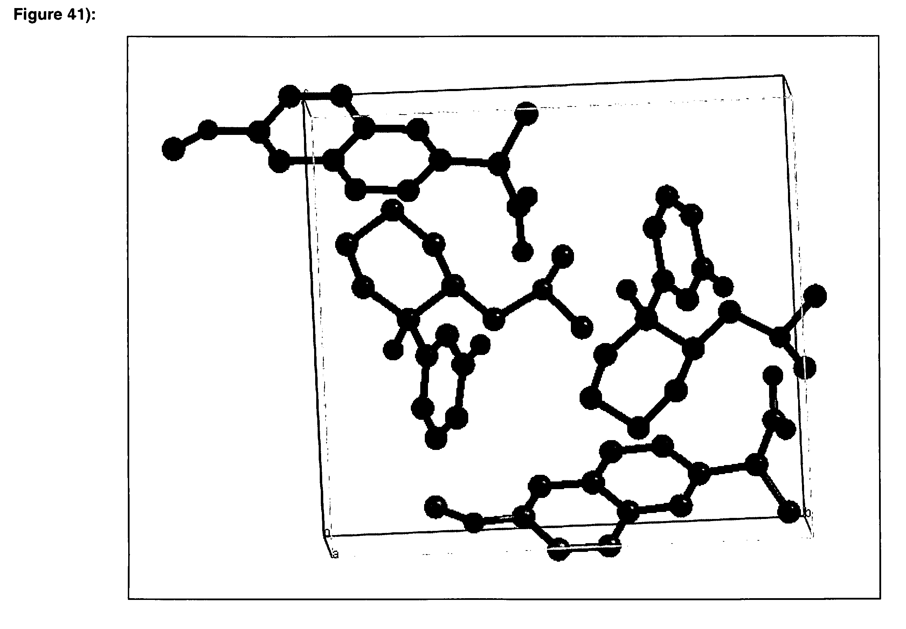

The crystal structure of form B is depicted in figure 41.
Simulation of the XRPD diffractogram from single crystal data gives an almost identical diagram to the experimental one presented above.

### Example 13a: (rac)-O-desmethyl-Tramadol-(S)-Naproxen (1:1) salt

To an assay tube equipped with a magnetic stirrer containing (*rac*)-*O*-desmethyl-Tramadol (52 mg, 0.21 mmol) and (*S*)-Naproxen (48 mg, 0.21 mmol, 1 eq.), was added CHCl₃ (chloroform) (1 ml). The mixture was heated until reach complete dissolution. The resultant solution was slowly cooled to room temperature and, then, stirred at room temperature and partially evaporated over 6 days. The precipitate was filtered with a sintered funnel (porosity 4), washed with CHCl₃ (chloroform) (0.2 ml) and dried under vacuum at room temperature to give salt form (*rac*)-*O-*desmethyl-Tramadol-(*S*)-Naproxen (1:1) as a white solid (65 mg, 65 % yield).

### Example 13b: (rac)-O-desmethyl-Tramadol-(S)-Naproxen (1:1) salt

To an assay tube equipped with a magnetic stirrer containing (*rac*)-*O*-desmethyl-Tramadol (52 mg, 0.21 mmol) and (*S*)-Naproxen (48 mg, 0.21 mmol, 1 eq.) was added CHCl₃ (chloroform) (0.5 ml). The mixture was heated until reach complete dissolution. The resultant solution was slowly cooled to room temperature, seeded with form obtained in example 13a and stirred for 3h at room temperature. The precipitate was filtered with a sintered funnel (porosity 4), washed with CHCl₃ (chloroform) (0.2 ml) and dried under vacuum at room temperature to give salt form *(rac)-O*-desmethyl-Tramadol-(*S*)-Naproxen (1:1), as a white solid (66 mg, 66 % yield).

### ¹H NMR

Proton nuclear magnetic resonance analysis was recorded in deuterated dimethyl sulfoxide (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR ¹H NMR spectrum in d6-DMSO at 400 MHz shows peaks at 9.13 (s br, 1H); 7.80-7.75 (m, 2H); 7.71 (s, 1H); 7.40 (dd, *J =* 8.6 Hz, *J* = 2.2 Hz, 1H); 7.28 (d, *J* = 2.4 Hz, 1H); 7.14 (dd, *J* =

9.0 Hz, *J* = 3.0 Hz, 1H); 7.06 (t, *J* = 8.0 Hz, 1H); 6.89 (s, 1H); 6.82 (d, *J* = 8.0 Hz, 1H); 6.54 (dd, *J* = 8.0 Hz, *J* = 1.6 Hz, 1H); 3.86 (s, 3H); 3.79 (q, *J* = 7.2 Hz, 1H); 2.12 (dd, *J* = 12.2 Hz and 9.4 Hz, 1H); 1.92 (s, 6H); 1.79-1.22 (m, 13H) ppm.

### IR

The FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3221.5 (m, br), 2938.9 (m), 2855.7 (m), 1632.0 (m), 1604.0 (s), 1576.3 (s), 1503.6 (m), 1482.0 (m), 1448.8 (s), 1390.0 (s), 1359.9 (s), 1268.1 (s), 1230.0 (s), 1216.2 (s), 1170.8 (m), 1031.3 (m), 1002.0 (m), 926.5 (m), 864.4 (m) 851.6 (m), 791.0 (m), 706.2 (m), 476.4 (m) cm⁻¹.

### DSC

DSC analysis was recorded with a Mettler DSC822^{e}. A sample of 1.6440 mg was weighed into a 40 µl aluminium crucible with a pinhole lid, and was heated, under nitrogen (50 ml/min), at 10 °C/min from 30 to 200 °C.

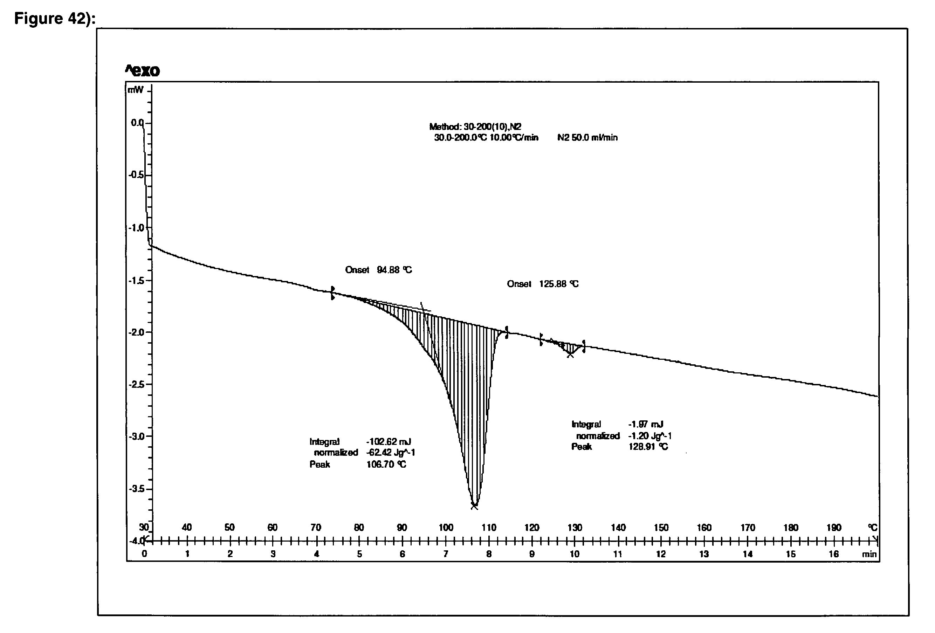

The novel type of crystal of the present invention is **characterized in that** the endothermic sharp peak corresponding to the melting point has an onset at 94.88 °C (fusion enthalpy -62.42 J/g), measured by DSC analysis (10 °C/min), see figure 42.

### TG

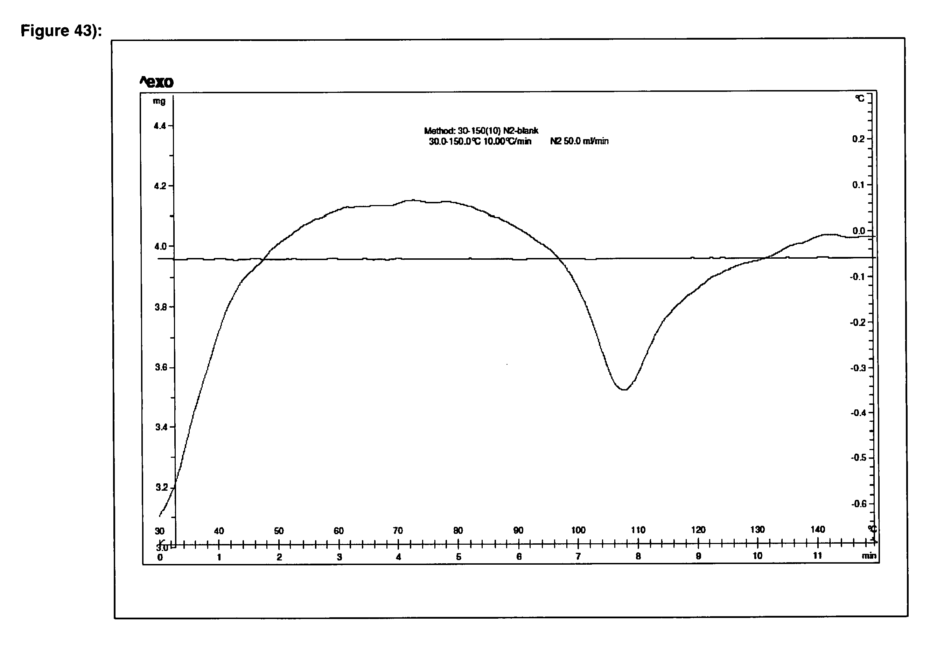

Thermogravimetric analysis was recorded with a thermogravimetric analyzer Mettler TGA/SDTA851^{e}. A sample of 3.9573 mg was weighed into a 70 µl aluminium crucible with a pinhole lid, and was heated at 10 °C/min from 30 to 150 °C, under nitrogen (50 ml/min).
The TG analysis of the crystalline form according to the invention shows no weight loss between 30 and 150 °C (see figure 43).

### XRPD

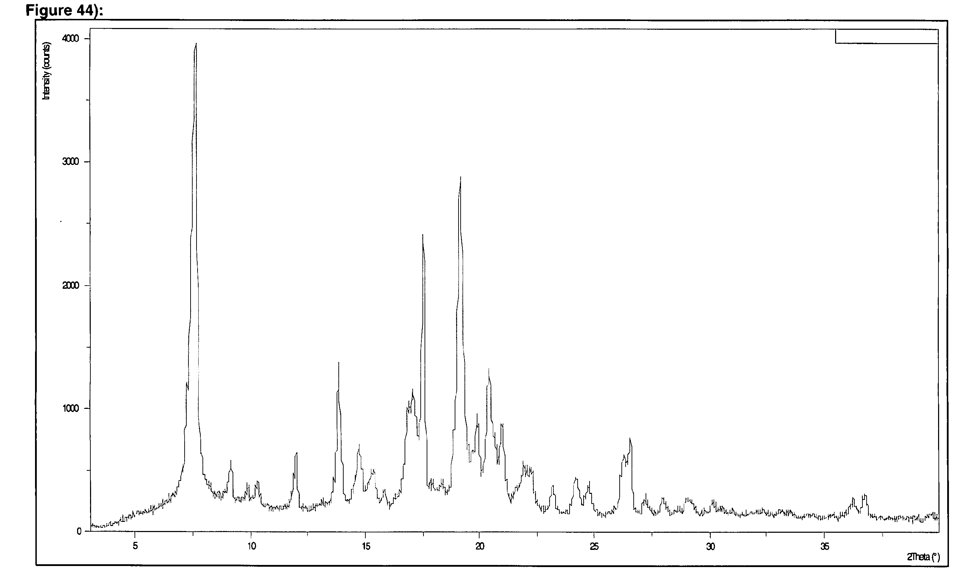

XRPD analysis was performed using a Philips X'Pert diffractometer with Cu 1% radiation in Bragg-Brentano geometry. The system is equipped with a monodimensional, real time multiple strip detector. The measurement parameters were as follows: the range of 2θ was 3° to 40° at a scan rate of 8.8° per minute (see figure 44).

### List of selected peaks:

| **2**θ **(°)¹** | **d (Å)** | **I (%)** |
|---|---|---|
| 7.69 | 11.50 | 100 |
| 9.15 | 9.67 | 12 |
| 9.91 | 8.93 | 6 |
| 10.34 | 8.55 | 7 |
| 11.98 | 7.39 | 15 |
| 13.89 | 6.38 | 37 |
| 14.72 | 6.02 | 16 |
| 15.38 | 5.76 | 11 |
| 15.84 | 5.59 | 5 |
| 16.92 | 5.14 | 28 |
| 17.10 | 5.19 | 32 |
| 17.56 | 5.05 | 74 |
| 18.36 | 4.83 | 7 |
| 19.17 | 4.63 | 89 |
| 19.91 | 4.46 | 26 |
| 10.41 | 4.35 | 38 |
| 20.96 | 4.24 | 22 |
| 21.92 | 4.05 | 12 |
| 22.29 | 3.99 | 11 |
| 23.14 | 3.84 | 7 |
| 24.17 | 3.68 | 10 |
| 24.75 | 3.60 | 8 |
| 26.26 | 3.39 | 16 |
| 26.56 | 3.36 | 20 |
| 27.20 | 3.28 | 5 |
| 27.95 | 3.19 | 5 |
| 29.02 | 3.08 | 4 |
| 30.17 | 2.96 | 4 |
| 30.59 | 2.92 | 2 |
| 33.33 | 2.69 | 1 |
| 36.27 | 2.48 | 5 |
| 36.78 | 2.44 | 6 |

| | | |
|---|---|---|
| ¹The 2θ values were obtained using copper radiation (Cu_{Kα}). | | |

### Example 14a: (+)-O-desmethyl-Tramadol-(R)-Flurbiprofen (1:1) salt

To a vial containing (R)-Flurbiprofen (50 mg, 0.21 mmol) and (+)-*O*-desmethyl-Tramadol (51 mg, 0.21 mmol, 1.0 eq), was added 4 ml ethanol. The mixture was heated to ensure complete dissolution. The resultant solution was cooled to room temperature and the solvent was evaporated slowly at room temperature. After complete evaporation, salt form (+)-*O*-desmethyl-Tramadol-(*R*)-Flurbiprofen (1:1) was obtained as colourless needles (101 mg, quantitative yield).

### Example 14b: (+)-O-desmethyl-Tramadol-(R)-Flurbiprofen (1:1) salt

To a vial equipped with magnetic stirrer containing (R)-Flurbiprofen (99 mg, 0.41 mmol), was added at room temperature toluene (3.5 ml). (+)-*O*-desmethyl-Tramadol (101 mg, 0.41 mmol, 1.0 eq) was added to the resultant solution before stirring the suspension overnight at room temperature. The white suspension was filtered with a sintered funnel (porosity 4) and washed with toluene (1 ml). After drying under vacuum at room temperature, salt form (+)-*O*-desmethyl-Tramadol-(R)-Flurbiprofen 1:1 was obtained as a white powder (177 mg, 88 % yield).

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

The ¹H NMR spectrum in CDCl₃ at 400 MHz has peaks at 7.48-7.12 (m, 12H); 6.86 (d, *J* = 11.2 Hz, 1H); 6.73 (dd, *J* = 8.0 Hz, *J* = 2.4 Hz, 1H); 5.08 (s, br); 3.74 (q, *J* = 7.2 Hz, 1H); 2.69 (dd, *J* = 13.6 Hz, *J* = 6.0 Hz, 1H); 2.28 (s, 6H); 1.06-1.85 (m, H); 1.54 (d, 6.8 Hz); 1.34- 1.23 (m, 1H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The Infra Red spectrum of the sample (as KBr pellet) has absorption bands at 3395.6 (br); 2931.0 (m); 2853.7 (w); 1571.0 (s); 1482.5 (s); 1448.2 (s); 1415.3 (m); 1387.7 (s); 1358.1 (s); 1318.9 (m); 1279.0 (s); 1238.4 (m); 1208.2 (m); 1171.9 (m); 1131.8 (m), 1109.0 (w); 1093.0 (w); 999.5 (w); 964.5 (m); 861.7 (m); 703.9 (m) cm⁻¹.

### DSC

DSC analysis was recorded with a Mettler DSC822^{e}. A sample of 0.7220 mg was weighed into 40 µl aluminium crucible with a pinhole lid and was heated, under nitrogen (50 ml/min), at 10 °C/min from 30 to 200 °C.

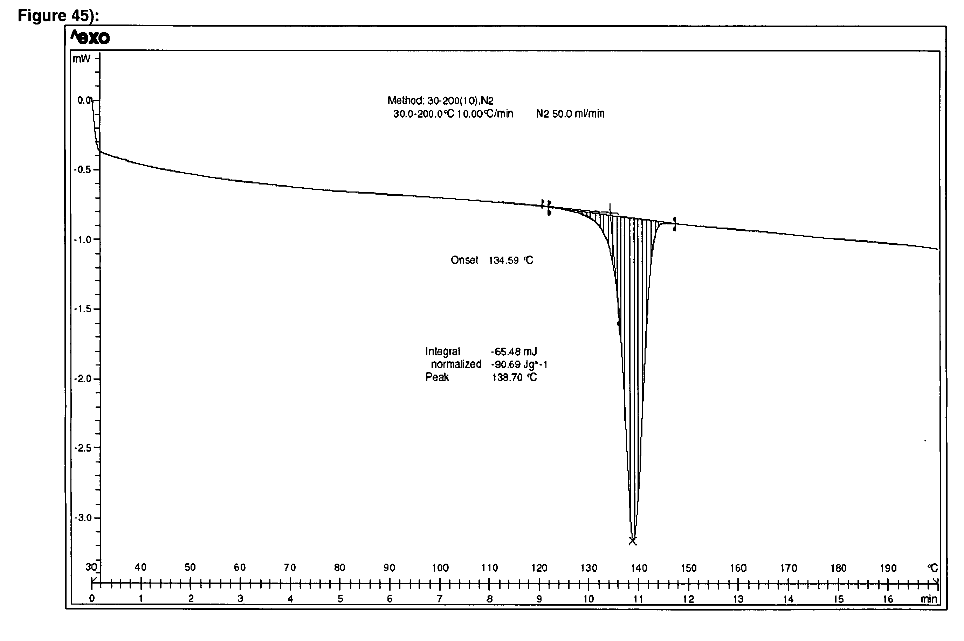

The novel type of crystal of the present invention is **characterized in that** the endothermic sharp peak corresponding to the melting point has an onset at 134.59 °C (fusion enthalpy -90.7 J/g), measured by DSC analysis (10 °C/min) (see figure 45).

### TG

Thermogravimetric analysis was recorded in a thermogravimetric analyzer Mettler TGA/SDTA851^{e}. A sample of 5.2306mg was weighed into a 70 µl aluminium crucible with a pinhole lid and was heated at 10 °C/min from 30 to 300 °C, under nitrogen (50 ml/min).

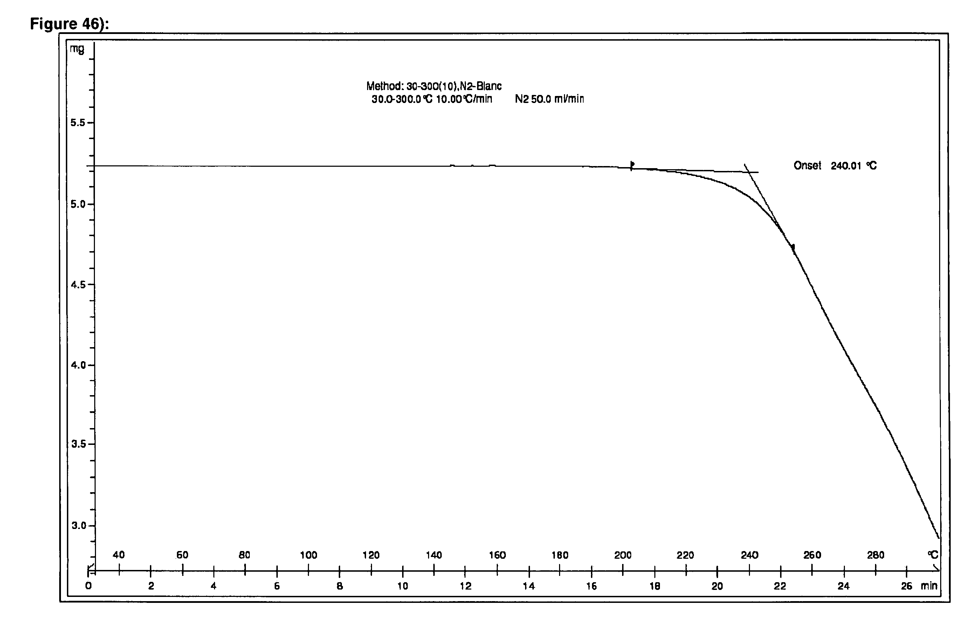

The TG analysis of the crystalline form according to the invention shows constant mass followed by a decrease in mass with onset at 240.0 °C (see figure 46).

### XRPD

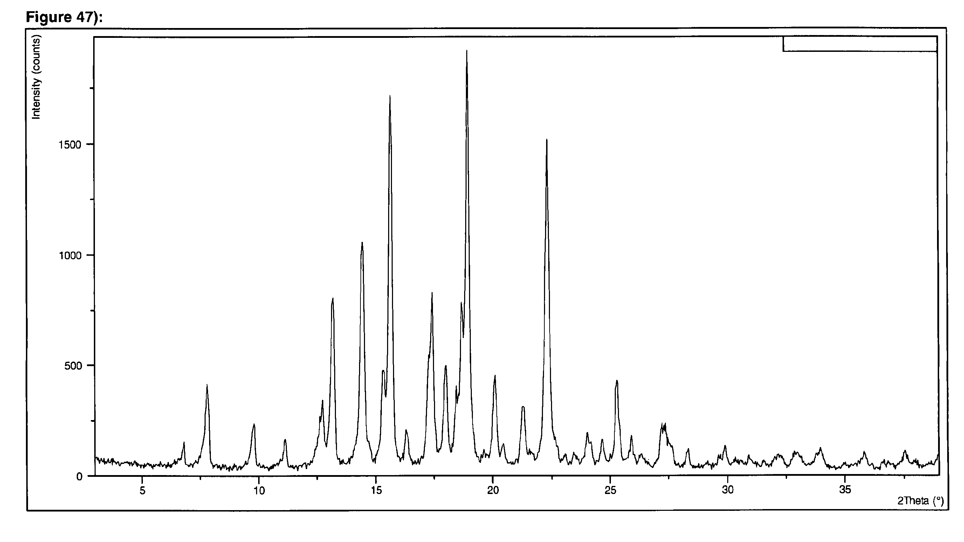

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute (see figure 47).

### List of selected peaks:

| **2**θ **(°)¹** | **d (Å)** | **I (%)** |
|---|---|---|
| 6.80 | 13.00 | 5 |
| 7.83 | 11.29 | 19 |
| 9.79 | 9.03 | 11 |
| 11.14 | 7.94 | 7 |
| 12.74 | 6.95 | 16 |
| 13.19 | 6.71 | 41 |
| 14.46 | 6.13 | 55 |
| 15.32 | 5.78 | 24 |
| 15.67 | 5.66 | 90 |
| 16.33 | 5.43 | 8 |
| 17.44 | 5.08 | 43 |
| 18.00 | 4.93 | 25 |
| 18.67 | 4.75 | 40 |
| 18.96 | 4.68 | 100 |
| 20.10 | 4.42 | 20 |
| 20.43 | 4.35 | 6 |
| 21.29 | 4.17 | 15 |
| 22.33 | 3.98 | 80 |
| 24.03 | 3.70 | 9 |
| 24.67 | 3.61 | 7 |
| 25.29 | 3.52 | 20 |
| 25.91 | 3.44 | 8 |
| 27.16 | 3.28 | 9 |
| 28.29 | 3.16 | 4 |
| 29.88 | 2.99 | 5 |

| | | |
|---|---|---|
| ¹The 2θ values were obtained using copper radiation (Cu_{Kα}). | | |

### Single crystal X-ray diffraction

The crystal structure of the salt form (-)-*O*-desmethyl-Tramadol-(*R*)-Flurbiprofen (1:1) has been determined from single crystal X-ray diffraction data. The colourless prism used (0.41 × 0.30 × 0.17 mm) was obtained by crystallization from a seeded DMSO solution of equimolar amounts of (+)-*O*-desmethyl-Tramadol and (*R*)-Flurbiprofen.
Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters.

### Relevant structural data:

| Crystal system | Orthorhombic |
|---|---|
| Space group | *P*2₁2₁2₁ |
| a (Å) | 8.3463(5) |
| b (Å) | 12.5585(8) |
| c (Å) | 26.0291(17) |
| Volume (Å³) | 2728.3(3) |
| Z | 4 |
| D calc. (Mg/m³) | 1.202 |
| N. of refl. | 6722 |
| Refl. with I > 2σ(I) | 4030 |
| R (I > 2σ(I)) | 0.484 |

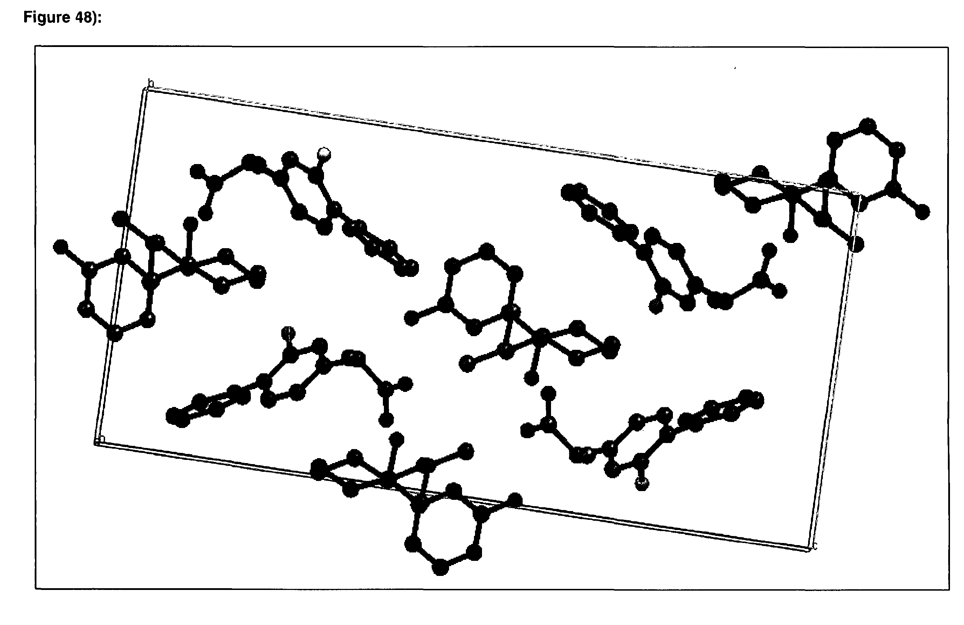

The crystal structure is depicted in figure 48 (fluorine atom is disordered over the two chemically equivalent sites and only the one with higher occupation is shown).
Simulation of the XRPD diffractogram from single crystal data gives an almost identical diagram to the experimental one presented above.

### Example 15a: (-)-O-desmethyl-Tramadol-(R)-Flurbiprofen (1:1) salt

To a vial equipped with a magnetic stirrer containing (*R*)-Flurbiprofen (56 mg, 0.23 mmol) diluted with 1.5 ml acetone, was added (-)-*O*-desmethyl-Tramadol (57 mg, 0.23 mmol, 1 eq). Then, the resultant suspension was stirred at room temperature overnight. The suspension was filtered with a sintered funnel (porosity 4) and washed with acetone (0.5 ml). After drying under vacuum at room temperature, salt form (-)-*O*-desmethyl-Tramadol -(*R*)-Flurbiprofen (1:1) was obtained as a white powder (109 mg, 96 % yield).

### Example 15b: (-)-O-desmethyl-Tramadol-(R)-Flurbiprofen (1:1) salt

To a vial equipped with a magnetic stirrer containing (R)-Flurbiprofen (49 mg, 0.20 mmol) and (-)-*O*-desmethyl-Tramadol (50 mg, 0.20 mmol, 1 eq.), was added water (3 ml). The resultant mixture was heated at 72 °C and ethanol was added dropwise until reach complete dissolution (Vₑₜₕₐₙₒₗ=1.1ml). Then, the mixture was cooled slowly to room temperature and stirred 24 h. The white solid was filtered with a sintered funnel (porosity 3). After drying under vacuum at room temperature salt form (-)-*O*-desmethyl-Tramadol-(*R*)-Flurbiprofen (1:1) (61 mg, 62 % yield) was obtained as lamina crystals.

### ¹H NMR

Proton nuclear magnetic resonance analysis was recorded in deuterated chloroform (CDCl₃) in a Varian Mercury 400 spectrometer, equipped with a broadband probe ATB 1H/19F/X of 5 mm. Spectrum was acquired dissolving 5-10 mg of sample in 0.6 ml of deuterated solvent.

The ¹H NMR spectrum in CDCl₃ at 400 MHz has peaks at 7.53-7.31(m, 12H); 7.23-7.15 (m, 12H); 7.14 (s, br); 6.90 (d, *J* = 7.6 Hz, 1H); 6.73(dd, *J* = 8.0, 2.4 Hz, 1H); 3.74 (q, *J* = 7.2 Hz, 1H); 2.63 (dd, *J* = 13.6, *J =* 6.0 Hz, 1H); 2.28 (s, 6H); 2.06-1.85 (m, H); 1.54 (d, 6.8 Hz); 1.34-1.23 (m, 1H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The Infra Red spectrum of the sample (as KBr pellet) has absorption bands at 3415.3 (m br); 2972.3 (w), 2932.5 (m), 2853.0 (w), 1570.3 (s); 1482.1 (s); 1451.2 (s); 1415.1 (s), 1389.5 (s), 1358.8 (s), 1274.3 (s), 1242.4 (m), 1207.1 (m), 1772.0 (m), 1131.6 (m), 1109.5 (m), 1000.8 (m), 927.7 (m), 868.2 (m), 790.3 (m), 770.0 (m), 731.8 (m), 705.4 (m) cm⁻¹.

### DSC

DSC analysis was recorded with a Mettler DSC822^{e}. A sample of 0.6790mg was weighed into 40 µl aluminium crucible with a pinhole lid and was heated, under nitrogen (50 ml/min), at 10 °C/min from 30 to 300 °C.

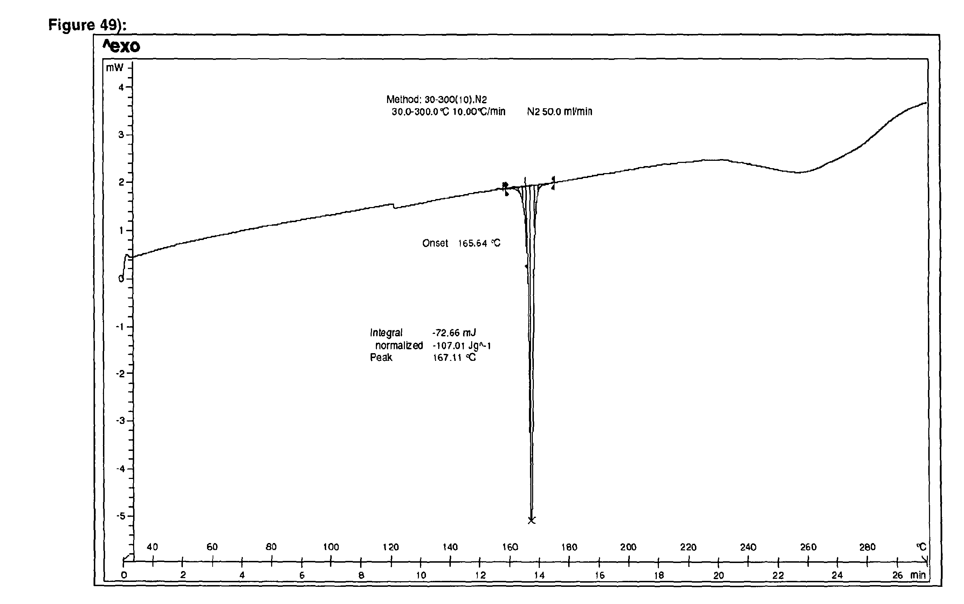

The novel type of crystal of the present invention is **characterized in that** the endothermic sharp peak corresponding to the melting point has an onset at 165.64 °C (fusion enthalpy -107.01 J/g), measured by DSC analysis (10 °C/min) (see figure 49).

### TG

Thermogravimetric analysis was recorded in a thermogravimetric analyzer Mettler TGA/SDTA851^{e}. A sample of 3.3980 mg was weighed into a 70 µl aluminium crucible with a pinhole lid and was heated at 10 °C/min from 30 to 300 °C, under nitrogen (50 ml/min).

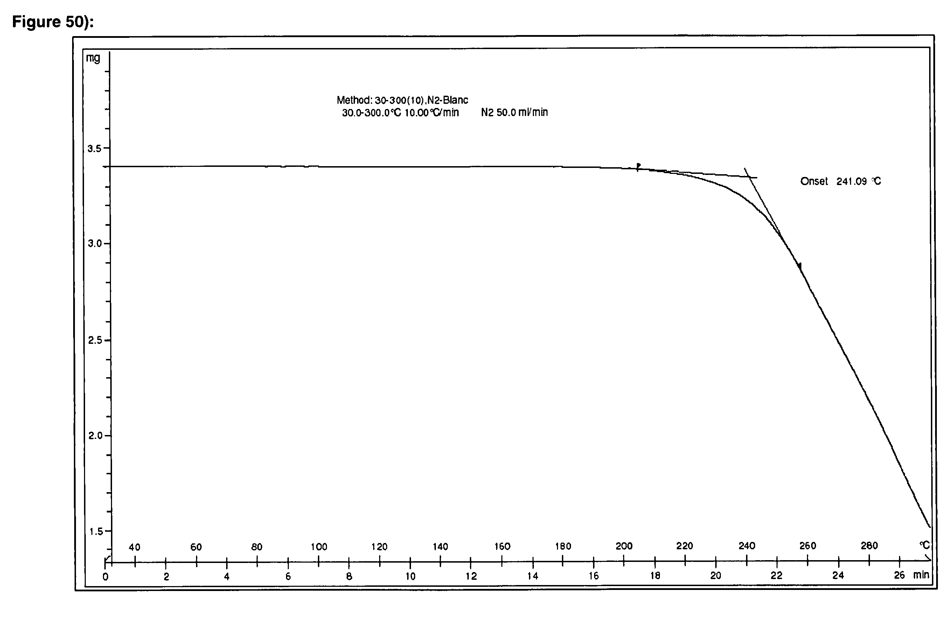

The TG analysis of the crystalline form according to the invention shows constant mass followed by a decrease in mass with onset at 241.1 °C (see figure 50).

### XRPD

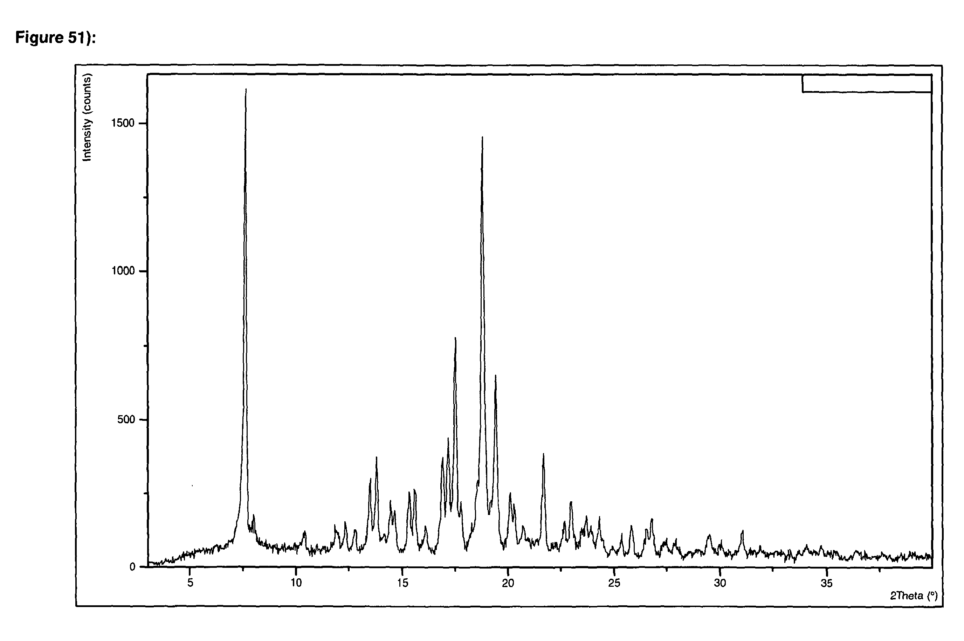

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 51).

### List of selected peaks:

| **2**θ **(°)¹** | **d (Å)** | **I (%)** |
|---|---|---|
| 7.66 | 11.54 | 100 |
| 8.03 | 11.02 | 7 |
| 10.41 | 8.49 | 4 |
| 11.93 | 7.42 | 4 |
| 12.35 | 7.17 | 6 |
| 12.81 | 6.91 | 5 |
| 13.51 | 6.55 | 16 |
| 13.83 | 6.40 | 21 |
| 14.46 | 6.13 | 11 |
| 14.66 | 6.04 | 9 |
| 15.36 | 5.77 | 13 |
| 15.62 | 5.67 | 13 |
| 16.12 | 5.50 | 6 |
| 16.94 | 5.24 | 21 |
| 17.18 | 5.16 | 25 |
| 17.53 | 5.06 | 46 |
| 17.79 | 4.99 | 11 |
| 18.82 | 4.72 | 93 |
| 19.43 | 4.57 | 39 |
| 20.10 | 4.42 | 13 |
| 20.32 | 4.37 | 10 |
| 20.70 | 4.29 | 6 |
| 21.70 | 4.10 | 19 |
| 22.67 | 3.92 | 6 |
| 23.00 | 3.87 | 11 |
| 23.67 | 3.76 | 6 |
| 24.30 | 3.66 | 6 |
| 25.38 | 3.51 | 4 |
| 25.81 | 3.45 | 6 |
| 26.81 | 3.33 | 7 |
| 27.45 | 3.25 | 3 |
| 27.89 | 3.20 | 2 |
| 29.54 | 3.02 | 4 |
| 30.04 | 2.97 | 2 |
| 31.04 | 2.88 | 5 |
| 34.10 | 2.63 | 2 |
| 34.77 | 2.58 | 2 |
| 36.38 | 2.47 | 1 |

| | | |
|---|---|---|
| ¹The 2θ values were obtained using copper radiation (Cu_{κα}). | | |

### Example 16a: (rac)-O-desmethyl-Tramadol-(rac)-Flurbiprofen (2:3) co-crystal

To a vial equipped with magnetic stirrer containing (*rac*)-*O*-desmethyl-Tramadol (50 mg, 0.20 mmol), was added Et₂O (Diethyl ether) (0.3 ml). To the resultant suspension was added *(rac)-*Flurbiprofen (49 mg, 0.20 mmol, 1 eq.) diluted with Et₂O (Diethyl ether) (0.7 ml). The suspension was stirred overnight at room temperature.
The solid was filtered with a sintered funnel (porosity n°3) and washed with Et₂O (Diethyl ether) (0.5 ml). After drying under vacuum at room temperature, crystal form (*rac*)-*O*-desmethyl-Tramadol-(*rac*)-Flurbiprofen (2:3) was obtained as a white solid (39 mg, 47% yield).

### Example 16b: (rac)-O-desmethyl-Tramadol-(rac)-Flurbiprofen (2:3) co-crystal

To a vial equipped with magnetic stirrer containing (*rac*)-*O*-desmethyl-Tramadol (20 mg, 0.08 mmol), was added (*rac*)-Flurbiprofen (58 mg, 0.24 mmol, 3 eq.) diluted with AcO*i*Bu (butyl acetate) (1 ml). The suspension was seeded with form obtained in example 16a and, then, stirred 6 h at room temperature.
The solid was filtered with a sintered funnel (porosity n°4) and washed with AcO*i*Bu (butyl acetate) (0.5 ml). After drying under vacuum at room temperature, co-crystal form (*rac*)-*O-*desmethyl-Tramadol-(*rac*)-Flurbiprofen (2:3) was obtained as a white solid (32 mg, 65% yield).

### ¹H NMR

Proton nuclear magnetic resonance analyses were recorded in deuterated chloroform (CDCl₃) in a Varian Mercury 400 spectrometer, equipped with a broadband probe ATB 1H/19F/X of 5 mm. Spectra were acquired dissolving 5-10 mg of sample in 0.6 ml of deuterated solvent.

¹H NMR spectrum in CDCl₃ at 400 MHz shows peaks at 7.59-7.49 (m, 1.5×2H); 7.46-7.31 (m, 1.5×4H); 7.23-7.15 (m, 1.5×2H); 7.10-7.06 (m, 1H); 6.93 (d, *J* = 7.8 Hz, 1H); 6.74 (dd, *J=* 2Hz, *J* = 7.8 Hz, 1H); 3.77 (q, *J =* 7.2 Hz, 1.5×1H); 2.67-2.56 (dd, *J =* 7.0 Hz, *J =* 13.4 Hz, 1H); 2.40 (d, *J=* 13.4 Hz, 1H); 2.32 (s, 6H); 2.13-2.00 (m, 1H); 1.88-1.57 (m, 7H); 1.54 (d, *J=* 7.2 Hz, 1.5×3H); 1.35-1.19 (m, 1H).

### IR

The FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3240.4 (m br), 2976.0 (w), 2941.0 (m), 2856.5 (w), 1723.2 (m), 1575.6 (m), 1482.8 (s), 1455.8 (s), 1446.4 (s), 1388.7 (s), 1361.2 (s), 1279.0 (s), 1229.0 (s), 1132.3 (m), 925.2 (m), 765.8 (m), 696.8 (s) cm⁻¹.

### DSC

DSC analyses were recorded with a Mettler DSC822^{e}. A sample of 1.5900 mg was weighed into 40 µl aluminium crucible with a pinhole lid and was heated, under nitrogen (50 ml/min), at 10°C/min from 30 to 200 °C.

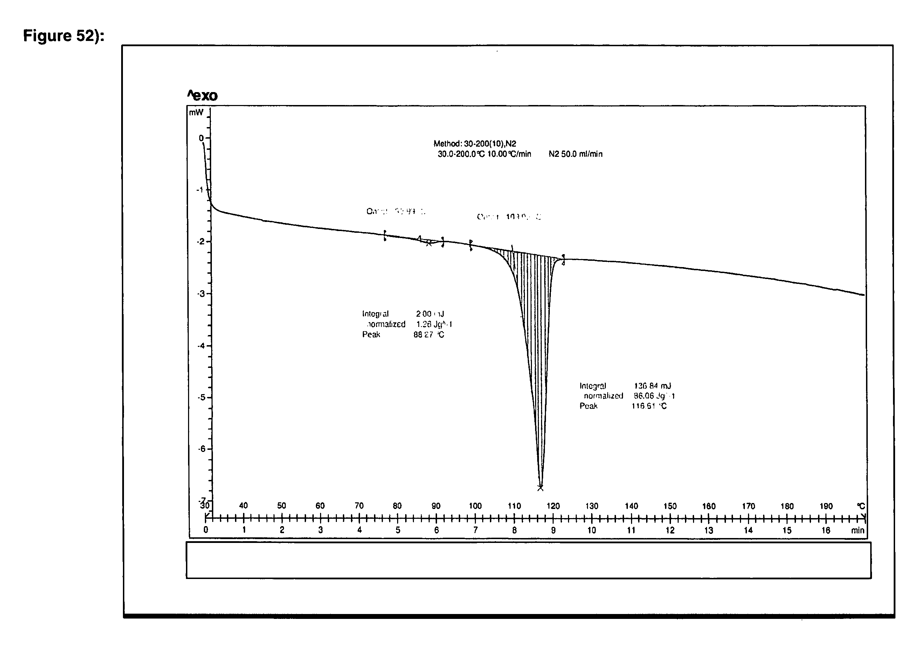

The novel type of co-crystal of the present invention is **characterized in that** the endothermic sharp peak corresponding to the melting point has an onset at 109.93 °C (fusion enthalpy -86.06 J/g), measured by DSC analysis (10 °C/min) (see figure 52).

### TG

Thermogravimetric analyses were recorded in a thermogravimetric analyzer Mettler TGA/SDTA851^{e}. A sample of 6.9629 mg was weighed into a 70 µl aluminium crucible with a pinhole lid and was heated at 10 °C/min from 30 to 300 °C, under nitrogen (50 ml/min).

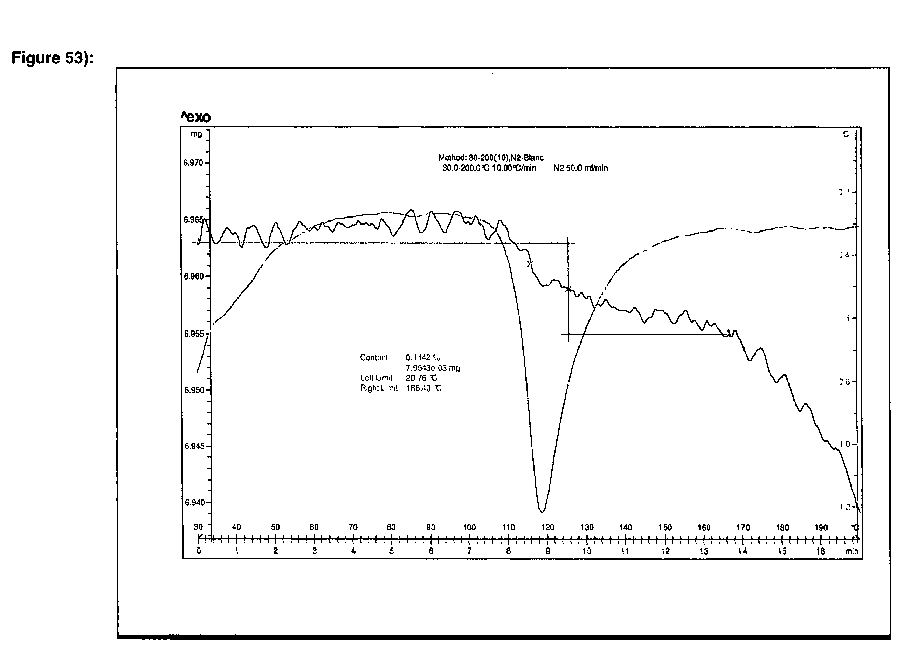

The TG analysis of this co-crystal form according to the invention shows no significant weight loss between 30 and 130 °C (see figure 53).

### XRPD

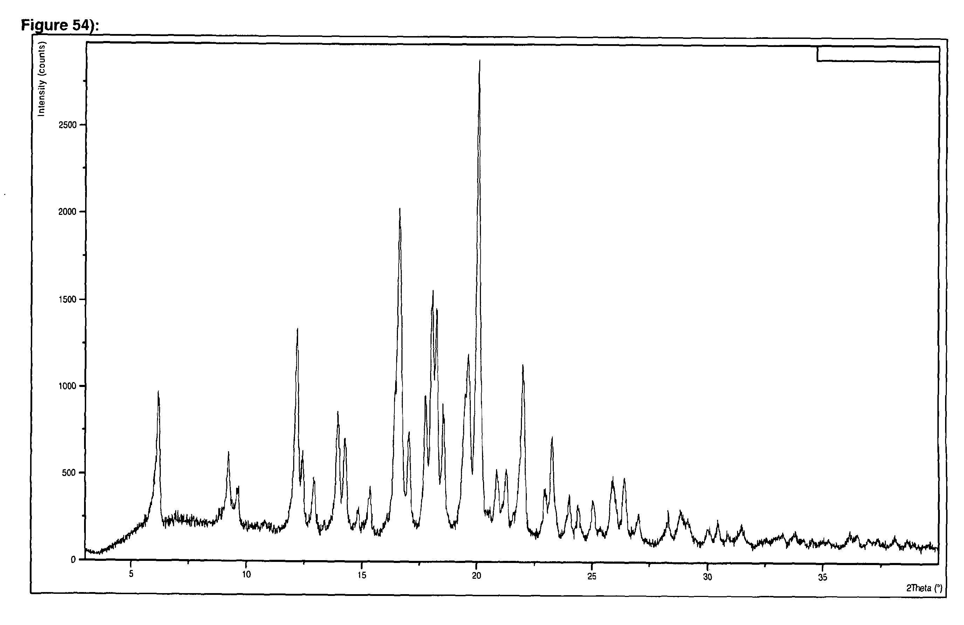

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 54).

### List of selected peaks:

| **2**θ **(°)¹** | **d (Å)** | **I(%)** |
|---|---|---|
| 6.23 | 14.18 | 28 |
| 9.24 | 9.57 | 19 |
| 9.63 | 9.18 | 11 |
| 12.22 | 7.24 | 41 |
| 12.46 | 7.11 | 17 |
| 12.94 | 6.84 | 13 |
| 13.99 | 6.33 | 26 |
| 14.29 | 6.20 | 22 |
| 14.85 | 5.97 | 7 |
| 15.36 | 5.77 | 11 |
| 16.42 | 5.40 | 29 |
| 16.63 | 5.33 | 71 |
| 17.07 | 5.19 | 22 |
| 17.79 | 4.99 | 31 |
| 18.07 | 4.91 | 52 |
| 18.27 | 4.86 | 49 |
| 18.58 | 4.78 | 26 |
| 19.44 | 4.57 | 27 |
| 19.63 | 4.52 | 40 |
| 20.07 | 4.42 | 100 |
| 20.86 | 4.26 | 16 |
| 21.28 | 4.18 | 16 |
| 22.00 | 4.04 | 38 |
| 22.94 | 3.88 | 11 |
| 23.29 | 3.82 | 21 |
| 24.03 | 3.70 | 10 |
| 24.40 | 3.65 | 8 |
| 25.05 | 3.56 | 9 |
| 25.86 | 3.45 | 14 |
| 26.41 | 3.37 | 14 |
| 27.01 | 3.30 | 6 |
| 28.29 | 3.15 | 6 |
| 28.81 | 3.10 | 7 |
| 29.19 | 3.06 | 5 |
| 30.04 | 2.97 | 3 |
| 30.46 | 2.93 | 5 |
| 31.49 | 2.84 | 4 |
| 33.26 | 2.69 | 3 |
| 33.83 | 2.65 | 3 |
| 36.13 | 2.49 | 3 |
| 36.48 | 2.46 | 2 |
| 38.11 | 2.36 | 2 |
| 38.72 | 2.33 | 1 |

| | | |
|---|---|---|
| ¹The 2θ values were obtained using copper radiation (Cu_{κα}) | | |

### Example 17a: (+)-O-desmethyl-Tramadol-(S)-Ibuprofen-H₂O (1:1:0.3) salt

To an assay tube equipped with a magnetic stirrer containing (+)-*O*-desmethyl-Tramadol (321 mg, 1.29 mmol) and (*S*)-Ibuprofen (265 mg, 1.29 mmol, 1 eq.) was added methyl isobutyl ketone (1.2 ml). The resultant mixture was heated until reach a slightly turbid solution. The solution was slowly cooled to room temperature and seeded with crystals of this form obtained previously by wet grinding of (+)-*O*-desmethyl-Tramadol-(*S*)-Ibuprofen (1:1) with toluene and no changes were observed. Then, the solution was cooled to 0-4°C with an ice-water bath and seeded again. After precipitation was observed, stirring was maintained during 30 min. The white solid was filtered with a sintered funnel (porosity 4), washed with cold methyl isobutyl ketone (0.4 ml) and dried under vacuum at room temperature to give salt form (+)-*O*-desmethyl-Tramadol-(*S*)-Ibuprofen-H₂O (1:1:0.3) as a white solid (367 mg, 62 % yield).

### Example 17b: (+)-O-desmethyl-Tramadol-(S)-Ibuprofen-H₂O (1:1:0.3) salt

To a vial containing (+)-*O*-desmethyl-Tramadol (106 mg, 0.43 mmol) and (*S*)-Ibuprofen (88 mg, 0.43 mmol) was added ethyl acetate (4 ml). The mixture was heated but some particles remained undissolved and were filtered with a Nylon filter (pore 0.45 µm) at room temperature.
The solution was seeded with form obtained in example 17a and evaporated slowly without stirring at room temperature under atmospheric pressure. After complete evaporation, salt form (+)-*O*-desmethyl-Tramadol-(*S*)-Ibuprofen-H₂O (1:1:0.25) was obtained as colourless needles (196 mg, quantitative yield).

### ¹H NMR

Proton nuclear magnetic resonance analyses were recorded in deuterated chloroform (CDCl₃) in a Varian Mercury 400 spectrometer, equipped with a broadband probe ATB 1H/ 9F/X of 5 mm. Spectra were acquired dissolving 5-10 mg of sample in 0.6 ml of deuterated solvent.

¹H NMR spectrum, in CDCl₃ at 400 MHz shows peaks at 7.25 (d, *J =* 7.4 Hz, 2H); 7.14 (t, *J* = 7.8 Hz, 2H); 7.03 (d, *J* = 8.2 Hz, 2H); 6.83 (d, *J* = 7.4 Hz, 1H); 6.72 (dd, *J =* 1.8Hz, *J* = 8.0 Hz, 1H); 3.65 (q, *J* = 7.2 Hz, 1H); 2.66 (dd, *J* = 7.4 Hz, *J=* 13.3 Hz, 1H); 2.41 (d, *J* = 7.0 Hz, 2H); 2.31 (d, *J=* 12.9 Hz, 1H); 2.23 (s, 6H); 1.99 (s br, 1H); 1.85-1.15 (m, 12H); 0.88 (d, *J=* 6.6 Hz, 6H).

### IR

The FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3403.9 (s br), 2955.0 (s), 2934.0 (s), 2866.3 (m), 1591.8 (s), 1479.5 (s), 1457.1 (s), 1388.9 (s), 1355.8 (s), 1278.5 (s), 1251.6 (s), 1136.3 (s), 1002.3 (s), 989.4 (s), 880.9 (m), 863.6 (s), 790.2 (s), 706.1 (s) cm⁻¹.

### DSC

DSC analyses were recorded with a Mettler DSC822^{e}. A sample of 1.4410 mg was weighed into 40 µl aluminium crucible with a pinhole lid and was heated, under nitrogen (50 ml/min), at 10 °C/min from 30 to 200 °C.

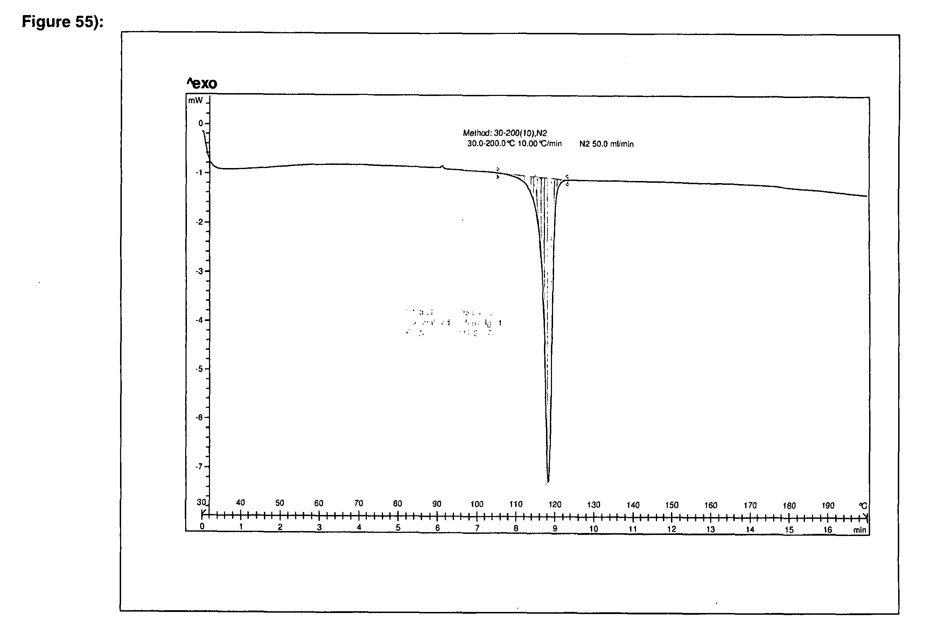

The novel type of crystal of the present invention is **characterized in that** the endothermic sharp peak corresponding to the melting point has an onset at 115.00 °C (fusion enthalpy -68.67 J/g), measured by DSC analysis (10 °C/min) (see figure 55).

### TG

Thermogravimetric analyses were recorded in a thermogravimetric analyzer Mettler TGA/SDTA851^{e}. A sample of 3.0167 mg was weighed into a 70 µl alumina crucible with a pinhole lid and was heated at 10 °C/min from 30 to 200 °C, under nitrogen (50 ml/min).

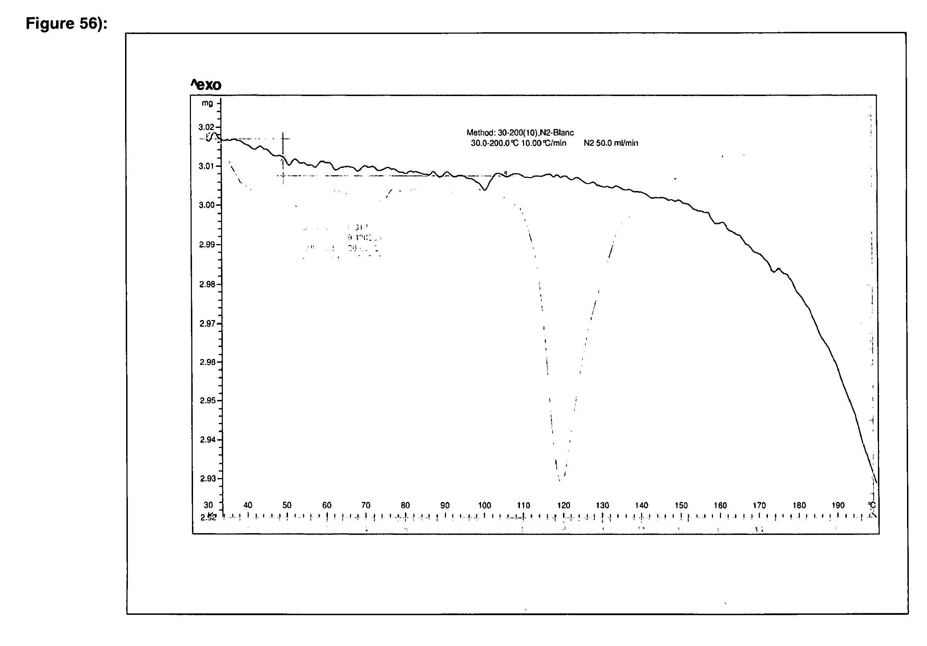

The TG analysis of this crystalline form according to the invention, obtained from example 17b, shows an approximated weight loss of 0.31 % between 30 °C and 106 °C) (this value would correspond to a 0.08 % of water loss) (see figure 56).

### Karl Fisher

Karl Fisher analysis was recorded with a Metrohm 787 KF Trinito. Analysis of 50 mg of sample from Example 17a was carried out using the following reactants: Hydranal-Composite 5 (Riedel de Haën Ref. 34805), Hydranal Methanol Rapid (Riedel de Haën Ref. 37817) and Hydranal Water Standard 10.0 (Riedel de Haën Ref. 34849 used to calculate the factor).

The KF analysis of the crystalline form according to the invention shows an average content of 1.1 % water (the results of the 3 analyses of the same sample fall between 1.04 and 1.10 %).

### XRPD

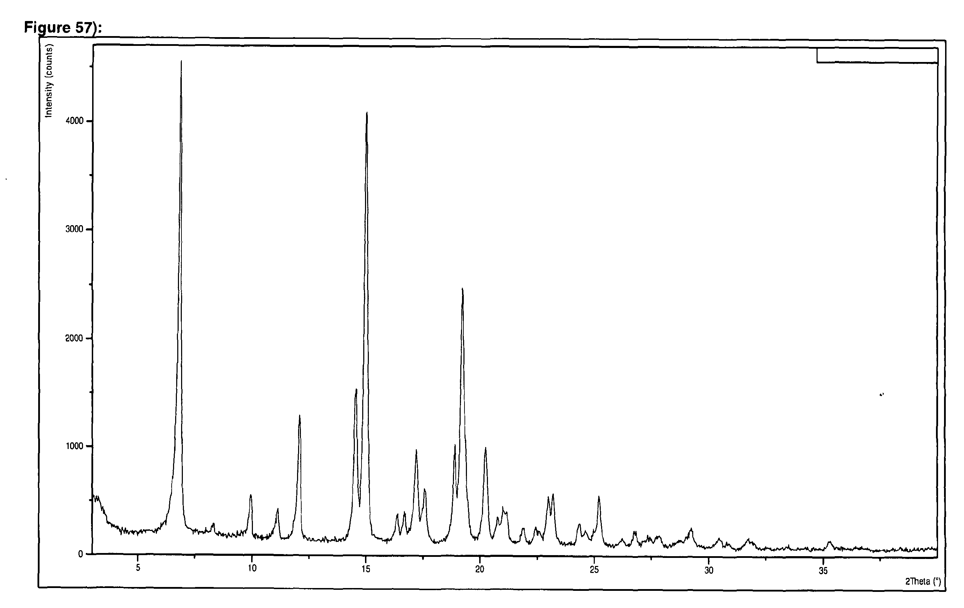

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 34.7° per minute (see figure 57).

### List of selected peaks:

| **2**θ **(°)¹** | **d (Å)** | **I (%)** |
|---|---|---|
| 6.88 | 12.85 | 100 |
| 8.30 | 10.66 | 2 |
| 9.96 | 8.89 | 9 |
| 11.15 | 7.93 | 6 |
| 12.09 | 7.32 | 26 |
| 14.55 | 6.09 | 34 |
| 15.01 | 5.90 | 92 |
| 16.39 | 5.41 | 6 |
| 16.69 | 5.31 | 6 |
| 17.21 | 5.15 | 19 |
| 17.59 | 5.04 | 12 |
| 18.88 | 4.70 | 21 |
| 19.22 | 4.62 | 56 |
| 20.25 | 4.39 | 21 |
| 21.21 | 4.19 | 6 |
| 21.89 | 4.06 | 3 |
| 22.42 | 3.97 | 3 |
| 22.97 | 3.87 | 10 |
| 23.21 | 3.83 | 11 |
| 24.36 | 3.65 | 5 |
| 25.22 | 3.53 | 10 |
| 26.21 | 3.40 | 1 |
| 26.81 | 3.33 | 3 |
| 27.82 | 3.21 | 2 |
| 29.25 | 3.05 | 4 |
| 30.47 | 2.93 | 2 |
| 31.69 | 2.82 | 2 |
| 35.30 | 2.54 | 2 |

| | | |
|---|---|---|
| ¹The 2θ values were obtained using copper radiation (Cu_{κα}) | | |

### Single crystal X-ray diffraction

The crystal structure of this form has been determined from single crystal X-ray diffraction data. The colourless plate used (0.35 × 0.21 × 0.04 mm) was obtained from the evaporation of a toluene solution, with a little amount of H₂O, of equimolar amounts of (+)-*O*-desmethyl-Tramadol and (*S*)-Ibuprofen.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters, except the partial-occupancy oxygen atom of the water molecule.

### Relevant structural data:

| Crystal system | Monoclinic |
|---|---|
| Space group | *P*2₁ |
| a (Å) | 10.7295(15) |
| b (Å) | 10.0318(15) |
| c (Å) | 12.9665(18) |
| β (°) | 98.776(3) |
| Volume (Å³) | 1379.3(3) |
| Z | 2 |
| D calc. (Mg/m³) | 1.106 |
| N. of refl. | 5559 |
| Refl. with I > 2σ(I) | 1852 |
| R (I > 2σ(I)) | 0.0811 |

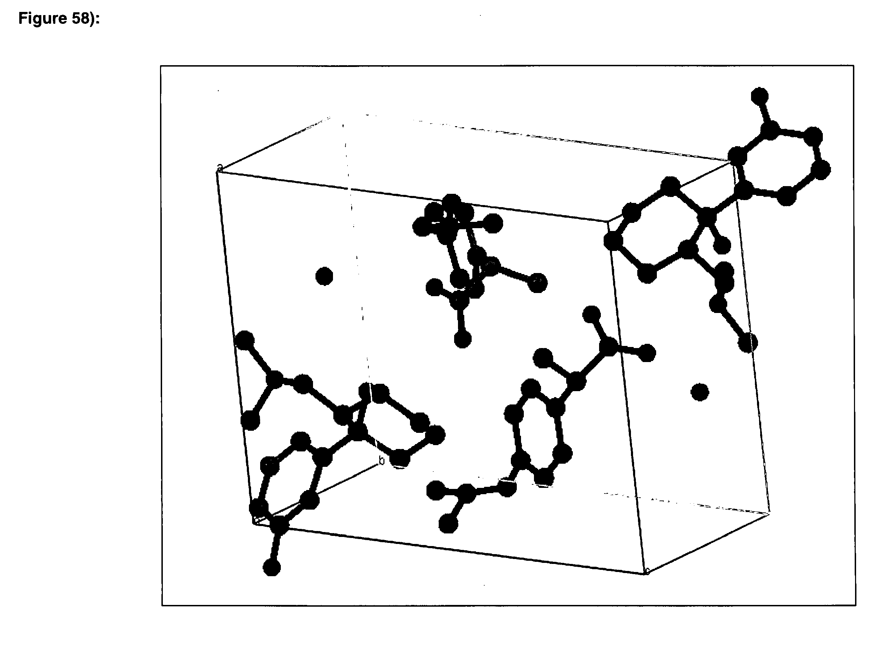

The crystal structure of this form is depicted in figure 58.
Simulation of the XRPD diffractogram from single crystal data gives an almost identical diagram to the experimental one presented above.

### Example 18a: (+)-O-desmethyl-Tramadol-(S)-Ibuprofen (1:1) salt

To an assay tube equipped with a magnetic stirrer containing (+)-*O*-desmethyl-Tramadol (53 mg, 0.21 mmol) and chloroform (0.05 ml) was added a solution of (*S*)-Ibuprofen (44 mg, 0.21 mmol, 1 eq.) in chloroform (0.05 ml) at room temperature. 0.10 ml more of chloroform and 3 drops of water were added to the mixture. The biphasic solution was evaporated slowly without stirring at room temperature under atmospheric pressure. After a few days, the remaining residue was redissolved in chloroform (0.2 ml) and stirred with a stirring bar. In a few seconds a white solid precipitated. The resultant suspension was dried at room temperature under atmospheric pressure and then under vacuum. Salt form (+)-*O*-desmethyl-Tramadol-(*S*)-Ibuprofen (1:1) was obtained (97 mg, quantitative yield).

### Example 18b: (+)-O-desmethyl-Tramadol-(S)-Ibuprofen (1:1) salt

To an assay tube equipped with a magnetic stirrer containing (+)-*O*-desmethyl-Tramadol (55 mg, 0.22 mmol), (*S*)-Ibuprofen (45 mg, 0.22 mmol, 1 eq.) and some particles of salt obtained in example 18a as seeding, chloroform (0.5 ml) was added at room temperature. Some solid remained undissolved but in a few minutes a white precipitate appeared. The resultant slurry was stirred for 3 h. Then, the white solid was filtered with a sintered funnel (porosity 4) and washed with cold chloroform (0.2 ml). After vacuum drying at room temperature, salt form (+)-*O-*desmethyl-Tramadol-(*S*)-Ibuprofen (1:1) was obtained (96 mg, 96 % yield).

### ¹H NMR

Proton nuclear magnetic resonance analyses were recorded in deuterated chloroform (CDCl₃) in a Varian Mercury 400 spectrometer, equipped with a broadband probe ATB 1H/19F/X of 5 mm. Spectra were acquired dissolving 5-10 mg of sample in 0.6 ml of deuterated solvent.

¹H NMR spectrum in CDCl₃ at 400 MHz shows peaks at 7.25 (d, *J =* 6.6Hz, 2H); 7.21-7.13 (m, 2H); 7.09-7.02 (m, 2H); 6.92-6.83 (m, 1H); 6.72 (dd, *J* = 1.8Hz, *J* = 8.0Hz, 1H); 3.67 (q, *J* = 6.2Hz, 1H); 2.66-2.51 (m, 1H); 2.42 (d, *J =* 7.0Hz, 2H); 2.29 (d, *J* = 11.7Hz, 1H); 2.23 (s, 6H); 2.03-1.93 (m, 1H); 1.86-1.20 (m, 12H); 0.88 (d, *J* = 6.6Hz, 6H).

### IR

The FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3394.8 (br), 2953.8 (s), 2930.1 (s), 2867.7 (m), 1568.0 (s), 1480.9 (m), 1448.4 (s), 1389.3 (s), 1357.6 (m), 1278.9 (s), 1244.0 (m), 1170.5 (m), 999.6 (m), 864.5 (m), 787.2 (m), 705.1 (m) cm⁻¹.

### DSC

DSC analyses were recorded with a Mettler DSC822^{e}. A sample of 2.0110 mg was weighed into 40 µl aluminium crucible with a pinhole lid and was heated, under nitrogen (50 ml/min), at 2°C/min from 30 to 200 °C.

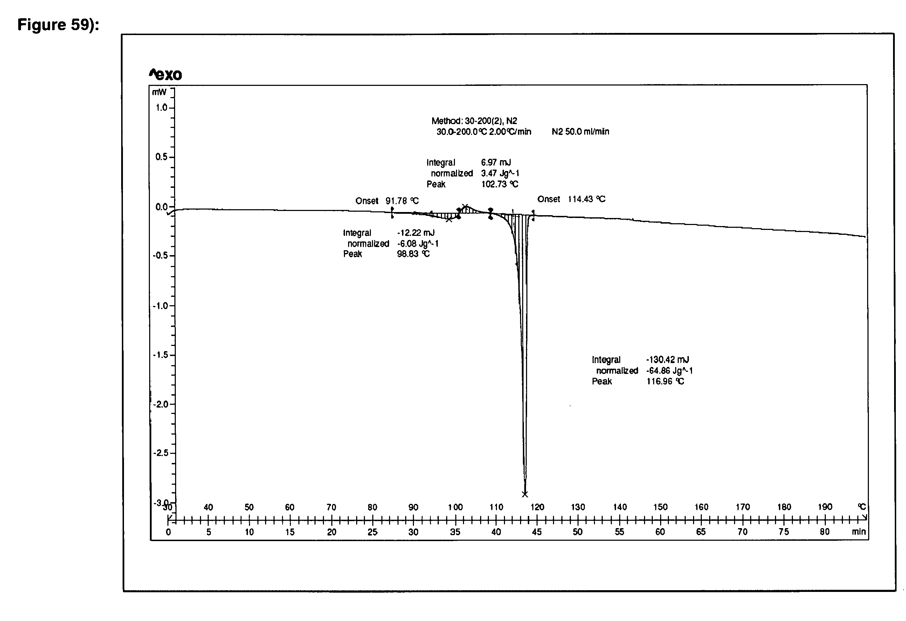

The novel type of crystal of the present invention is **characterized in that** the endothermic sharp peak corresponding to the melting point has an onset at 91.78 °C (fusion enthalpy -6.08 J/g), measured by DSC analysis (2 °C/min) (see figure 59). Another endothermic sharp peak is observed corresponding to the melting point of salt form (+)-*O*-desmethyl-Tramadol-(*S*)-Ibuprofen-H₂O (1:1:0.3) with an onset at 114.43 °C (fusion enthalpy -64.86 J/g), corresponding to the value of the form in example 17, thus indicating a transformation of salt form (+)-*O-*desmethyl-Tramadol-(*S*)-Ibuprofen (1:1) to the dehydration product of salt form (+)-*O-*desmethyl-Tramadol-(*S*)-Ibuprofen-H₂O (1:1:0.3) (example 17).

### TG

Thermogravimetric analyses were recorded in a thermogravimetric analyzer Mettler TGA/SDTA851^{e}. A sample of 2.3360 mg was weighed into a 70 µl alumina crucible with a pinhole lid and was heated at 10 °C/min from 30 to 200 °C, under nitrogen (50 ml/min).

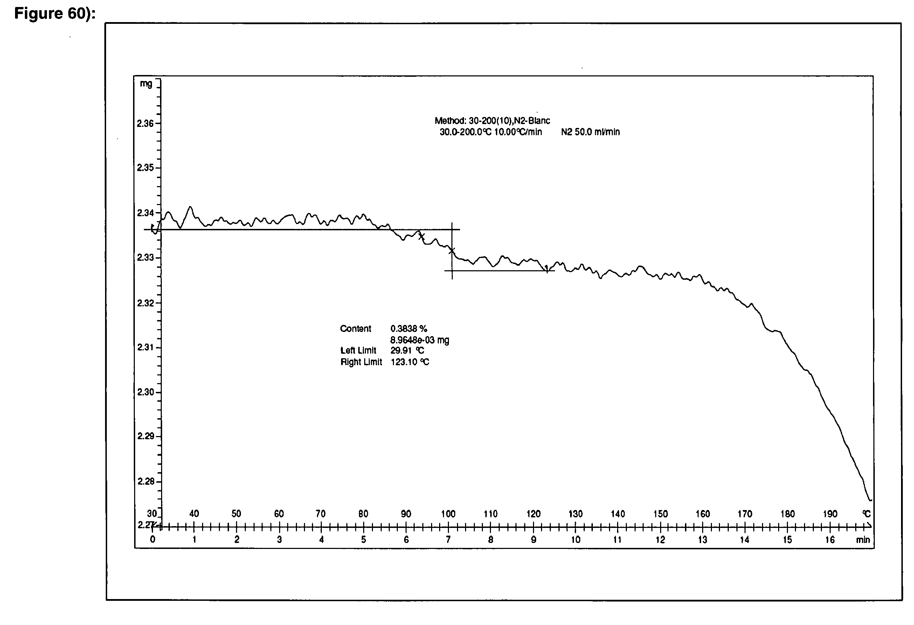

The TG analysis of crystalline of this form according to the invention shows no significant weight loss at temperatures lower than the melting point (see figure 60).

### XRPD

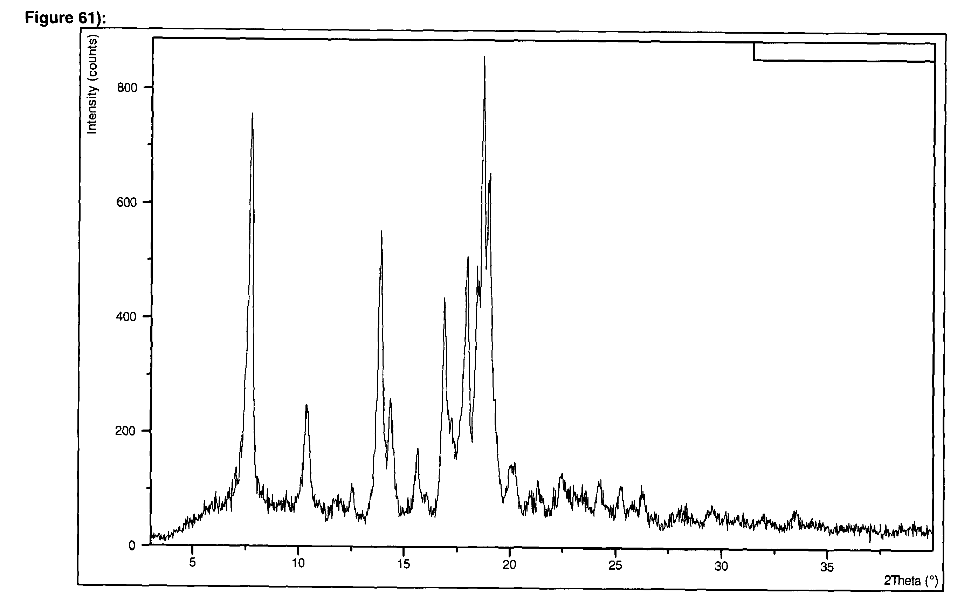

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 34.7° per minute (see figure 61).

### List of selected peaks:

| **2**θ **(°)¹** | **d (Å)** | **I (%)** |
|---|---|---|
| 7.79 | 11.35 | 80 |
| 10.49 | 8.43 | 18 |
| 12.54 | 7.06 | 6 |
| 13.91 | 6.37 | 55 |
| 14.33 | 6.18 | 25 |
| 15.60 | 5.68 | 13 |
| 16.88 | 5.25 | 46 |
| 17.16 | 5.17 | 29 |
| 17.97 | 4.94 | 55 |
| 18.46 | 4.81 | 69 |
| 18.71 | 4.74 | 100 |
| 18.99 | 4.67 | 72 |
| 20.15 | 4.41 | 12 |
| 20.99 | 4.23 | 10 |
| 21.37 | 4.16 | 12 |
| 22.49 | 3.95 | 9 |
| 24.20 | 3.68 | 8 |
| 25.25 | 3.53 | 8 |
| 25.92 | 3.44 | 4 |
| 26.27 | 3.39 | 11 |
| 28.03 | 3.18 | 2 |
| 29.48 | 3.03 | 3 |
| 33.50 | 2.68 | 3 |

| | | |
|---|---|---|
| ¹The 2θ values were obtained using copper radiation (Cu_{κα}). | | |

### Example 19a: (-)-O-desmethyl-Tramadol-(S)-Ibuprofen (1:1) salt

To a vial containing (-)-*O*-desmethyl-Tramadol (55 mg, 0.22 mmol) and (*S*)-Ibuprofen (45 mg, 0.22 mmol, 1eq.) was added tetrahydrofuran (2 ml). The mixture was stirred at room temperature.
The solution was evaporated slowly without stirring at room temperature under atmospheric pressure. After complete evaporation, salt form (-)-*O*-desmethyl-Tramadol-(*S*)-Ibuprofen (1:1) was obtained as colourless crystals (100 mg, quantitative yield).

### Example 19b: (-)-O-desmethyl-Tramadol-(S)-Ibuprofen (1:1) salt

To an assay tube equipped with a magnetic stirrer containing (-)-*O*-desmethyl-Tramadol (55 mg, 0.22 mmol) and (*S*)-Ibuprofen (45 mg, 0.22 mmol) was added toluene (0.5 ml). The resultant mixture was heated and more toluene was added (0.5 ml). The solution was slowly cooled to room temperature. A white precipitate was formed. The solid was filtered with a sintered funnel (porosity 4), washed with cold toluene (0.2 ml) and dried under vacuum at room temperature to give salt form (-)-*O*-desmethyl-Tramadol-(*S*)-Ibuprofen (1:1) as a white solid (83 mg, 83 % yield).

### Example 19c: (-)-O-desmethyl-Tramadol-(S)-Ibuprofen (1:1) salt

To an assay tube equipped with a magnetic stirrer containing (-)-*O*-desmethyl-Tramadol (55 mg, 0.22 mmol) and methyl isobutyl ketone (0.2 ml) was added a solution of (*S*)-Ibuprofen (45 mg, 0.22 mmol) in methyl isobutyl ketone (0.2 ml) at room temperature. The resultant slurry was stirred for 18 h. Then, the white solid was filtered with a sintered funnel (porosity 4) and washed twice with cold methyl isobutyl ketone (0.4 and 0.3 ml). After vacuum drying at room temperature, salt form (-)-*O*-desmethyl-Tramadol-(*S*)-Ibuprofen (1:1) was obtained (87 mg, 87 % yield).

### Example 19d: (-)-O-desmethyl-Tramadol-(S)-Ibuprofen (1:1) salt

To an assay tube equipped with a magnetic stirrer containing (-)-*O*-desmethyl-Tramadol (55 mg, 0.22 mmol) and acetone (1 ml) was added a solution of (*S*)-Ibuprofen (45 mg, 0.22 mmol) in acetone (0.25 ml) at room temperature. After a few minutes a white precipitate appeared and the mixture was stirred for 1 h. Then, the white solid was filtered with a sintered funnel (porosity 4) and washed with cold acetone (0.1 ml). After vacuum drying at room temperature, salt form (-)-*O*-desmethyl-Tramadol-(*S*)-Ibuprofen (1:1) was obtained (87 mg, 87 % yield).

### ¹H NMR

Proton nuclear magnetic resonance analyses were recorded in deuterated dimethylsulfoxide (d6-DMSO) in a Varian Mercury 400 spectrometer, equipped with a broadband probe ATB 1H/19F/X of 5 mm. Spectra were acquired dissolving 5-10 mg of sample in 0.6 ml of deuterated solvent.

¹H NMR spectrum in d6-DMSO at 400 MHz shows peaks at 7.18 (d, *J* = 7.8 Hz, 2H); 7.12-7.01 (m, 3H); 6.89 (s, 1H); 6.82 (d, *J* = 7.4 Hz, 1H); 6.55 (d, *J* = 8.2 Hz, 1H); 3.60 (q, *J* = 7.0 Hz, 1H); 2.40 (d, *J* = 7.0 Hz, 2H); 2.14 (dd, *J* = 10.0 Hz, *J* = 11.9 Hz, 1H); 1.93 (s, 6H); 1.85-1.25 (m, 13H); 0.85 (d, *J* = 6.3 Hz, 6H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3359.4 (s, br), 3086.5 (m, br), 3025.7 (m), 2984.5 (m), 2956.3 (s), 2929.7 (s), 2869.4 (m), 1579.4 (s, br), 1510.0 (s), 1482.9 (s), 1447.2 (s), 1411.8 (m), 1389.6 (s), 1357.5 (s), 1277.7 (m), 1240.4 (m), 1209.3 (m), 1170.1 (m), 1108.8 (m), 1000.7 (s), 877.3 (m), 867.1 (s), 860.8 (s), 790.9 (m), 722.5 (m), 705.0 (m), 681.4 (m), 540.7 (m), 453.3 (m) cm⁻¹.

### DSC

DSC analyses were recorded with a Mettler DSC822^{e}. A sample of 1.4770 mg was weighed into 40 µl aluminium crucible with a pinhole lid and was heated, under nitrogen (50 ml/min), at 10°C/min from 30 to 300 °C.

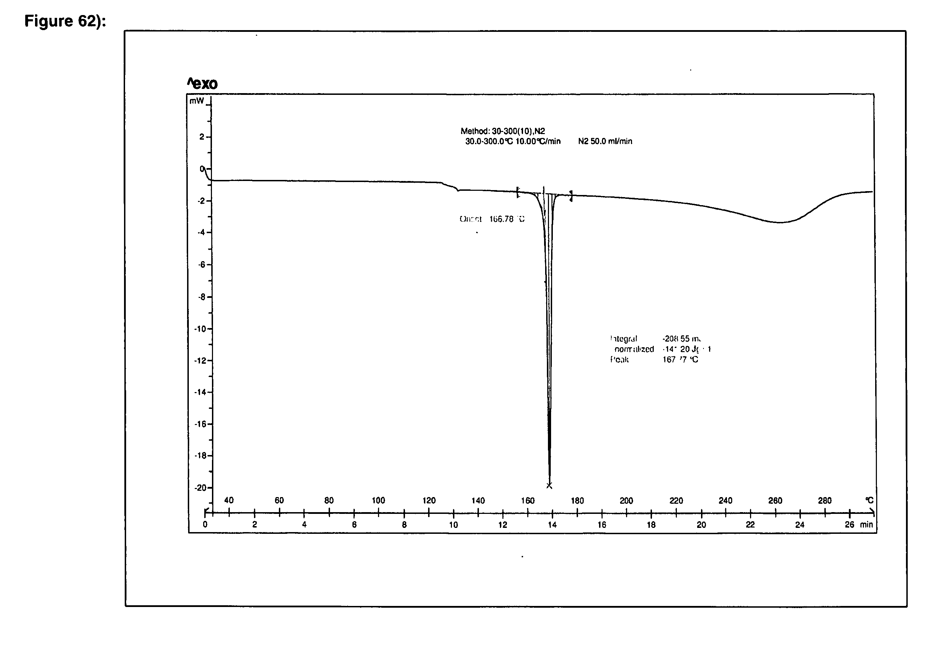

The novel type of crystal of the present invention is **characterized in that** the endothermic sharp peak corresponding to the melting point has an onset at 166.78 °C (fusion enthalpy -141.20 J/g), measured by DSC analysis (10 °C/min) (see figure 62).

### TG

Thermogravimetric analyses were recorded in a thermogravimetric analyzer Mettler TGA/SDTA851^{e}. A sample of 5.7417 mg was weighed into a 70 µl alumina crucible with a pinhole lid and was heated at 10 °C/min from 30 to 300 °C, under nitrogen (50 ml/min).

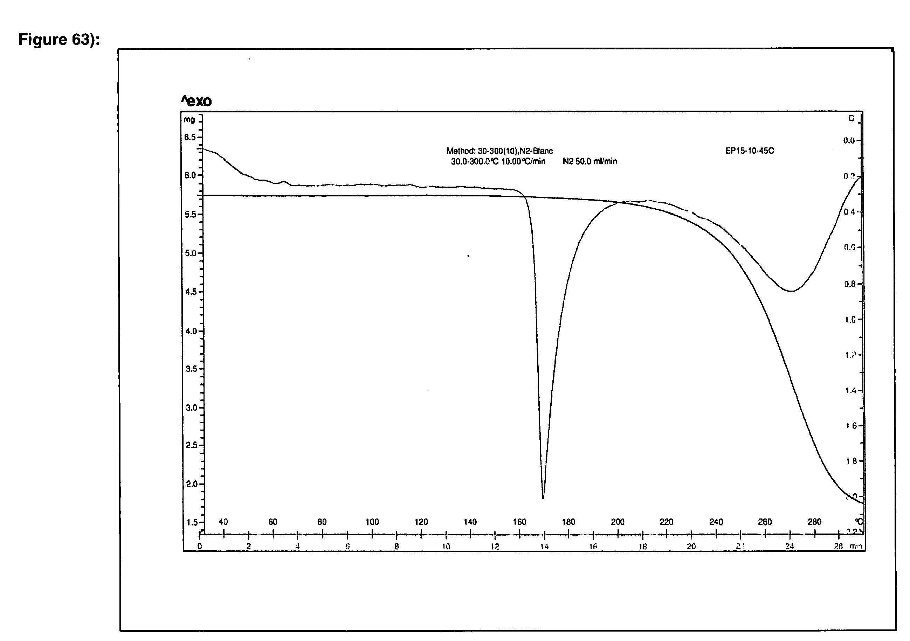

The TG analysis of the crystalline form according to the invention shows no weight loss at temperatures lower than the melting point (see figure 63).

### XRPD

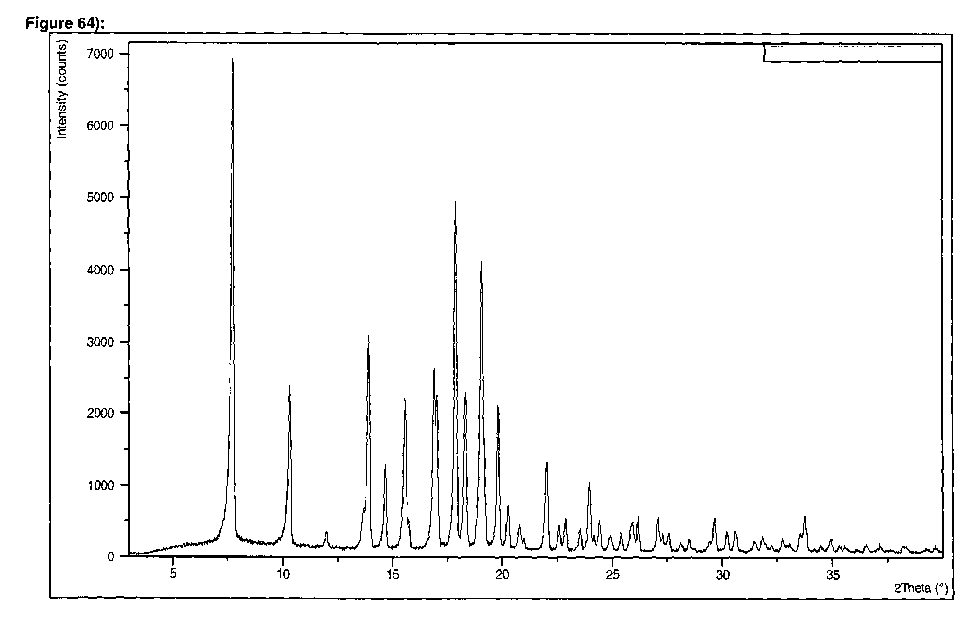

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 64).

### List of selected peaks:

| **2**θ **(°)¹** | **d (Å)** | **I (%)** |
|---|---|---|
| 7.79 | 11.35 | 100 |
| 10.36 | 8.54 | 35 |
| 12.02 | 7.36 | 5 |
| 13.95 | 6.35 | 46 |
| 14.70 | 6.02 | 19 |
| 15.60 | 5.68 | 33 |
| 16.89 | 5.25 | 41 |
| 17.05 | 5.20 | 33 |
| 17.90 | 4.96 | 75 |
| 18.34 | 4.84 | 35 |
| 19.07 | 4.65 | 64 |
| 19.83 | 4.48 | 32 |
| 20.28 | 4.38 | 10 |
| 20.81 | 4.27 | 6 |
| 20.99 | 4.23 | 4 |
| 22.04 | 4.03 | 20 |
| 22.59 | 3.94 | 6 |
| 22.89 | 3.89 | 8 |
| 23.54 | 3.78 | 5 |
| 23.96 | 3.71 | 16 |
| 24.43 | 3.64 | 8 |
| 24.87 | 3.58 | 4 |
| 25.39 | 3.51 | 5 |
| 25.94 | 3.44 | 7 |
| 26.18 | 3.40 | 8 |
| 27.08 | 3.29 | 8 |
| 27.28 | 3.27 | 5 |
| 27.56 | 3.24 | 5 |
| 28.10 | 3.18 | 2 |
| 28.49 | 3.13 | 3 |
| 29.66 | 3.01 | 8 |
| 30.23 | 2.96 | 5 |
| 30.60 | 2.92 | 5 |
| 31.47 | 2.84 | 3 |
| 31.83 | 2.81 | 4 |
| 32.24 | 2.78 | 2 |
| 32.74 | 2.74 | 3 |
| 33.75 | 2.66 | 9 |
| 34.47 | 2.60 | 2 |
| 34.92 | 2.57 | 3 |
| 35.31 | 2.54 | 2 |
| 35.47 | 2.53 | 1 |
| 36.51 | 2.46 | 2 |
| 37.15 | 2.42 | 2 |
| 38.25 | 2.35 | 2 |
| 39.29 | 2.29 | 1 |
| 39.64 | 2.28 | 2 |

| | | |
|---|---|---|
| ¹The 2θ values were obtained using copper radiation (Cu_{κα}). | | |

### Single crystal X-ray diffraction

The crystal structure of this form has been determined from single crystal X-ray diffraction data. The colourless plate used (0.29 × 0.26 × 0.07 mm) was obtained from the evaporation of an acetonitrile solution of equimolar amounts of (-)-*O*-desmethyl-Tramadol and (*S*)-Ibuprofen. Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters.

### Relevant structural data:

| Crystal system | Monoclinic |
|---|---|
| Space group | P2₁ |
| a (Å) | 8.9646(10) |
| b (Å) | 12.9258(14) |
| c (Å) | 11.3901(13) |
| β (°) | 93.959(2) |
| Volume (Å³) | 1316.7(3) |
| Z | 2 |
| D calc. (Mg/m³) | 1.149 |
| N. of refl. | 5415 |
| Refl. with I > 2σ(I) | 2783 |
| R (I > 2σ(I)) | 0.0714 |

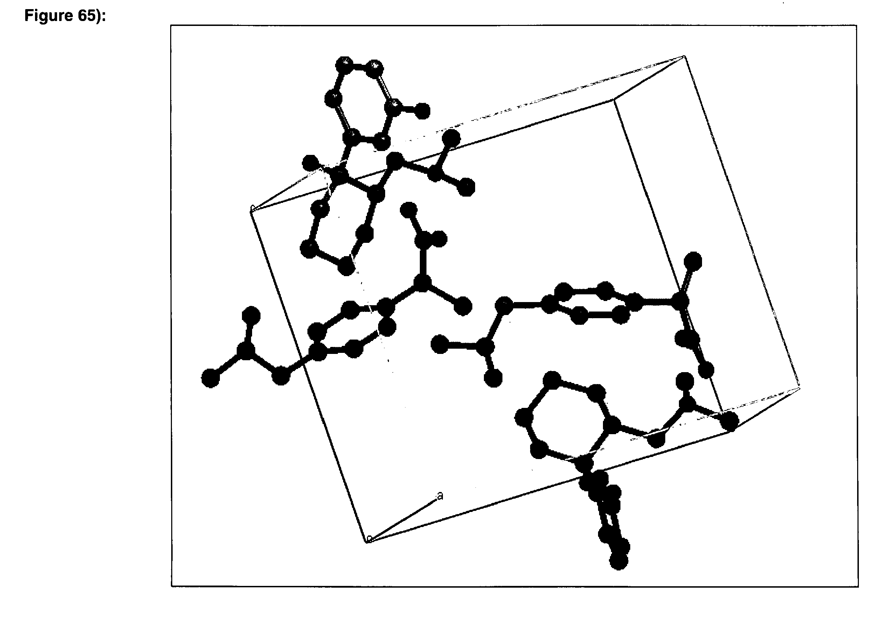

The crystal structure of this form is depicted in figure 65.
Simulation of the XRPD diffractogram from single crystal data gives an almost identical diagram to the experimental one presented above.

### Example 20a: (rac)-O-desmethyl-Tramadol-(S)-Ibuprofen (2:3) co-crystal

To an assay tube equipped with a magnetic stirrer containing (*rac*)-*O*-desmethyl-Tramadol (29 mg, 0.12 mmol) and (*S*)-Ibuprofen (71 mg, 0.35 mmol, 3 eq.) was added toluene (0.4 ml). The resultant slurry was stirred for 3 h at room temperature.
The solid was filtered with a sintered funnel (porosity 4) and washed with cold toluene (0.05 ml). After vacuum drying at room temperature, crystal form (*rac*)-*O*-desmethyl-Tramadol-(*S*)-Ibuprofen (2:3) was obtained as a white solid (59 mg, 91 % yield).

### Example 20b: (rac)-O-desmethyl-Tramadol-(S)-Ibuprofen (2:3) co-crystal

To an assay tube equipped with a magnetic stirrer containing (*rac*)-*O*-desmethyl-Tramadol (38 mg, 0.15 mmol), (*S*)-Ibuprofen (62 mg, 0.30 mmol) and some particles of crystal form obtained in example 20a as seeding, isobutyl acetate (1 ml) was added at room temperature. The mixture was stirred 4 h at room temperature.
The solid was filtered with a sintered funnel (porosity n°4) and washed with cold AcO*i*Bu (butyl acetate) (0.1 ml). After drying under vacuum at room temperature, crystal form (*rac*)-*O-*desmethyl-Tramadol-(*S*)-Ibuprofen (2:3) was obtained as a white solid (76 mg, 90 % yield).

### ¹H NMR

Proton nuclear magnetic resonance analyses were recorded in deuterated chloroform (CDCl₃) in a Varian Mercury 400 spectrometer, equipped with a broadband probe ATB 1H/19F/X of 5 mm. Spectra were acquired dissolving 5-10 mg of sample in 0.6 ml of deuterated solvent.

¹H NMR spectrum in CDCl₃ at 400 MHz shows peaks at 7.24 (d, *J=* 8.2Hz, 1.5×2H); 7.16 (t, *J* = 8.0Hz, 1H); 7.10 (s br, 1H); 7.05 (d, *J* = 7.8Hz, 1.5×2H); 6.86 (s br, 1H); 6.73 (dd, *J* = 1.8Hz, *J=* 8.0Hz, 1H); 3.67 (q, *J =* 7.0Hz, 1.5×1H); 2.69-2.59 (m, 1H); 2.42 (d, *J =* 7.4Hz, 1.5×2H); 2.38-2.30 (m, 1H); 2.26 (s, 3H); 2.25 (s, 3H); 2.01 (s br, 1H); 1.90-1.13 (m, 14H); 0.89 (d, *J* = 6.6Hz, 1.5×6H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3251.9 (m, br), 2943.7 (m), 1719.2 (s), 1579.3 (s), 1204.5 (m), 1175.7 (m), 1110.7 (m), 1062.8 (m), 1001.5 (s), 966.5 (m), 879.5 (m), 864.1 (s), 790.4 (s), 706.4 (s), 668.1 (m), 633.8 (m), 596.8 (m), 466.6 (m) cm⁻¹.

### DSC

DSC analyses were recorded with a Mettler DSC822^{e}. A sample of 1.3520 mg was weighed into 40 µl aluminium crucible with a pinhole lid and was heated, under nitrogen (50 ml/min), at 10°C/min from 30 to 200 °C.

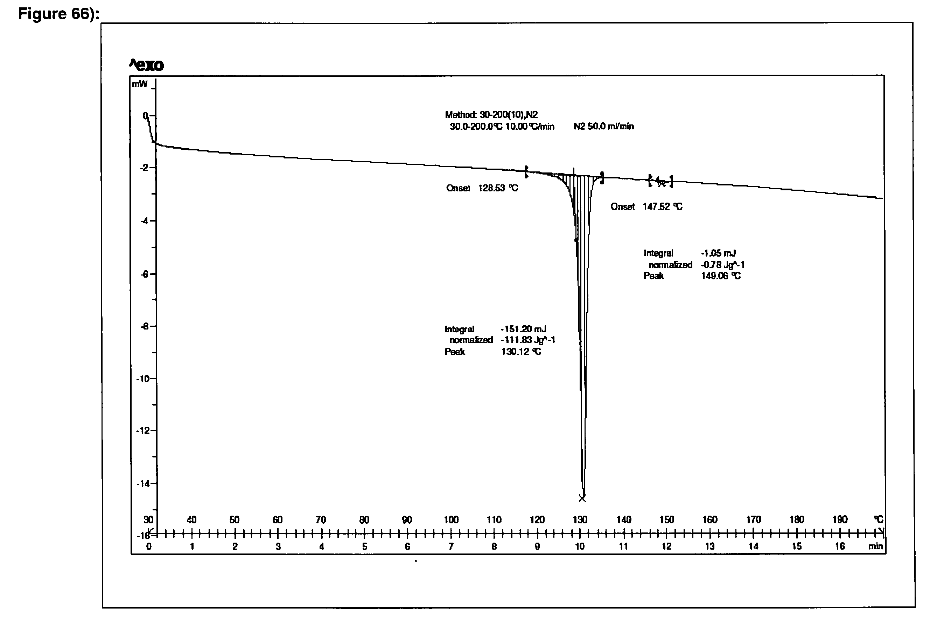

The novel type of crystal of the present invention is **characterized in that** the endothermic sharp peak corresponding to the melting point has an onset at 128.53 °C (fusion enthalpy -111.83 J/g), measured by DSC analysis (10 °C/min) (see figure 66).

### TG

Thermogravimetric analyses were recorded in a thermogravimetric analyzer Mettler TGA/SDTA851^{e}. A sample of 2.1826 mg was weighed into a 70 µl alumina crucible with a pinhole lid and was heated at 10 °C/min from 30 to 200 °C, under nitrogen (50 ml/min).

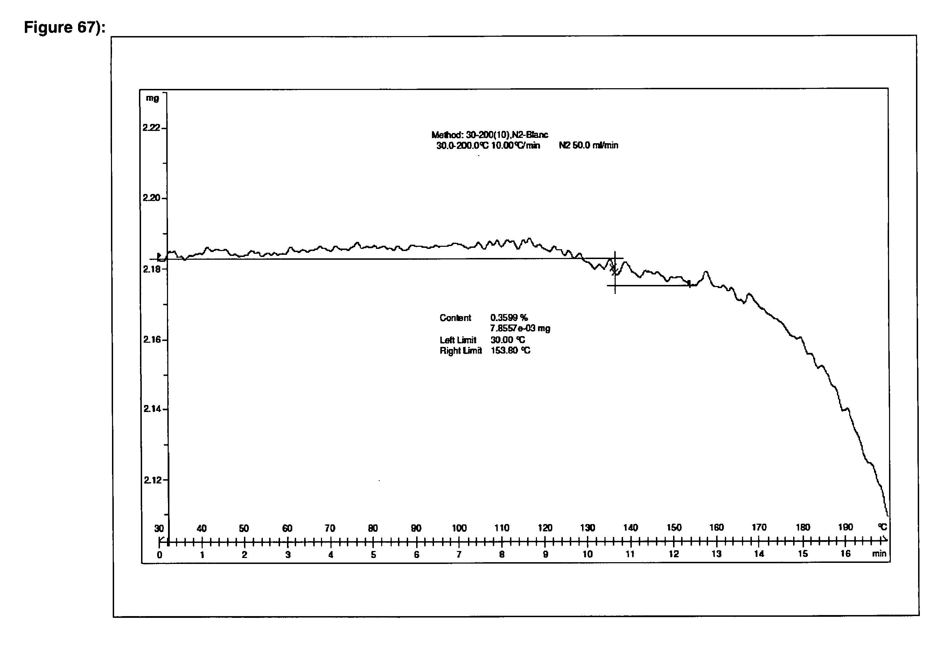

The TG analysis of the crystalline form according to the invention shows no weight loss at temperatures lower than the melting point (see figure 67).

### XRPD

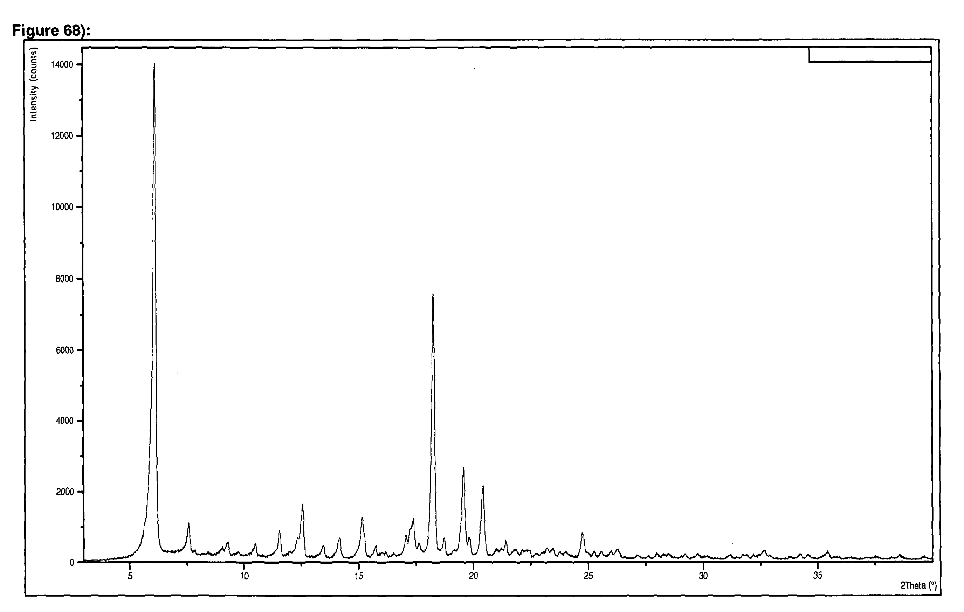

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute (see figure 68).

### List of selected peaks:

| **2θ (°)¹** | **d (Å)** | **I (%)** |
|---|---|---|
| 6.16 | 14.34 | 100 |
| 7.61 | 11.61 | 8 |
| 9.04 | 9.79 | 2 |
| 9.30 | 9.51 | 3 |
| 9.75 | 9.07 | 1 |
| 10.48 | 8.44 | 3 |
| 11.57 | 7.65 | 6 |
| 12.57 | 7.04 | 11 |
| 13.48 | 6.57 | 3 |
| 14.17 | 6.25 | 4 |
| 15.16 | 5.85 | 9 |
| 15.75 | 5.63 | 3 |
| 17.06 | 5.20 | 5 |
| 17.37 | 5.10 | 9 |
| 17.64 | 5.03 | 3 |
| 18.29 | 4.85 | 55 |
| 18.73 | 4.74 | 4 |
| 19.60 | 4.53 | 19 |
| 19.81 | 4.48 | 5 |
| 20.43 | 4.35 | 15 |
| 21.40 | 4.15 | 4 |
| 21.86 | 4.07 | 2 |
| 22.47 | 3.96 | 2 |
| 23.21 | 3.83 | 2 |
| 23.49 | 3.79 | 2 |
| 23.78 | 3.74 | 1 |
| 24.02 | 3.71 | 2 |
| 24.76 | 3.60 | 6 |
| 25.27 | 3.52 | 2 |
| 25.58 | 3.48 | 2 |
| 26.03 | 3.42 | 2 |
| 26.31 | 3.39 | 2 |
| 28.00 | 3.19 | 1 |
| 28.53 | 3.13 | 1 |
| 32.71 | 2.74 | 2 |
| 35.44 | 2.53 | 1 |

| | | |
|---|---|---|
| ¹The 2θ values were obtained using copper radiation (Cu_{Kα}). | | |

### Single crystal X-ray diffraction

The crystal structure of this form has been determined from single crystal X-ray diffraction data. The colourless plate used (0.32 × 0.24 × 0.06 mm) was obtained from the evaporation of a MIK (methyl isobutyl ketone) solution of (*rac*)-*O*-desmethyl-Tramadol and (*S*)-Ibuprofen in a 1:2 ratio. Analysis was performed at room temperature using a Bruker Smart Apex diffractometer with graphite monochromated Mo K_{α}, radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0). The structure was solved with direct methods and least-squares refinement of Fₒ² against all measured intensities was carried out (program used: SHELXTL-NT 6.1). All non-hydrogen atoms were refined with anisotropic displacement parameters, except some carbon atoms of one disordered (*S*)-Ibuprofen molecule.

### Relevant structural data:

| Crystal system | Orthorhombic |
|---|---|
| Space group | *P*2₁2₁2₁ |
| a (Å) | 12.6916(11) |
| b (Å) | 18.0869(15) |
| c (Å) | 28.729(3) |
| Volume (Å³) | 6594.9(16) |
| Z | 4 |
| D calc. (Mg/m³) | 1.126 |
| N. of refl. | 16248 |
| Refl. with I > 2σ(I) | 5725 |
| R (I > 2σ(I)) | 0.0935 |

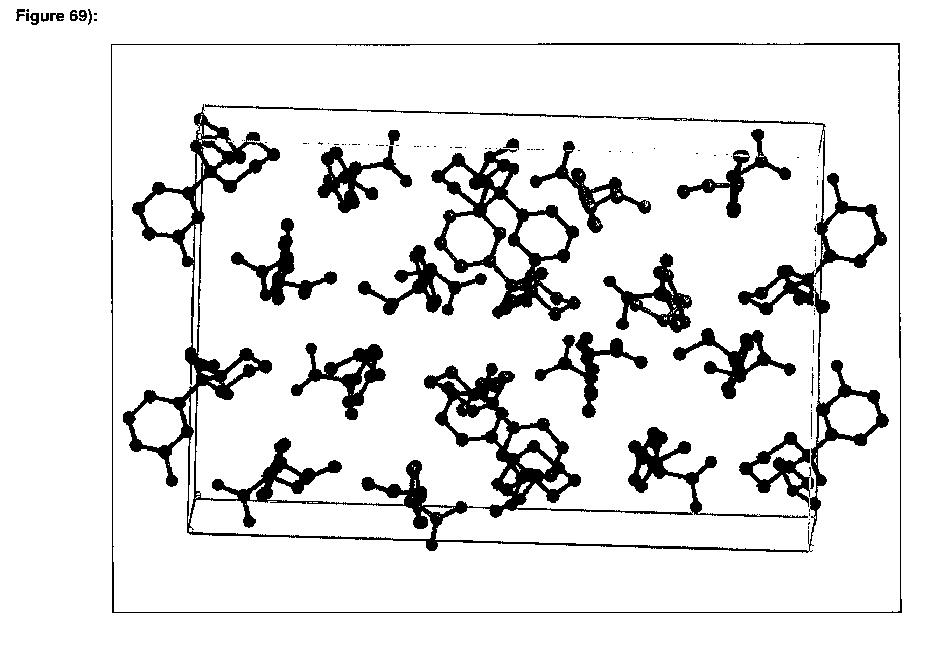

The crystal structure of this form is depicted in figure 69.
Simulation of the XRPD diffractogram from single crystal data gives an almost identical diagram to the experimental one presented above.

### Example 21a: (rac)-O-desmethyl-Tramadol·HCl-Paracetamol (1:1) co-crystal

To an assay tube with magnetic stirring containing (*rac*)-*O*-desmethyl-Tramadol·HCl (70 mg, 0.24 mmol) and Paracetamol (37 mg, 0.24 mmol, 1 eq.) in suspension in 0.4 ml CH₂Cl₂ was added at room temperature MeOH until reach complete dissolution (V_{MeOH}=0.09 ml). Then, 0.3 ml CH₂Cl₂ was added slowly before seeding with this form obtained as described in the note below. The mixture was cooled to 0 °C and stirred for 2 h at 0 °C. The white solid is filtered with a sinter funnel (porosity 3) and washed with 0.1 ml CH₂Cl₂. After drying at room temperature under vacuum, co-crystal form (*rac*)-*O*-desmethyl-Tramadol·HCl-Paracetamol 1:1 was obtained as white solid (20 mg, 19 % yield).

Note: this form was obtained first by wet grinding MeOH between (*rac*)-*O*-desmethyl-Tramadol·HCl and Paracetamol (1:1).

### Example 21b: (rac)-O-desmethyl-Tramadol·HCl-Paracetamol (1:1) co-crystal

To a 50 ml flask with magnetical stirring containing (*rac*)-*O*-desmethyl-Tramadol·HCl (1 g, 3.5 mmol) and Paracetamol (530 mg, 3.5 mmol, 1 eq.) in suspension in 3.7 ml CH₂Cl₂ was added at room temperature 1.5 ml MeOH. A part of the solvent was distillated until reach complete dissolution (V_{dist}= 1.5 ml). Then, the solution was cooled to room temperature before adding slowly 6 ml CH₂Cl₂ and seeding with form obtained in example 21a.
The mixture was cooled to 0 °C before adding dropwise 10 ml CH₂Cl₂ (3 ml/h). After the addition, the mixture was stirred 1 h at 0 °C. The white solid was filtered with a sinter funnel (porosity 3) and washed with 1.5 ml CH₂Cl₂. After drying at room temperature under vacuum, co-crystal form (*rac*)-*O*-desmethyl-Tramadol·HCl-Paracetamol 1:1 was obtained as white solid (950 mg, 62 % yield).

### ¹H NMR

Proton nuclear magnetic resonance analysis was recorded in deuterated dimethylsulfoxide (d6-DMSO) in a Varian Mercury 400 spectrometer, equipped with a broadband probe ATB 1H/19F/X of 5 mm. Spectrum was acquired dissolving 5-10 mg of sample in 0.6 ml of deuterated solvent.

¹H NMR spectrum in d6-DMSO at 400 MHz shows peaks at 9.63 (s br, 1H); 9.30 (s, 1H); 9.20 (s br, 1H); 9.11 (s, 1H); 7.34-7.28 (m, 2H); 7.11 (t, *J* = 7.8 Hz, 3H); 6.94-6.89 (m, 1H); 6.87 (d br, *J* = 7.8 Hz, 1H); 6.68-6.62 (m, 2H); 6.61 (dd, *J=* 2.3 Hz, *J=* 7.8 Hz, 2H); 5.00 (s, -O*H*, 1H); 2.90-2.76 (m, 1H); 2.60 (s br, N-C*H*₃, 3H); 2.44 (s br, N-C*H*₃, 3H); 2.44-2.34 (m, 1H); 2.15-2.04 (m, 1H); 1.96 (s, 3H); 1.90-1.78 (m, 1H); 1.77-1.29 (m, 7H) ppm.

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

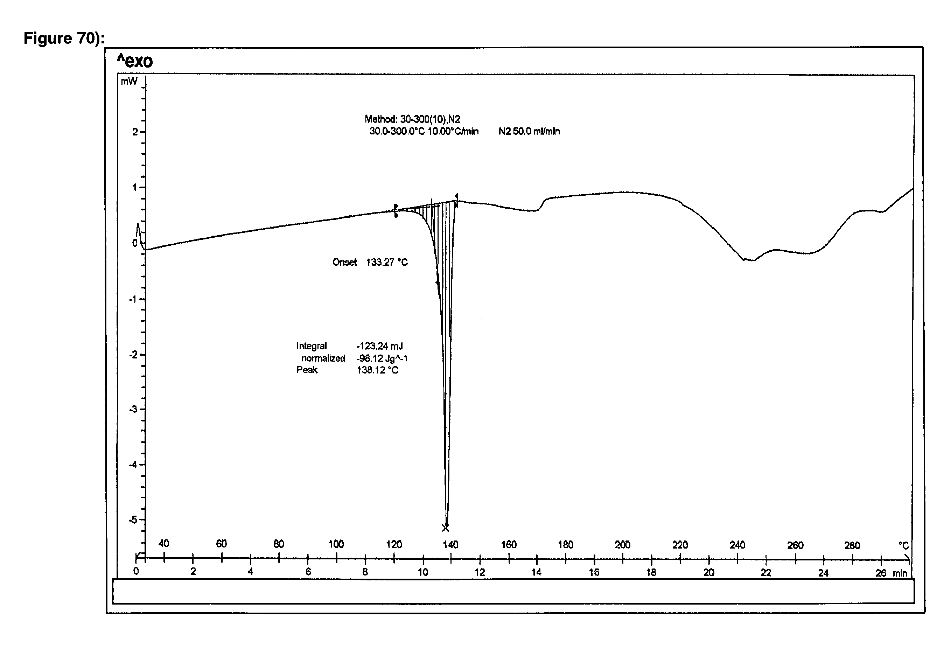

The sample (KBr pellets) shows a Fourier Transform Infra Red spectrum with absorption bands at 3308.5 (s, br), 3116.8 (s, br), 2930.9 (m), 2861.9 (m), 2677.3 (m, br), 1653.6 (s), 1610.2 (m), 1582.4 (s), 1537.5 (s), 1511.9 (s), 1263.6 (s), 1239.3 (m), 1212.8 (m), 1177.7 (m), 1162.3 (m), 1132.5 (m), 986.9 (m), 792.0 (s), 706.3 (m), 523.0 (m), 503.5 (m) cm⁻¹ (see figure 70).

### DSC

DSC analysis was recorded with a Mettler DSC822^{e}. A sample of 1.2560 mg was weighed into 40 µl aluminium crucible with a pinhole lid and was heated, under nitrogen (50 ml/min), at 10 °C/min from 30 to 300 °C.

The novel type of crystal of the present invention is **characterized in that** the endothermic sharp peak corresponding to the melting point has an onset at 133.27 °C (fusion enthalpy -98.12 J/g), measured by DSC analysis (10 °C/min) (see figure 70).

### TG

Thermogravimetric analysis was recorded in a thermogravimetric analyzer Mettler TGA/SDTA851^{e}. A sample of 3.0318 mg was weighed into a 70 µl alumina crucible with a pinhole lid and was heated at 10 °C/min from 30 to 300 °C, under nitrogen (50 ml/min).

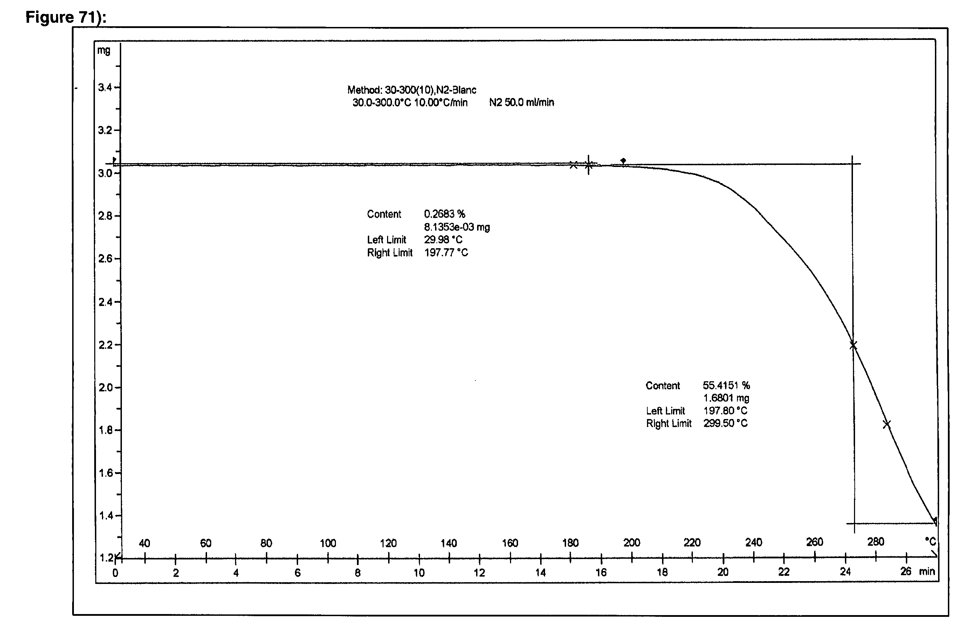

The TG analysis of the crystalline form according to the invention shows no significant weight loss at temperatures lower than the melting point (see figure 71).

### XRPD: X-ray powder diffraction pattern

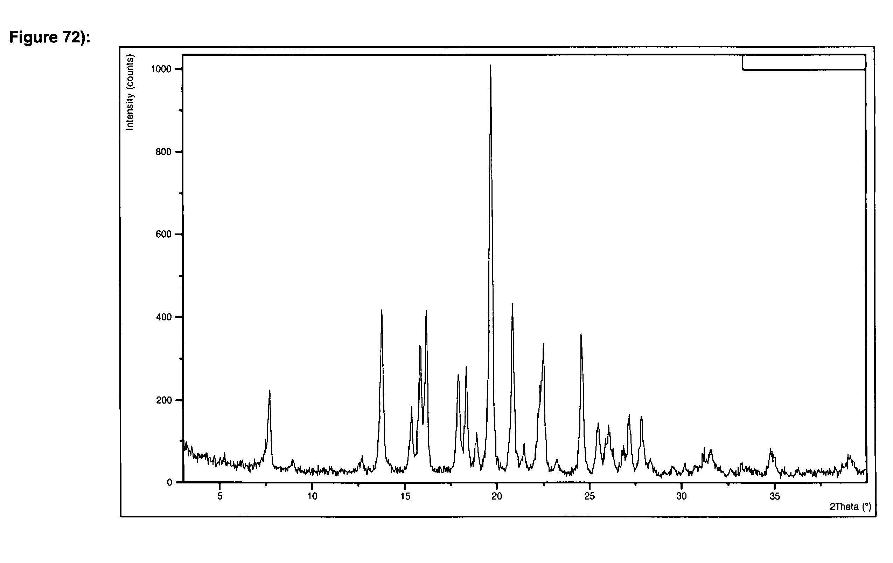

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 40° at a scan rate of 1.8° per minute (see figure 72).

### List of selected peaks:

| **2**θ **(°)¹** | **d (Å)** | **I (%)** |
|---|---|---|
| 7.71 | 11.47 | 48 |
| 8.95 | 9.89 | 5 |
| 12.72 | 6.96 | 4 |
| 13.78 | 6.43 | 57 |
| 15.36 | 5.77 | 17 |
| 15.86 | 5.59 | 38 |
| 16.19 | 5.48 | 48 |
| 17.93 | 4.95 | 26 |
| 18.35 | 4.83 | 27 |
| 18.89 | 4.70 | 8 |
| 19.72 | 4.50 | 100 |
| 20.87 | 4.26 | 39 |
| 21.48 | 4.14 | 7 |
| 22.52 | 3.95 | 27 |
| 13.24 | 3.83 | 3 |
| 24.60 | 3.62 | 28 |
| 25.48 | 3.50 | 10 |
| 26.09 | 3.42 | 7 |
| 26.84 | 3.32 | 3 |
| 27.14 | 3.29 | 9 |
| 27.84 | 3.20 | 10 |
| 29.54 | 3.02 | 1 |
| 30.18 | 2.96 | 2 |
| 31.21 | 2.87 | 3 |
| 31.59 | 2.83 | 4 |
| 32.64 | 2.74 | 1 |
| 33.20 | 2.70 | 2 |
| 34.80 | 2.58 | 3 |
| 36.21 | 2.48 | 1 |
| 38.30 | 2.35 | 1 |
| 39.04 | 2.31 | 2 |

| | | |
|---|---|---|
| ¹The 2θ values were obtained using copper radiation (Cu_{Kα}). | | |

### Single crystal X-ray diffraction analysis

Crystal structure of this form has been determined from single crystal X-ray diffraction data. The colourless crystal used (0.29 × 0.13 × 0.04 mm) was obtained from vapour diffusion, with a seeded solution of equimolar amounts of (*rac*)-*O*-desmethyl-Tramadol·HCl and Paracetamol in dichloromethane, in an atmosphere of methanol.
Analysis was performed at room temperature using a Bruker Smart Apex diffractometer with graphite monochromated Mo K_{α} radiation equipped with a CCD detector.
Data were collected using phi and omega scans (program used: SMART 5.6). No significant decay of standard intensities was observed. Data reduction (Lorentz and polarization corrections) and absorption correction were applied (program used: SAINT 5.0).
The structure was solved with direct methods and least-squares refinement of Fₒ² against all measured intensities was carried out (program used: SHELXTL -NT 6.1). All non-hydrogen atoms were refined with anisotropic displacement parameters.

### Relevant structural data:

| Crystal system | Monoclinic |
|---|---|
| Space group | Cc |
| a (Å) | 23.538(3) |
| b (Å) | 10.9852(14) |
| c (Å) | 9.2583(12) |
| β (°) | 100.862(3) |
| Volume (Å³) | 2351.0(5) |
| Z | 4 |
| D calc. (Mg/m³) | 1.235 |
| N. of refl. | 2091 |
| Refl. with I > 2σ(I) | 3820 |
| R (I > 2σ(I)) | 0.0575 |

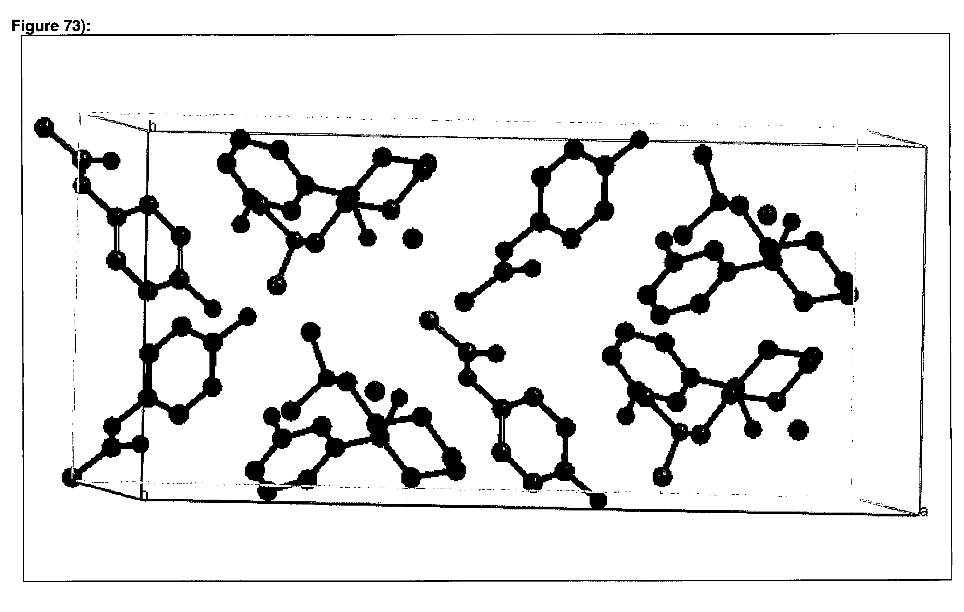

The crystal structure of this form is depicted in figure 73.
Simulation of XRPD diffractogram from single crystal data gives an almost identical diagram to the experimental one presented above.

## Claims

1. Pharmaceutical compound comprising *O*-desmethyl-Tramadol and at least one COX-inhibitor.

2. Pharmaceutical compound according to claim 1, wherein the *O*-desmethyl-Tramadol is racemic *O*-desmethyl-Tramadol, (+)-*O*-desmethyl-Tramadol, (-)-*O*-desmethyl-Tramadol.

3. Pharmaceutical compound according to any one of claims 1 or 2, wherein the COX-inhibitor is selected from the group consisting of
Acetylsalicylic acid, Diflunisal, Ethenzamide, Salicylamide, Triflusal, Fosfosal, Benorylate, Paracetamol, Propacetamol, Phenidine, Etofenamate, Flufenamic acid, Meclofenamic acid, Mefenamic acid, Niflumic acid, Tolfenamic acid, Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, Felbinac, Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, Clidanac, Metamizol, Propylphenazone, Kebuzone, Mofebutazone, Oxyphenbutazone, Phenylbutazone, Apazone, Isoxicam, Lornoxicam, Piroxicam, Tenoxicam, Ketorolac, Proquazone, Oxaprozin, Ditazole, Etodolac, Meloxicam, Nimesulide, Celecoxib, Etoricoxib, Lumiracoxib, Parecoxib, Rofecoxib, Valdecoxib, Cimicoxib, Bermoprofen, Pelubiprofen, Tenosal, Aceneuramic acid, Pirazolac, Xinoprofen, Flobufen, Anirolac, Zoliprofen, Bromfenac, Pemedolac, Dexpemedolac, Bindarit, Romazarit, Fenbufen; or their stereoisomers, salts or metabolites;
more preferably is selected from the group consisting of
Acetylsalicylic acid, Triflusal, HTB (2-hydroxy-4-trifluoromethyl benzoic acid), Diflunisal, Meclofenamic acid, Mefenamic acid, Niflumic acid, Flufenamic acid, Diclofenac, Lonazolac, Acemetacin, Indomethacin, Tolmetin, Sulindac, Etodolac, Ketorolac, Flurbiprofen, (*RS*)-Flurbiprofen, (*R*)-Flurbiprofen, Esflurbiprofen, Ibuprofen, (*RS*)-Ibuprofen, (*S*)-(+)-Ibuprofen, Ketoprofen, (*rac*)-Ketoprofen, (*R*)-(-)-Ketoprofen, Bermoprofen, Pelubiprofen, Tenosal, Aceneuramic acid, Pirazolac, Xinoprofen, Flobufen, Anirolac, Zoliprofen, Bromfenac, Pemedolac, Dexpemedolac, Bindarit, Romazarit, Naproxen, (*S*)-Naproxen, Tiaprofenic acid, Fenbufen, Fenoprofen, Flobufen, Oxaprozin or Paracetamol; their stereoisomers or salts.

4. Pharmaceutical compound according to any one of claims 1 - 3, wherein the compound is a salt.

5. Pharmaceutical compound according to claim 4, wherein the salt is selected from the group consisting of (*rac*)-*O*-desmethyl-Tramadol - Diclofenac, (+)-*O*-desmethyl-Tramadol - Diclofenac, (*rac*)-*O*-desmethyl-Tramadol - Acetylsalicylic acid, (+)-*O-*desmethyl-Tramadol - Acetylsalicylic acid, (*rac*)-*O*-desmethyl-Tramadol - Triflusal, (+)-*O*-desmethyl-Tramadol - Triflusal, (*rac*)-*O*-desmethyl-Tramadol - (*S*)-Naproxen, (+)-*O-*desmethyl-Tramadol - (*S*)-Naproxen, (-)-*O*-desmethyl-Tramadol - (*S*)-Naproxen, (+)-*O-*desmethyl-Tramadol - (*R*)-Flurbiprofen, (-)-*O*-desmethyl-Tramadol - (R)-Flurbiprofen, (+)-*O*-desmethyl-Tramadol - (*S*)-Ibuprofen, or (-)-*O*-desmethyl-Tramadol - (*S*)-Ibuprofen.

6. Pharmaceutical compound according to claim 5, wherein the molecular ratio in the salt of *O*-desmethyl-Tramadol to COX-inhibitor is 1:2 to 2:1, preferably is 2:3 to 3:2 or 1:1.

7. Pharmaceutical compound according to claim 4, wherein the salt is a hydrate.

8. Pharmaceutical compound according to any of claims 1 - 7, wherein the compound is a crystal of the salt or hydrate.

9. Pharmaceutical compound according to claim 8, wherein the compound is a crystal selected from crystal forms of
• (*rac*)-*O*-desmethyl-Tramadol - Diclofenac (1:1) salt, form A,
• (*rac*)-*O*-desmethyl-Tramadol - Diclofenac (1:1) salt, form B,
• (+)-*O*-desmethyl-Tramadol - Diclofenac - H₂O (1:1:0.7-0.8) salt,
• (*rac*)-*O*-desmethyl-Tramadol - Acetylsalicylic acid (1:1) salt,
• (+)-*O*-desmethyl-Tramadol - Acetylsalicylic acid (1:1) salt, form A,
• (+)-*O*-desmethyl-Tramadol - Acetylsalicylic acid (1:1) salt, form B,
• (*rac*)-*O*-desmethyl-Tramadol - Triflusal (1:1) salt,
• (+)-*O*-desmethyl-Tramadol - Triflusal (1:1) salt,
• (+)-*O*-desmethyl-Tramadol - (*S*)-Naproxen (1:1) salt, form A,
• (+)-*O*-desmethyl-Tramadol - (*S*)-Naproxen (1:1) salt, form B,
• (-)-*O*-desmethyl-Tramadol - (*S*)-Naproxen (1:1) salt, form A,
• (-)-*O*-desmethyl-Tramadol - (*S*)-Naproxen (1:1) salt, form B,
• (*rac*)-*O*-desmethyl-Tramadol - (*S*)-Naproxen (1:1) salt,
• (+)-*O*-desmethyl-Tramadol - (*R*)-Flurbiprofen (1:1) salt,
• (-)-*O*-desmethyl-Tramadol - (*R*)-Flurbiprofen (1:1) salt,
• (+)-*O*-desmethyl-Tramadol - (*S*)-Ibuprofen - H₂O (1:1:0.3) salt,
• (+)-*O*-desmethyl-Tramadol - (*S*)-Ibuprofen (1:1) salt, or
• (-)-*O*-desmethyl-Tramadol - (*S*)-Ibuprofen (1:1) salt.

10. Pharmaceutical compound according to any one of claims 1 to 3, wherein the compound is a co-crystal comprising *O*-desmethyl-Tramadol as a free base or as its pharmaceutically acceptable salt and at least one COX-inhibitor.

11. Pharmaceutical compound according to claim 10, wherein the molecular ratio in the co-crystal of *O*-desmethyl-Tramadol to COX-inhibitor is 1:2 to 2:1, preferably is 2:3 to 3:2 or 1:1.

12. Pharmaceutical compound according to claim 10, wherein the co-crystal is selected from (*rac*)-*O*-desmethyl-Tramadol - (*rac*)-Flurbiprofen, (*rac*)-*O*-desmethyl-Tramadol - (*S*)-Ibuprofen or (*rac*)-*O*-desmethyl-Tramadol·HCl - Paracetamol, preferably is selected from (*rac*)-*O*-desmethyl-Tramadol - (*rac*)-Flurbiprofen (2:3), (*rac*)-*O*-desmethyl-Tramadol - (*S*)-Ibuprofen (2:3) co-crystal or (*rac*)-*O*-desmethyl-Tramadol·HCl - Paracetamol (1:1) co-crystal.

13. Medicament comprising at least one pharmaceutical compound according to any of claims 1 to 12 and optionally one or more pharmaceutically acceptable excipients.

14. Pharmaceutical composition comprising a therapeutically effective amount of the pharmaceutical compound according to any one of claims 1 to 7, crystal of claims 8 or 9 or co-crystal of claims 10 to 12 in a physiologically acceptable medium.

15. Use of a pharmaceutical compound according to any one of claims 1 to 12 for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia.
